# EUROPEAN PATENT SPECIFICATION

(11) **EP 3 122 870 B1**
(45) Date of publication and mention of the grant of the patent: **29.06.2022**
(21) Application number: 15768011.7
(22) Date of filing: 25.03.2015
(51) Int. Cl.: C12N 5/00, C12N 15/00, C12N 15/87, C12N 15/74, A61K 48/00, C07H 21/04

(54) **METHODS AND GENETIC SYSTEMS FOR CELL ENGINEERING**
VERFAHREN UND GENETISCHE SYSTEME ZUR ZELLEN-ENGINEERING
MÉTHODES ET SYSTÈMES GÉNÉTIQUES POUR LE GÉNIE CELLULAIRE

(30) Priority: 25.03.2014 US 201461970024 P
(43) Date of publication of application: 01.02.2017
(73) Proprietor: Ginkgo Bioworks Inc., Boston, Massachusetts 02210 (US)
(72) Inventor: BOYLE, Patrick, Boston, MA 02130 (US); SHETTY, Reshma P., Boston, Massachusetts 02127 (US)
(74) Representative: Mewburn Ellis LLP
(86) International application number: PCT/US2015/022508
(87) International publication number: WO 2015/148680

(56) References cited:
- WO-A2-2015/034872
- US-A1- 2009 110 664
- US-A1- 2013 045 539
- US-A1- 2014 068 797
- US-B2- 7 700 723
- BIKARD DAVID ET AL: "CRISPR Interference Can Prevent Natural Transformation and Virulence Acquisition during In Vivo Bacterial Infection", CELL HOST & MICROBE, vol. 12, no. 2, 16 August 2012 (2012-08-16), pages 177-186, XP028934538, ISSN: 1931-3128, DOI: 10.1016/J.CHOM.2012.06.003
- R. SAPRANAUSKAS ET AL: "The Streptococcus thermophilus CRISPR/Cas system provides immunity in Escherichia coli", NUCLEIC ACIDS RESEARCH, vol. 39, no. 21, 3 August 2011 (2011-08-03), pages 9275-9282, XP055265024, ISSN: 0305-1048, DOI: 10.1093/nar/gkr606
- RAN F ANN ET AL: "Genome engineering using the CRISPR-Cas9 system", NATURE PROTO, NATURE PUBLISHING GROUP, GB, vol. 8, no. 11, 1 November 2013 (2013-11-01), pages 2281-2308, XP009174668, ISSN: 1750-2799, DOI: 10.1038/NPROT.2013.143
- MART KRUPOVIC ET AL: "Casposons: a new superfamily of self-synthesizing DNA transposons at the origin of prokaryotic CRISPR-Cas immunity", BMC BIOLOGY, BIOMED CENTRAL, LONDON, GB, GB, vol. 12, no. 1, 19 May 2014 (2014-05-19), page 36, XP021188194, ISSN: 1741-7007, DOI: 10.1186/1741-7007-12-36
- HAGENS ET AL.: 'Therapy of experimental pseudomonas infections with a nonreplicating genetically modified phage.' ANTIMICROB AGENTS CHEMOTHER vol. 48, no. 10, October 2004, pages 3817 - 3822, XP002483597
- GOMAA ET AL.: 'Programmable Removal of Bacterial Strains by Use of Genome- Targeting CRISPR-Cas Systems.' MBIO vol. 5, no. 1 E009, pages 1 - 9, XP055227960
- JINEK M ET AL.: 'A programmable dual- RNA-guided DNA endonuclease in adaptive, bacterial immunity.' SCIENCE vol. 337, no. 6096, 17 August 2012, pages 816 - 821, XP055067747

### SEQUENCE LISTING

<110> Ginkgo BioWorks, Inc. Boyle, Patrick Shetty, Reshma P.
<120> Methods and Genetic Systems for Cell Engineering
<130> 134395-010501/PCT
<140> US 61/970,024
   <141> 2014-03-25
<160> 20
<170> PatentIn version 3.5
<210> 1
   <211> 4409
   <212> DNA
   <213> Unknown
<220>
   <223> Synthetic
<400> 1
   mgcccacagc taacaccacg tcgtccctat ctgctgccct aggtctatga gtggttgctg      60
   gataacttta cgggcatgca taaggctcgt atgatatatt cagggagacc acaacggttt     120
   ccctctacaa ataattttgt ttaacttttc acacaggaaa gtactagatg gacaagaaat     180
   actcgatagg gctggatatc gggaccaact ccgtcggttg ggctgttatc acggatgaat     240
   ataaggtgcc cagcaaaaag ttcaaggtgc taggtaacac cgaccggcac agtatcaaaa     300
   aaaacttgat aggagcgttg ctgtttgaca gtggtgagac cgctgaagct acgcgcctta     360
   aaaggaccgc gcgccgtaga tatacccgca ggaagaaccg catttgctat ctccaagaga     420
   ttttttcgaa cgagatggct aaagtagatg atagtttctt tcatcgattg gaagaaagct     480
   ttttagttga agaagacaag aagcatgaac gccatccaat ttttggtaac atagtagatg      540
   aagtggcgta tcatgagaaa tatccgacga tctatcatct acgaaaaaaa ttagtagata     600
   gcacggataa agctgattta cggctgatct atttagcact ggctcatatg attaagtttc     660
   gcggccattt tttaattgag ggtgatctga acccagataa cagtgatgtt gacaaactct     720
   ttatccaatt agtacagact tacaaccagc tgtttgaaga aaatccaatt aatgccagtg     780
   gtgtagatgc gaaagctatt ttgagcgccc gattaagtaa atcgcgtcga ctggaaaacc     840
   ttattgcgca acttcctggc gagaagaaaa acgggctgtt tggaaacctt attgcgttat     900
   cgttaggctt aactccaaac tttaaatcga actttgattt agccgaagat gcgaaactgc     960
   aattgtcgaa agatacgtac gatgatgatc tggataacct gttagctcag attggtgatc    1020
   agtatgcgga tttattttta gccgcgaaga acctgtcgga tgcgattctg ttgtcggata    1080
   tcctccgtgt aaacacggaa ataacgaagg cgcctctctc ggcgtcgatg attaaacggt    1140
   acgatgaaca tcatcaggac ttaacgttgc tgaaagcgct ggtgcgacag cagttgccgg 1200
   aaaagtataa agaaatcttt tttgatcagt cgaaaaatgg ttatgccggc tatattgatg 1260
   gaggtgcgtc ccaggaagaa ttttataaat ttatcaaacc gattctggaa aaaatggatg 1320
   gcacggagga actgttggtt aaactcaacc gcgaagattt gctacggaag cagaggactt 1380
   ttgacaatgg gagcattcct catcagattc acttgggcga gctacatgcg attttgcgtc 1440
   gtcaggaaga cttttatccg tttctgaaag acaaccgcga gaagattgaa aaaatcttga 1500
   cgtttcgaat cccatattat gtgggcccgt tagctcgcgg gaacagtcgc tttgcctgga 1560
   tgacgaggaa gtcggaagaa accattactc cgtggaactt tgaagaagtg gtcgataaag 1620
   gcgcgtcggc gcagtcgttt attgaacgga tgaccaattt tgataaaaac ttgccgaacg 1680
   aaaaagtact cccgaaacat agtttattgt atgagtattt tacagtgtat aatgaattaa 1740
   ccaaggtcaa atatgtgacg gaaggtatgc gaaaaccggc ctttttgtcg ggcgaacaaa 1800
   agaaagcaat tgtggatctg cttttcaaaa ccaaccgaaa agtaactgtg aagcagctga 1860
   aagaagatta tttcaaaaaa atagaatgct ttgatagtgt ggaaatttcg ggtgtggaag 1920
   atcgttttaa cgcgtcgctg ggcacttacc atgatttact caaaattatt aaagataaag 1980
   attttttaga taacgaagaa aacgaagata tcctggagga tattgtgctg accttaactc 2040
   tgtttgaaga tagagagatg attgaggaac gtttgaaaac ctatgcgcac ctttttgatg 2100
   ataaggttat gaaacaattg aaacgccggc gctatacggg ctggggtcgc ttaagccgaa 2160
   aattaattaa cggcattaga gataagcaga gcgggaaaac catactggat tttttaaaat 2220
   cggatggctt tgcaaaccgg aactttatgc aactaatcca tgatgatagt ttaaccttta 2280
   aagaagacat tcagaaagcc caggttagcg gtcaggggga tagtctgcat gaacatattg 2340
   ccaacctggc gggctcccca gcgattaaaa aaggcattct gcaaacggta aaagtggtgg 2400
   atgaattagt caaagtaatg ggaaggcata agccggaaaa catcgtgatt gaaatggccc 2460
   gcgaaaacca aaccacgcag aaggggcaaa aaaactcacg agagcgcatg aaacgaatcg 2520
   aagaaggcat caaagaactg ggtagtcaaa ttttgaaaga gcatccagtg gaaaacacgc 2580
   agttacagaa cgaaaagctt tatctttatt atcttcagaa cggtcgtgac atgtatgttg 2640
   accaggaact ggatattaac cgcctgagtg attatgatgt cgatcacatt gtgccgcaga 2700
   gtttcttgaa agacgattcg atagacaaca aggtcctgac acgcagcgat aaaaaccgcg 2760
   gcaaatcaga taatgtgccg agtgaagaag tagtcaaaaa gatgaaaaat tattggcgtc 2820
   agttgctcaa tgcaaagctg atcacgcagc gcaagtttga taacctgaca aaagcggaac 2880
   gcggtggctt aagtgaattg gataaagcgg gctttatcaa acggcagtta gtggaaacgc 2940
   ggcagatcac gaagcatgtt gcccagattt tagatagtcg gatgaacacg aaatacgatg 3000
   aaaacgataa attgattcga gaggtgaaag ttattactct gaaaagcaaa ctggtgagcg 3060
   acttccgaaa agatttccag ttctataaag tacgcgagat taataactac catcatgcac 3120
   atgatgctta tctcaacgca gtcgtgggta cggcgttaat taagaaatat ccgaaattgg 3180
   aatcagagtt tgtctatggc gattataaag tgtatgatgt gcgcaaaatg attgcgaagt 3240
   cggagcagga aatagggaaa gccactgcca aatatttctt ttacagcaac atcatgaatt 3300
   tcttcaaaac cgaaattacc ttggccaacg gtgagattcg gaaacggcca ctcatcgaaa 3360
   cgaacggaga aacgggtgaa attgtctggg ataaaggacg agattttgca accgttcgga 3420
   aagtattgtc catgcctcag gtcaacattg tcaagaaaac cgaagtacaa accgggggtt 3480
   tctccaagga gtcgattctg ccgaaacgca actcagacaa gttgattgcg cgcaaaaaag 3540
   actgggatcc gaaaaaatat ggcggcttcg attccccgac agtagcgtac tcggtcctcg 3600
   tcgtggcgaa ggtcgaaaaa gggaaatcca agaagctgaa atccgtgaaa gagctgctcg 3660
   ggatcaccat catggagcgc tcctccttcg agaagaaccc catcgacttc ctggaggcga 3720
   agggctacaa ggaggtgaag aaggacctga tcatcaagct ccccaagtac tccctctttg 3780
   agctggagaa cggccgcaag aggatgctcg caagtgcagg tgaattacaa aaaggtaatg 3840
   aactagcgct accgtccaaa tatgttaact ttctgtatct ggcgagtcat tatgaaaagt 3900
   taaagggcag tccggaagat aatgaacaga aacagttatt tgttgagcaa cataagcatt 3960
   atctggatga gattattgag cagatcagtg aatttagcaa gcgcgtgatt ctggccgatg 4020
   caaacctgga taaagtgttg agtgcctata ataaacatcg tgacaaaccg atacgcgaac 4080
   aggccgaaaa cattattcat ctgtttacat taacaaactt gggtgcgcct gcggcgttta 4140
   aatattttga taccaccatt gatcgcaaac gatatacaag caccaaagaa gtgctggatg 4200
   caacgttgat ccatcagtct atcacgggct tgtatgaaac ccggattgat ttaagtcaac 4260
   tcggtggcga ctaagcccca ggcatcaaat aaaacgaaag gctcagtcga aagactgggc 4320
   ctttcgtttt atctgttgtt tgtcggtgaa cgctctctac tagagtcaca ctggctcacc 4380
   ttcgggtggg cctttctgcg tttatagcc 4409
<210> 2
   <211> 259
   <212> **DNA**
   <213> Unknown
<220>
   <223> Synthetic
<400> 2
   mgcctcccta tcagtgatag agattgacat ccctatcagt gatagagata ctgagcacgc      60
   cggaaccatt caaaacagca tagcaagtta aaataaggct agtccgttat caacttgaaa     120
   aagtggcacc gagtcggtgc gccccggctt atcggtcagt ttcacctgat ttacgtaaaa     180
   acccgcttcg gcgggttttt gcttttggag gggcagaaag atgaatgact gtccacgacg     240
   ctatacccaa aagaaagcc                                                  259
<210> 3
   <211> 376
   <212> **DNA**
   <213> unknown
<220>
   <223> Synthetic
<400> 3
   mgcctcccta tcagtgatag agattgacat ccctatcagt gatagagata ctgagcacgc      60
   caggggcttt tcaagactga agtctagctg agacaaatag tgcgattacg aaatttttta      120
   gacaaaaata gtctacgagg ttttagagct atgctgtttt gaatggtccc aaaacatggc     180
   aatgaaagac ggtgagctgg tgatagtttt agagctatgc tgttttgaat ggtctccatt     240
   cgccccaggc atcaaataaa acgaaaggct cagtcgaaag actgggcctt tcgttttatc     300
   tgttgtttgt cggtgaacgc tctctactag agtcacactg gctcaccttc gggtgggcct     360
   ttctgcgttt atagcc                                                     376
<210> 4
   <211> 3223
   <212> **DNA**
   <213> Unknown
<220>
   <223> Synthetic
<400> 4
   maaaaaaaat ccttagcttt cgctaaggat gatttctgga attcgcggcc gcttctagag      60
   cccacagcta acaccacgtc gtccctatct gctgccctag gtctatgagt ggttgctgga     120
   taactttacg ggcatgcata aggctcgtat gatatattca gggagaccac aacggtttcc     180
   ctctacaaat aattttgttt aacttttcac acaggaaagt actagatgac catgattacg     240
   gattcactgg ccgtcgtttt acaacgtcgt gactgggaaa accctggcgt tacccaactt     300
   aatcgccttg cagcacatcc ccctttcgca agctggcgta atagcgagga ggcccgcacc     360
   gatcgccctt cccaacagtt gcgcagcctg aatggcgaat ggcgcgcggg cggcagcgaa     420
   ggcggcggca gcgaacatca tcatcatcat catggcagcg aacgtaaagg agaagaactt     480
   ttcactggag ttgtcccaat tcttgttgaa ttagatggtg atgttaatgg gcacaaattt     540
   tctgtcagtg gagagggtga aggtgatgca acatacggaa aacttaccct taaatttatt     600
   tgcactactg gaaaactacc tgttccatgg ccaacacttg tcactaccct gacctatggt     660
   gttcaatgct ttgcgagata cccagatcat atgaaacagc atgacttttt caagagtgcc     720
   atgcccgaag gttatgtaca ggaaagaact atatttttca aagatgacgg gaactacaag 780
   acacgtgctg aagtcaagtt tgaaggtgat acccttgtta atagaatcga gttaaaaggt 840
   attgatttta aagaagatgg aaacattctt ggacacaaat tggaatacaa ctataactca 900
   cacaatgtat acatcatggc agacaaacaa aagaatggaa tcaaagttaa cttcaaaatt 960
   agacacaaca ttgaagatgg aagcgttcaa ctagcagacc attatcaaca aaatactcca 1020
   attggcgatg gccctgtcct tttaccagac aaccattacc tgtccacaca atctgccctt 1080
   tcgaaagatc ccaacgaaaa gagagaccac atggtccttc ttgagtttgt aacagctgct 1140
   gggattacac atggcatgga tgaactatac aagtaagcca cacgcgctct cccccctccg 1200
   gtgtaatcgg gggagagcgc gtgtccgctg cagtccggca aaaaagggca aggtgtcacc 1260
   accctgccct ttttctttaa aaccgaaaag attacttcgc gttatgcagg cttcctcgct 1320
   cactgactcg ctgcgctcgg tcgttcggct gcggcgagcg gtatcagctc actcaaaggc 1380
   ggtaatacgg ttatccacag aatcagggga taacgcagga aagaacatgt gagcaaaagg 1440
   ccagcaaaag gccaggaacc gtaaaaaggc cgcgttgctg gcgtttttcc acaggctccg 1500
   cccccctgac gagcatcaca aaaatcgacg ctcaagtcag aggtggcgaa acccgacagg 1560
   actataaaga taccaggcgt ttccccctgg aagctccctc gtgcgctctc ctgttccgac 1620
   cctgccgctt accggatacc tgtccgcctt tctcccttcg ggaagcgtgg cgctttctca 1680
   tagctcacgc tgtaggtatc tcagttcggt gtaggtcgtt cgctccaagc tgggctgtgt 1740
   gcacgaaccc cccgttcagc ccgaccgctg cgccttatcc ggtaactatc gtcttgagtc 1800
   caacccggta agacacgact tatcgccact ggcagcagcc actggtaaca ggattagcag 1860
   agcgaggtat gtaggcggtg ctacagagtt cttgaagtgg tggcctaact acggctacac 1920
   tagaagaaca gtatttggta tctgcgctct gctgaagcca gttaccttcg gaaaaagagt 1980
   tggtagctct tgatccggca aacaaaccac cgctggtagc ggtggttttt ttgtttgcaa 2040
   gcagcagatt acgcgcagaa aaaaaggatc tcaagaagat cctttgatct tttctacggg 2100
   gtctgacgct cagtggaacg aaaactcacg ttaagggatt ttggtcatga gattatcaaa 2160
   aaggatcttc acctagatcc ttttaaatta aaaatgaagt tttaaatcaa tctaaagtat 2220
   atatgagtaa acttggtctg acagctcgag gcttggattc tcaccaataa aaaacgcccg 2280
   gcggcaaccg agcgttctga acaaatccag atggagttct gaggtcatta ctggatctat 2340
   caacaggagt ccaagcgagc tcgatatcaa attacgcccc gccctgccac tcatcgcagt 2400
   actgttgtaa ttcattaagc attctgccga catggaagcc atcacaaacg gcatgatgaa 2460
   cctgaatcgc cagcggcatc agcaccttgt cgccttgcgt ataatatttg cccatggtga 2520
   aaacgggggc gaagaagttg tccatattgg ccacgtttaa atcaaaactg gtgaaactca 2580
   cccagggatt ggctgagacg aaaaacatat tctcaataaa ccctttaggg aaataggcca 2640
   ggttttcacc gtaacacgcc acatcttgcg aatatatgtg tagaaactgc cggaaatcgt     2700
   cgtggtattc actccagagc gatgaaaacg tttcagtttg ctcatggaaa acggtgtaac     2760
   aagggtgaac actatcccat atcaccagct caccgtcttt cattgccata cgaaattccg     2820
   gatgagcatt catcaggcgg gcaagaatgt gaataaaggc cggataaaac ttgtgcttat     2880
   ttttctttac ggtctttaaa aaggccgtaa tatccagctg aacggtctgg ttataggtac     2940
   attgagcaac tgactgaaat gcctcaaaat gttctttacg atgccattgg gatatatcaa     3000
   cggtggtata tccagtgatt tttttctcca ttttagcttc cttagctcct gaaaatctcg     3060
   ataactcaaa aaatacgccc ggtagtgatc ttatttcatt atggtgaaag ttggaacctc     3120
   ttacgtgccc gatcaactcg agtgccactt gacgtctaag aaaccattat tatcatgaca     3180
   ttaacctata aaaataggcg tatcacgagg cagaatttca gat                       3223
<210> 5
   <211> 5391
   <212> DNA
   <213> Unknown
<220>
   <223> Synthetic
<400> 5
   mttatgacaa cttgacggct acatcattca ctttttcttc acaaccggca cggaactcgc 60
   tcgggctggc cccggtgcat tttttaaata cccgcgagaa atagagttga tcgtcaaaac 120
   caacattgcg accgacggtg gcgataggca tccgggtggt gctcaaaagc agcttcgcct 180
   ggctgatacg ttggtcctcg cgccagctta agacgctaat ccctaactgc tggcggaaaa 240
   gatgtgacag acgcgacggc gacaagcaaa catgctgtgc gacgctggcg atatcaaaat 300
   tgctgtctgc caggtgatcg ctgatgtact gacaagcctc gcgtacccga ttatccatcg 360
   gtggatggag cgactcgtta atcgcttcca tgcgccgcag taacaattgc tcaagcagat 420
   ttatcgccag cagctccgaa tagcgccctt ccccttgccc ggcgttaatg atttgcccaa 480
   acaggtcgct gaaatgcggc tggtgcgctt catccgggcg aaagaacccc gtattggcaa 540
   atattgacgg ccagttaagc cattcatgcc agtaggcgcg cggacgaaag taaacccact 600
   ggtgatacca ttcgcgagcc tccggatgac gaccgtagtg atgaatctct cctggcggga 660
   acagcaaaat atcacccggt cggcaaacaa attctcgtcc ctgatttttc accaccccct 720
   gaccgcgaat ggtgagattg agaatataac ctttcattcc cagcggtcgg tcgataaaaa 780
   aatcgagata accgttggcc tcaatcggcg ttaaacccgc caccagatgg gcattaaacg 840
   agtatcccgg cagcagggga tcattttgcg cttcagccat acttttcata ctcccgccat 900
   tcagagaaga aaccaattgt ccatattgca tcagacattg ccgtcactgc gtcttttact 960
   ggctcttctc gctaaccaaa ccggtaaccc cgcttattaa aagcattctg taacaaagcg 1020
   ggaccaaagc catgacaaaa acgcgtaaca aaagtgtcta taatcacggc agaaaagtcc 1080
   acattgatta tttgcacggc gtcacacttt gctatgccat agcattttta tccataagat 1140
   tagcggatcc tacctgacgc tttttatcgc aactctctac tgtttctcca taaaagagga 1200
   gaaatactag atgaccatga ttacggattc actggccgtc gttttacaac gtcgtgactg 1260
   ggaaaaccct ggcgttaccc aacttaatcg ccttgcagca catccccctt tcgcaagctg 1320
   gcgtaatagc gaggaggccc gcaccgatcg cccttcccaa cagttgcgca gcctgaatgg 1380
   cgaatggcgc gcgggcggca gcgaaggcgg cggcagcgaa catcatcatc atcatcatgg 1440
   cagcgaacgt aaaggagaag aacttttcac tggagttgtc ccaattcttg ttgaattaga 1500
   tggtgatgtt aatgggcaca aattttctgt cagtggagag ggtgaaggtg atgcaacata 1560
   cggaaaactt acccttaaat ttatttgcac tactggaaaa ctacctgttc catggccaac 1620
   acttgtcact accctgacct atggtgttca atgctttgcg agatacccag atcatatgaa 1680
   acagcatgac tttttcaaga gtgccatgcc cgaaggttat gtacaggaaa gaactatatt 1740
   tttcaaagat gacgggaact acaagacacg tgctgaagtc aagtttgaag gtgataccct 1800
   tgttaataga atcgagttaa aaggtattga ttttaaagaa gatggaaaca ttcttggaca 1860
   caaattggaa tacaactata actcacacaa tgtatacatc atggcagaca aacaaaagaa 1920
   tggaatcaaa gttaacttca aaattagaca caacattgaa gatggaagcg ttcaactagc 1980
   agaccattat caacaaaata ctccaattgg cgatggccct gtccttttac cagacaacca 2040
   ttacctgtcc acacaatctg ccctttcgaa agatcccaac gaaaagagag accacatggt 2100
   ccttcttgag tttgtaacag ctgctgggat tacacatggc atggatgaac tatacaagta 2160
   atactagtag cggccgctgc aggagtcact aagggttagt tagttagatt agcagaaagt 2220
   caaaagcctc cgaccggagg cttttgacta aaacttccct tggggttatc attggggctc 2280
   actcaaaggc ggtaatcaga taaaaaaaat ccttagcttt cgctaaggat gatttctgct 2340
   agagctgtca gaccaagttt acgagctcgc ttggactcct gttgatagat ccagtaatga 2400
   cctcagaact ccatctggat ttgttcagaa cgctcggttg ccgccgggcg ttttttattg 2460
   gtgagaatcc aagcactagg gacagtaaga cgggtaagcc tgttgatgat accgctgcct 2520
   tactgggtgc attagccagt ctgaatgacc tgtcacggga taatccgaag tggtcagact 2580
   ggaaaatcag agggcaggaa ctgctgaaca gcaaaaagtc agatagcacc acatagcaga 2640
   cccgccataa aacgccctga gaagcccgtg acgggctttt cttgtattat gggtagtttc 2700
   cttgcatgaa tccataaaag gcgcctgtag tgccatttac ccccattcac tgccagagcc 2760
   gtgagcgcag cgaactgaat gtcacgaaaa agacagcgac tcaggtgcct gatggtcgga 2820
   gacaaaagga atattcagcg atttgcccga gcttgcgagg gtgctactta agcctttagg 2880
   gttttaaggt ctgttttgta gaggagcaaa cagcgtttgc gacatccttt tgtaatactg 2940
   cggaactgac taaagtagtg agttatacac agggctggga tctattcttt ttatcttttt 3000
   ttattctttc tttattctat aaattataac cacttgaata taaacaaaaa aaacacacaa 3060
   aggtctagcg gaatttacag agggtctagc agaatttaca agttttccag caaaggtcta 3120
   gcagaattta cagataccca caactcaaag gaaaaggaca tgtaattatc attgactagc 3180
   ccatctcaat tggtatagtg attaaaatca cctagaccaa ttgagatgta tgtctgaatt 3240
   agttgttttc aaagcaaatg aactagcgat tagtcgctat gacttaacgg agcatgaaac 3300
   caagctaatt ttatgctgtg tggcactact caaccccacg attgaaaacc ctacaaggaa 3360
   agaacggacg gtatcgttca cttataacca atacgctcag atgatgaaca tcagtaggga 3420
   aaatgcttat ggtgtattag ctaaagcaac cagagagctg atgacgagaa ctgtggaaat 3480
   caggaatcct ttggttaaag gctttgagat tttccagtgg acaaactatg ccaagttctc 3540
   aagcgaaaaa ttagaattag tttttagtga agagatattg ccttatcttt tccagttaaa 3600
   aaaattcata aaatataatc tggaacatgt taagtctttt gaaaacaaat actctatgag 3660
   gatttatgag tggttattaa aagaactaac acaaaagaaa actcacaagg caaatataga 3720
   gattagcctt gatgaattta agttcatgtt aatgcttgaa aataactacc atgagtttaa 3780
   aaggcttaac caatgggttt tgaaaccaat aagtaaagat ttaaacactt acagcaatat 3840
   gaaattggtg gttgataagc gaggccgccc gactgatacg ttgattttcc aagttgaact 3900
   agatagacaa atggatctcg taaccgaact tgagaacaac cagataaaaa tgaatggtga 3960
   caaaatacca acaaccatta catcagattc ctacctacat aacggactaa gaaaaacact 4020
   acacgatgct ttaactgcaa aaattcagct caccagtttt gaggcaaaat ttttgagtga 4080
   catgcaaagt aagtatgatc tcaatggttc gttctcatgg ctcacgcaaa aacaacgaac 4140
   cacactagag aacatactgg ctaaatacgg aaggatctga ggttcttatg gctcttgtat 4200
   ctatcagtga agcatcaaga ctaacaaaca aaagtagaac aactgttcac cgttacatat 4260
   caaagggaaa actgtccata tgcacagatg aaaacggtgt aaaaaagata gatacatcag 4320
   agcttttacg agtttttggt gcattcaaag ctgttcacca tgaacagatc gacaatgtaa 4380
   ctactagagg ttgatcgggc acgtaagagg ttccaacttt caccataatg aaataagatc 4440
   actaccgggc gtattttttg agttatcgag attttcagga gctaaggaag ctaaaatgga 4500
   gaaaaaaatc acgggatata ccaccgttga tatatcccaa tggcatcgta aagaacattt 4560
   tgaggcattt cagtcagttg ctcaatgtac ctataaccag accgttcagc tggatattac 4620
   ggccttttta aagaccgtaa agaaaaataa gcacaagttt tatccggcct ttattcacat 4680
   tcttgcccgc ctgatgaacg ctcacccgga gtttcgtatg gccatgaaag acggtgagct 4740
   ggtgatctgg gatagtgttc acccttgtta caccgttttc catgagcaaa ctgaaacgtt 4800
   ttcgtccctc tggagtgaat accacgacga tttccggcag tttctccaca tatattcgca 4860
   agatgtggcg tgttacggtg aaaacctggc ctatttccct aaagggttta ttgagaatat 4920
   gttttttgtc tcagccaatc cctgggtgag tttcaccagt tttgatttaa acgtggccaa 4980
   tatggacaac ttcttcgccc ccgttttcac gatgggcaaa tattatacgc aaggcgacaa 5040
   ggtgctgatg ccgctggcga tccaggttca tcatgccgtt tgtgatggct tccatgtcgg 5100
   ccgcatgctt aatgaattac aacagtactg tgatgagtgg cagggcgggg cgtaataata 5160
   ctagctccgg caaaaaaacg ggcaaggtgt caccaccctg ccctttttct ttaaaaccga 5220
   aaagattact tcgcgtttgc cacctgacgt ctaagaaaag gaatattcag caatttgccc 5280
   gtgccgaaga aaggcccacc cgtgaaggtg agccagtgag ttgattgcta cgtaattagt 5340
   tagttagccc ttagtgactc gaattcgcgg ccgcttctag agacctcgtg t 5391
<210> 6
   <211> 3848
   <212> DNA
   <213> Unknown
<220>
   <223> Synthetic
<400> 6
   mcacagctaa caccacgtcg tccctatctg ctgccctagg tctatgagtg gttgctggat 60
   aactttacgg gcatgcataa ggctcgtatg atatattcag ggagaccaca acggtttccc 120
   tctacaaata attttgttta acttttcaca caggaaagta ctagatggta agcaagggcg 180
   aggagctgtt caccggggtg gtgcccatcc tggtcgagct ggacggtgac gtaaacggtc 240
   acaagttcag cgtgagtggc gagggcgagg gtgatgccac ctacggtaag ctgaccctga     300
   agctgatctg caccaccggt aagctgcccg tgccctggcc cacccttgtg accaccctgg     360
   gctacggtct gcaatgcttc gcccgttacc ccgaccacat gaagcagcac gacttcttca 420
   agtccgccat gcccgaaggc tacgtccagg agcgcaccat cttcttcaag gacgacggca 480
   actacaagac ccgcgccgag gtgaagttcg agggcgacac cctggtgaac cgcatcgagc 540
   tgaagggcat cgacttcaag gaggacggca acatcctggg gcacaagctg gagtacaact 600
   acaacagcca caacgtctat atcaccgccg acaagcagaa gaacggcatc aaggccaact 660
   tcaagatccg ccacaacatc gaggacggcg gcgtgcagct cgccgaccac taccagcaga 720
   acacccccat cggcgacggc cccgtgctgc tgcccgacaa ccactacctg agctaccagt 780
   ccaagctgag caaagacccc aacgagaagc gcgatcacat ggtcctgctg gagttcgtga 840
   ccgccgccgg gatcactctc ggcatggacg agctgtacaa ggccaatgtc gagtaacatg 900
   gtaagcaagg gcgaggagaa taacatggcc atcatcaagg agttcatgcg cttcaaggtg 960
   cacatggagg gcagtgtgaa cggccacgag ttcgagatcg agggcgaggg cgagggccgt 1020
   ccctacgagg cctttcagac cgctaagctg aaggtgacca agggtggccc cctgcccttc 1080
   gcctgggaca tcctgtcccc tcagttcatg tacggctcca aggtctacat taagcaccca 1140
   gccgacatcc ccgactactt caagctgtcc ttccccgagg gcttcaggtg ggagcgcgtg 1200
   atgaacttcg aggacggcgg cattattcac gttaaccagg acagttccct gcaagacggc 1260
   gtgttcatct acaaggtgaa gctgcgcggc accaacttcc ccagtgacgg ccccgtaatg 1320
   cagaagaaga ccatgggctg ggaggccagt gaggagcgga tgtaccccga ggacggcgcc 1380
   ctgaagtctg agatcaaaaa gaggctgaag ctgaaggacg gcggccacta cgccgccgag 1440
   gtcaagacca cctacaaggc caagaagccc gtgcagctgc ccggcgccta catcgtcgac 1500
   atcaagttgg acatcgtgtc ccacaacgag gactacacca tcgtggaaca gtacgaacgc 1560
   gccgagggcc gccactccac cggcggcatg gacgagctgt acaaggccta accaggcatc 1620
   aaataaaacg aaaggctcag tcgaaagact gggcctttcg ttttatctgt tgtttgtcgg 1680
   tgaacgctct ctactagagt cacactggct caccttcggg tgggcctttc tgcgtttata 1740
   gccacacgcg ctctcccccc tccggtgtaa tcgggggaga gcgcgtgtcc gctgcagtcc 1800
   ggcaaaaaag ggcaaggtgt caccaccctg ccctttttct ttaaaaccga aaagattact 1860
   tcgcgttatg caggcttcct cgctcactga ctcgctgcgc tcggtcgttc ggctgcggcg 1920
   agcggtatca gctcactcaa aggcggtaat acggttatcc acagaatcag gggataacgc 1980
   aggaaagaac atgtgagcaa aaggccagca aaaggccagg aaccgtaaaa aggccgcgtt 2040
   gctggcgttt ttccacaggc tccgcccccc tgacgagcat cacaaaaatc gacgctcaag 2100
   tcagaggtgg cgaaacccga caggactata aagataccag gcgtttcccc ctggaagctc 2160
   cctcgtgcgc tctcctgttc cgaccctgcc gcttaccgga tacctgtccg cctttctccc 2220
   ttcgggaagc gtggcgcttt ctcatagctc acgctgtagg tatctcagtt cggtgtaggt 2280
   cgttcgctcc aagctgggct gtgtgcacga accccccgtt cagcccgacc gctgcgcctt 2340
   atccggtaac tatcgtcttg agtccaaccc ggtaagacac gacttatcgc cactggcagc 2400
   agccactggt aacaggatta gcagagcgag gtatgtaggc ggtgctacag agttcttgaa 2460
   gtggtggcct aactacggct acactagaag aacagtattt ggtatctgcg ctctgctgaa 2520
   gccagttacc ttcggaaaaa gagttggtag ctcttgatcc ggcaaacaaa ccaccgctgg 2580
   tagcggtggt ttttttgttt gcaagcagca gattacgcgc agaaaaaaag gatctcaaga 2640
   agatcctttg atcttttcta cggggtctga cgctcagtgg aacgaaaact cacgttaagg 2700
   gattttggtc atgagattat caaaaaggat cttcacctag atccttttaa attaaaaatg 2760
   aagttttaaa tcaatctaaa gtatatatga gtaaacttgg tctgacagct cgaggcttgg 2820
   attctcacca ataaaaaacg cccggcggca accgagcgtt ctgaacaaat ccagatggag 2880
   ttctgaggtc attactggat ctatcaacag gagtccaagc gagctcgata tcaaattacg 2940
   ccccgccctg ccactcatcg cagtactgtt gtaattcatt aagcattctg ccgacatgga 3000
   agccatcaca aacggcatga tgaacctgaa tcgccagcgg catcagcacc ttgtcgcctt 3060
   gcgtataata tttgcccatg gtgaaaacgg gggcgaagaa gttgtccata ttggccacgt 3120
   ttaaatcaaa actggtgaaa ctcacccagg gattggctga gacgaaaaac atattctcaa 3180
   taaacccttt agggaaatag gccaggtttt caccgtaaca cgccacatct tgcgaatata 3240
   tgtgtagaaa ctgccggaaa tcgtcgtggt attcactcca gagcgatgaa aacgtttcag 3300
   tttgctcatg gaaaacggtg taacaagggt gaacactatc ccatatcacc agctcaccgt 3360
   ctttcattgc catacgaaat tccggatgag cattcatcag gcgggcaaga atgtgaataa 3420
   aggccggata aaacttgtgc ttatttttct ttacggtctt taaaaaggcc gtaatatcca 3480
   gctgaacggt ctggttatag gtacattgag caactgactg aaatgcctca aaatgttctt 3540
   tacgatgcca ttgggatata tcaacggtgg tatatccagt gatttttttc tccattttag 3600
   cttccttagc tcctgaaaat ctcgataact caaaaaatac gcccggtagt gatcttattt 3660
   cattatggtg aaagttggaa cctcttacgt gcccgatcaa ctcgagtgcc acttgacgtc 3720
   taagaaacca ttattatcat gacattaacc tataaaaata ggcgtatcac gaggcagaat 3780
   ttcagataaa aaaaatcctt agctttcgct aaggatgatt tctggaattc gcggccgctt 3840
   ctagagcc 3848
<210> 7
   <211> 3210
   <212> DNA
   <213> Unknown
<220>
   <223> Synthetic
<400> 7
   mcacagctaa caccacgtcg tccctatctg ctgccctagg tctatgagtg gttgctggat        60
   aactttacgg gcatgcataa ggctcgtatg atatattcag ggagaccaca acggtttccc       120
   tctacaaata attttgttta actttgccag tacgtctgag cgtgataccc gctcactgaa       180
   gatggcccgg tagggccgaa acgtacctct acaaataatt ttgtttaagc ctcacacagg       240
   aaagtactag atggtaagca agggcgagga gctgttcacc ggggtggtgc ccatcctggt       300
   cgagctggac ggtgacgtaa acggtcacaa gttcagcgtg agtggcgagg gcgagggtga       360
   tgccacctac ggtaagctga ccctgaagct gatctgcacc accggtaagc tgcccgtgcc       420
   ctggcccacc cttgtgacca ccctgggcta cggtctgcaa tgcttcgccc gttaccccga       480
   ccacatgaag cagcacgact tcttcaagtc cgccatgccc gaaggctacg tccaggagcg       540
   caccatcttc ttcaaggacg acggcaacta caagacccgc gccgaggtga agttcgaggg 600
   cgacaccctg gtgaaccgca tcgagctgaa gggcatcgac ttcaaggagg acggcaacat 660
   cctggggcac aagctggagt acaactacaa cagccacaac gtctatatca ccgccgacaa 720
   gcagaagaac ggcatcaagg ccaacttcaa gatccgccac aacatcgagg acggcggcgt 780
   gcagctcgcc gaccactacc agcagaacac ccccatcggc gacggccccg tgctgctgcc 840
   cgacaaccac tacctgagct accagtccaa gctgagcaaa gaccccaacg agaagcgcga 900
   tcacatggtc ctgctggagt tcgtgaccgc cgccgggatc actctcggca tggacgagct 960
   gtacaaggcc taaccaggca tcaaataaaa cgaaaggctc agtcgaaaga ctgggccttt 1020
   cgttttatct gttgtttgtc ggtgaacgct ctctactaga gtcacactgg ctcaccttcg 1080
   ggtgggcctt tctgcgttta tagccacacg cgctctcccc cctccggtgt aatcggggga 1140
   gagcgcgtgt ccgctgcagt ccggcaaaaa agggcaaggt gtcaccaccc tgcccttttt 1200
   ctttaaaacc gaaaagatta cttcgcgtta tgcaggcttc ctcgctcact gactcgctgc 1260
   gctcggtcgt tcggctgcgg cgagcggtat cagctcactc aaaggcggta atacggttat 1320
   ccacagaatc aggggataac gcaggaaaga acatgtgagc aaaaggccag caaaaggcca 1380
   ggaaccgtaa aaaggccgcg ttgctggcgt ttttccacag gctccgcccc cctgacgagc 1440
   atcacaaaaa tcgacgctca agtcagaggt ggcgaaaccc gacaggacta taaagatacc 1500
   aggcgtttcc ccctggaagc tccctcgtgc gctctcctgt tccgaccctg ccgcttaccg 1560
   gatacctgtc cgcctttctc ccttcgggaa gcgtggcgct ttctcatagc tcacgctgta 1620
   ggtatctcag ttcggtgtag gtcgttcgct ccaagctggg ctgtgtgcac gaaccccccg 1680
   ttcagcccga ccgctgcgcc ttatccggta actatcgtct tgagtccaac ccggtaagac 1740
   acgacttatc gccactggca gcagccactg gtaacaggat tagcagagcg aggtatgtag 1800
   gcggtgctac agagttcttg aagtggtggc ctaactacgg ctacactaga agaacagtat 1860
   ttggtatctg cgctctgctg aagccagtta ccttcggaaa aagagttggt agctcttgat 1920
   ccggcaaaca aaccaccgct ggtagcggtg gtttttttgt ttgcaagcag cagattacgc 1980
   gcagaaaaaa aggatctcaa gaagatcctt tgatcttttc tacggggtct gacgctcagt 2040
   ggaacgaaaa ctcacgttaa gggattttgg tcatgagatt atcaaaaagg atcttcacct 2100
   agatcctttt aaattaaaaa tgaagtttta aatcaatcta aagtatatat gagtaaactt 2160
   ggtctgacag ctcgaggctt ggattctcac caataaaaaa cgcccggcgg caaccgagcg 2220
   ttctgaacaa atccagatgg agttctgagg tcattactgg atctatcaac aggagtccaa 2280
   gcgagctcga tatcaaatta cgccccgccc tgccactcat cgcagtactg ttgtaattca 2340
   ttaagcattc tgccgacatg gaagccatca caaacggcat gatgaacctg aatcgccagc 2400
   ggcatcagca ccttgtcgcc ttgcgtataa tatttgccca tggtgaaaac gggggcgaag     2460
   aagttgtcca tattggccac gtttaaatca aaactggtga aactcaccca gggattggct     2520
   gagacgaaaa acatattctc aataaaccct ttagggaaat aggccaggtt ttcaccgtaa     2580
   cacgccacat cttgcgaata tatgtgtaga aactgccgga aatcgtcgtg gtattcactc     2640
   cagagcgatg aaaacgtttc agtttgctca tggaaaacgg tgtaacaagg gtgaacacta     2700
   tcccatatca ccagctcacc gtctttcatt gccatacgaa attccggatg agcattcatc     2760
   aggcgggcaa gaatgtgaat aaaggccgga taaaacttgt gcttattttt ctttacggtc     2820
   tttaaaaagg ccgtaatatc cagctgaacg gtctggttat aggtacattg agcaactgac     2880
   tgaaatgcct caaaatgttc tttacgatgc cattgggata tatcaacggt ggtatatcca     2940
   gtgatttttt tctccatttt agcttcctta gctcctgaaa atctcgataa ctcaaaaaat     3000
   acgcccggta gtgatcttat ttcattatgg tgaaagttgg aacctcttac gtgcccgatc     3060
   aactcgagtg ccacttgacg tctaagaaac cattattatc atgacattaa cctataaaaa     3120
   taggcgtatc acgaggcaga atttcagata aaaaaaatcc ttagctttcg ctaaggatga     3180
   tttctggaat tcgcggccgc ttctagagcc                                      3210
<210> 8
   <211> 3204
   <212> **DNA**
   <213> Unknown
<220>
   <223> Synthetic
<400> 8
   macacgcgct ctcccccgtc acgtacgtcg cagtacgtgc tcttcgtcac actggctcac    60
   cttcgggtgg gcctttctgc gtttatagcc cacagctaac accacgtcgt ccctatctgc   120
   tgccctaggt ctatgagtgg ttgctggata actttacggg catgcataag gctcgtaata   180
   tatattcagg gagaccacaa cggtttccct ctacaaataa ttttgtttaa cttttcacac   240
   aggaaagtac tagatggtaa gcaagggcga ggagaataac atggccatca tcaaggagtt   300
   catgcgcttc aaggtgcaca tggagggcag tgtgaacggc cacgagttcg agatcgaggg   360
   cgagggcgag ggccgtccct acgaggcctt tcagaccgct aagctgaagg tgaccaaggg   420
   tggccccctg cccttcgcct gggacatcct gtcccctcag ttcatgtacg gctccaaggt   480
   ctacattaag cacccagccg acatccccga ctacttcaag ctgtccttcc ccgagggctt   540
   caggtgggag cgcgtgatga acttcgagga cggcggcatt attcacgtta accaggacag   600
   ttccctgcaa gacggcgtgt tcatctacaa ggtgaagctg cgcggcacca acttccccag   660
   tgacggcccc gtaatgcaga agaagaccat gggctgggag gccagtgagg agcggatgta   720
   ccccgaggac ggcgccctga agtctgagat caaaaagagg ctgaagctga aggacggcgg   780
   ccactacgcc gccgaggtca agaccaccta caaggccaag aagcccgtgc agctgcccgg 840
   cgcctacatc gtcgacatca agttggacat cgtgtcccac aacgaggact acaccatcgt 900
   ggaacagtac gaacgcgccg agggccgcca ctccaccggc ggcatggacg agctgtacaa 960
   ggcctaacca ggcatcaaat aaaacgaaag gctcagtcga aagactgggc ctttcgtttt 1020
   atctgttgtt tgtcggtgaa cgctctctac tagagtcaca ctggctcacc ttcgggtggg 1080
   cctttctgcg tttatagccc gaagagcacg tactgctaag ctgactgcgg gggagagcgc 1140
   gtgtccgctg cagtccggca aaaaagggca aggtgtcacc accctgccct ttttctttaa 1200
   aaccgaaaag attacttcgc gttatgcagg cttcctcgct cactgactcg ctgcgctcgg 1260
   tcgttcggct gcggcgagcg gtatcagctc actcaaaggc ggtaatacgg ttatccacag 1320
   aatcagggga taacgcagga aagaacatgt gagcaaaagg ccagcaaaag gccaggaacc 1380
   gtaaaaaggc cgcgttgctg gcgtttttcc acaggctccg cccccctgac gagcatcaca 1440
   aaaatcgacg ctcaagtcag aggtggcgaa acccgacagg actataaaga taccaggcgt 1500
   ttccccctgg aagctccctc gtgcgctctc ctgttccgac cctgccgctt accggatacc 1560
   tgtccgcctt tctcccttcg ggaagcgtgg cgctttctca tagctcacgc tgtaggtatc 1620
   tcagttcggt gtaggtcgtt cgctccaagc tgggctgtgt gcacgaaccc cccgttcagc 1680
   ccgaccgctg cgccttatcc ggtaactatc gtcttgagtc caacccggta agacacgact 1740
   tatcgccact ggcagcagcc actggtaaca ggattagcag agcgaggtat gtaggcggtg 1800
   ctacagagtt cttgaagtgg tggcctaact acggctacac tagaagaaca gtatttggta 1860
   tctgcgctct gctgaagcca gttaccttcg gaaaaagagt tggtagctct tgatccggca 1920
   aacaaaccac cgctggtagc ggtggttttt ttgtttgcaa gcagcagatt acgcgcagaa 1980
   aaaaaggatc tcaagaagat cctttgatct tttctacggg gtctgacgct cagtggaacg 2040
   aaaactcacg ttaagggatt ttggtcatga gattatcaaa aaggatcttc acctagatcc 2100
   ttttaaatta aaaatgaagt tttaaatcaa tctaaagtat atatgagtaa acttggtctg 2160
   acagctcgag gcttggattc tcaccaataa aaaacgcccg gcggcaaccg agcgttctga 2220
   acaaatccag atggagttct gaggtcatta ctggatctat caacaggagt ccaagcgagc 2280
   tcgatatcaa attacgcccc gccctgccac tcatcgcagt actgttgtaa ttcattaagc 2340
   attctgccga catggaagcc atcacaaacg gcatgatgaa cctgaatcgc cagcggcatc 2400
   agcaccttgt cgccttgcgt ataatatttg cccatggtga aaacgggggc gaagaagttg 2460
   tccatattgg ccacgtttaa atcaaaactg gtgaaactca cccagggatt ggctgagacg 2520
   aaaaacatat tctcaataaa ccctttaggg aaataggcca ggttttcacc gtaacacgcc 2580
   acatcttgcg aatatatgtg tagaaactgc cggaaatcgt cgtggtattc actccagagc 2640
   gatgaaaacg tttcagtttg ctcatggaaa acggtgtaac aagggtgaac actatcccat 2700
   atcaccagct caccgtcttt cattgccata cgaaattccg gatgagcatt catcaggcgg 2760
   gcaagaatgt gaataaaggc cggataaaac ttgtgcttat ttttctttac ggtctttaaa 2820
   aaggccgtaa tatccagctg aacggtctgg ttataggtac attgagcaac tgactgaaat 2880
   gcctcaaaat gttctttacg atgccattgg gatatatcaa cggtggtata tccagtgatt 2940
   tttttctcca ttttagcttc cttagctcct gaaaatctcg ataactcaaa aaatacgccc 3000
   ggtagtgatc ttatttcatt atggtgaaag ttggaacctc ttacgtgccc gatcaactcg 3060
   agtgccactt gacgtctaag aaaccattat tatcatgaca ttaacctata aaaataggcg 3120
   tatcacgagg cagaatttca gataaaaaaa atccttagct ttcgctaagg atgatttctg 3180
   gaattcgcgg ccgcttctag agcc 3204
<210> 9
   <211> 2684
   <212> DNA
   <213> Unknown
<220>
   <223> Synthetic
<400> 9
   mcacagctaa caccacgtcg tccctatctg ctgccctagg tctatgagtg gttgctggat      60
   aactttacgg gcatgcataa ggctcgtaat atatattcag ggagaccaca acggtttccc     120
   tctacaaata attttgttta acttttcaca caggaaagta ctagatgtcg agattagata     180
   aaagtaaagt gattaacagc gcattagagc tgcttaatga ggtcggaatc gaaggtttaa     240
   caacccgtaa actcgcccag aagctaggtg tagagcagcc tacattgtat tggcatgtaa     300
   aaaataagcg ggctttgctc gacgccttag ccattgagat gttagatagg caccatactc     360
   acttttgccc tttagaaggg gaaagctggc aagatttttt acgtaataac gctaaaagtt     420
   ttagatgtgc tttactaagt catcgcgatg gagcaaaagt acatttaggt acacggccta     480
   cagaaaaaca gtatgaaact ctcgaaaatc aattagcctt tttatgccaa caaggttttt     540
   cactagagaa tgcattatat gcactcagcg ctgtggggca ttttacttta ggttgcgtat     600
   tggaagatca agagcatcaa gtcgctaaag aagaaaggga aacacctact actgatagta     660
   tgccgccatt attacgacaa gctatcgaat tatttgatca ccaaggtgca gagccagcct     720
   tcttattcgg ccttgaattg atcatatgcg gattagaaaa acaacttaaa tgtgaaagtg     780
   ggtcgtaagc ctcacacagg aaagtactag atgaaatcta acaatgcgct catcgtcatt     840
   ctcggcaccg tcaccctgga cgctgtaggc ataggcttgg ttatgccggt actgccgggc     900
   ctcttgcggg atatcgtcca ttccgacagt attgccagtc actatggcgt gctgcttgcg     960
   ctctatgcgt tgatgcaatt tctttgcgca cccgttctcg gagccctgtc cgaccgcttt    1020
   ggccgccgtc cagtcctgct cgcttcgctc cttggagcca ctatcgacta cgcgatcatg 1080
   gcgaccacac ccgtcctgtg gattctctac gccggacgca tcgtggcggg catcacgggt 1140
   gccacaggtg cggttgctgg tgcctatatc gccgacatca ccgatgggga agatcgggct 1200
   cgccacttcg ggctcatgag cgcttgtttc ggcgtgggta tggtggcagg ccccgtggcc 1260
   gggggactgt tgggtgccat ctccttgcat gcaccattcc ttgcggcggc ggtgctcaac 1320
   ggcctcaacc tcctcctggg ctgcttcctt atgcaggaat cgcataaggg agagcgccgt 1380
   ccgatgccct tgcgtgcctt caatccagtc agctccttcc ggtgggcgcg gggcatgact 1440
   atcgtcgccg cacttatgac tgttttcttt atcatgcaac tcgtaggaca ggttccggca 1500
   gcgctctggg tcattttcgg cgaggaccgc tttcgctgga gcgcgacgat gatcggcctg 1560
   tcgcttgcgg tattcggaat cttgcacgcc ctcgctcaag ccttcgtcac gggccccgcc 1620
   accaaacgtt tcggcgagaa gcaggccatt atcgcgggca tggcggccga cgcgctgggc 1680
   tacgtcttgc tggcgttcgc gacgcgcggc tggatggcct tccccattat gattcttctc 1740
   gcttccggcg gcatcggtat gcccgcgttg caggccatgc tgtcccgcca agtagatgac 1800
   gaccatcagg gacagcttca agggtcgctc gcggctctta ccagcctcac ttcgatcatt 1860
   ggaccgctga tcgtcacggc gatttatgcc gcctcggcga gcacatggaa cgggttggca 1920
   tggattgtag gtgccgccct ttaccttgtc tgcctccccg cgttgcgtcg cggtgcatgg 1980
   agccgggcca cctcgaccta ataagccccc gtagaaaaga tcaaaagatc ttcttgagat 2040
   cctttttttc tgcgcgtaat ctgctacttg caaacaaaaa aaccaccgct accagcggtg 2100
   gtttgtttgc cggatcaaga gctaccaact ctttttccga aggtaactgg cttcagcaga 2160
   gcgcagatac caaatactgt tcttctagtg tagccgtagt taggccacca cttcaagaac 2220
   tctgtagcac cgcctacata cctcgctctg ctaatcctgt taccagtggc tgctgccagt 2280
   ggcgataagt cgtgtcttac cgggttggac tcaagacgat agttaccgga taaggcgcag 2340
   cggtcgggct gaacgggggg ttcgtgcaca cagcccagct tggagcgaac gacctacacc 2400
   gaactgagat acctacagcg tgagctatga gaaagcgcca cgcttcccga agggagaaag 2460
   gcggacaggt atccggtaag cggcagggtc ggaacaggag agcgcacgag ggagcttcca 2520
   gggggaaacg cctggtatct ttatagtcct gtcgggtttc gccacctctg acttgagcgt 2580
   cgatttttgt gatgctcgtc aggggggcgg agcctgtgga aaaacgccag caacgcggcc 2640
   tttttacggt tcctggcctt ttgctggcct tttgctcaca tgcc 2684
<210> 10
   <211> 2173
   <212> **DNA**
   <213> Unknown
<220>
   <223> Synthetic
<400> 10
   maattgtgag cggataacaa ttacgagctt catgcacagt gaaatcatga aaaatttatt      60
   tgctttgtga gcggataaca attataatat gtggaattgt gagcgctcac aattccacaa     120
   cggtttccct ctagaaataa ttttgtttaa cttttgccgt gaataccacg acgatttcgt     180
   tttagagcta gaaatagcaa gttaaaataa ggctagtccg ttatcaactt gaaaaagtgg     240
   caccgagtcg gtggtgcgcc ccaggcatca aataaaacga aaggctcagt cgaaagactg     300
   ggcctttcgt tttatctgtt gtttgtcggt gaacgctctc tactagagtc acactggctc     360
   accttcgggt gggcctttct gcgtttatag cccacagcta acaccacgtc gtccctatct     420
   gctgccctag gtctatgagt ggttgctgga taactttacg ggcatgcata aggctcgtaa     480
   tatatattca gggagaccac aacggtttcc ctctacaaat aattttgttt aacttttcac     540
   acaggaaacc tactagatga gtattcaaca tttccgtgtc gcccttattc ccttttttgc     600
   ggcattttgc cttcctgttt ttgctcaccc agaaacgctg gtgaaagtaa aagatgccga     660
   agatcagttg ggtgcacgtg tgggttacat cgaactggac ctcaacagcg gtaagattct     720
   tgagagtttt cgccccgaag aacgtttccc aatgatgagc acttttaaag ttctgctctg     780
   tggcgcggta ttatcccgta ttgacgccgg gcaagagcaa ctcggtcgcc gcatacacta     840
   ttctcagaat gacttggttg agtactcacc agtcacagaa aagcatctta cggacggcat     900
   gacagtacgc gaattatgca gcgctgccat aaccatgagt gataacacgg cggccaactt     960
   acttctgaca acgatcggag gaccgaagga gcttaccgct tttttgcaca acatgggtga    1020
   tcatgtaact cgccttgatc gttgggaacc ggagctgaat gaagccatac caaacgacga    1080
   gcgtgacacc acgatgcctg tagctatggc aacaacgttg cgcaaactct taactggcga    1140
   acttcttact ctcgcttccc ggcaacaatt aatagactgg atggaggcgg ataaagttgc    1200
   aggaccactt ctgcgctcgg cccttccggc tggctggttt attgctgata aatctggagc    1260
   cggtgagcgt gggtcccgcg gtattattgc agccctgggg ccagatggta agccctcccg    1320
   tatcgtagtt atctacacga cggggagcca ggcaactatg gacgaacgta atcgccagat    1380
   cgctgagata ggtgcctccc tgattaagca ttggtaagcc cttccgcttc ctcgctcact    1440
   gactcgctac gctcggtcgt tcgactgcgg cgagcggtgt cagctcactc aaaagcggta    1500
   atacggttat ccacagaatc aggggataaa gccggaaaga acatgtgagc aaaaagcaaa    1560
   gcaccggaag aagccaacgc cgcaggcgtt tttccatagg ctccgccccc ctgacgagca    1620
   tcacaaaaat cgacgctcaa gccagaggtg gcgaaacccg acaggactat aaagatacca    1680
   ggcgtttccc cctggaagct ccctcgtgcg ctctcctgtt ccgaccctgc cgcttaccgg    1740
   atacctgtcc gcctttctcc cttcgggaag cgtggcgctt tctcatagct cacgctgttg    1800
   gtatctcagt tcggtgtagg tcgttcgctc caagctgggc tgtgtgcacg aaccccccgt     1860
   tcagcccgac cgctgcgcct tatccggtaa ctatcgtctt gagtccaacc cggtaagaca     1920
   cgacttatcg ccactggcag cagccattgg taactgattt agaggacttt gtcttgaagt     1980
   tatgcacctg ttaaggctaa actgaaagaa cagattttgg tgagtgcggt cctccaaccc     2040
   acttaccttg gttcaaagag ttggtagctc agcgaacctt gagaaaacca ccgttggtag     2100
   cggtggtttt tctttattta tgagatgatg aatcaatcgg tctatcaagt caacgaacag     2160
   ctattccgtt gcc                                                        2173
<210> 11
   <211> 2073
   <212> DNA
   <213> Unknown
<220>
   <223> Synthetic
<400> 11
   mtccctatca gtgatagaga ttgacatccc tatcagtgat agagatactg agcacgccgt      60
   gaataccacg acgatttcgt tttagagcta gaaatagcaa gttaaaataa ggctagtccg     120
   ttatcaactt gaaaaagtgg caccgagtcg gtggtgcgcc ccaggcatca aataaaacga     180
   aaggctcagt cgaaagactg ggcctttcgt tttatctgtt gtttgtcggt gaacgctctc     240
   tactagagtc acactggctc accttcgggt gggcctttct gcgtttatag cccacagcta     300
   acaccacgtc gtccctatct gctgccctag gtctatgagt ggttgctgga taactttacg     360
   ggcatgcata aggctcgtaa tatatattca gggagaccac aacggtttcc ctctacaaat     420
   aattttgttt aacttttcac acaggaaacc tactagatga gtattcaaca tttccgtgtc     480
   gcccttattc ccttttttgc ggcattttgc cttcctgttt ttgctcaccc agaaacgctg     540
   gtgaaagtaa aagatgccga agatcagttg ggtgcacgtg tgggttacat cgaactggac     600
   ctcaacagcg gtaagattct tgagagtttt cgccccgaag aacgtttccc aatgatgagc     660
   acttttaaag ttctgctctg tggcgcggta ttatcccgta ttgacgccgg gcaagagcaa     720
   ctcggtcgcc gcatacacta ttctcagaat gacttggttg agtactcacc agtcacagaa     780
   aagcatctta cggacggcat gacagtacgc gaattatgca gcgctgccat aaccatgagt     840
   gataacacgg cggccaactt acttctgaca acgatcggag gaccgaagga gcttaccgct     900
   tttttgcaca acatgggtga tcatgtaact cgccttgatc gttgggaacc ggagctgaat     960
   gaagccatac caaacgacga gcgtgacacc acgatgcctg tagctatggc aacaacgttg    1020
   cgcaaactct taactggcga acttcttact ctcgcttccc ggcaacaatt aatagactgg    1080
   atggaggcgg ataaagttgc aggaccactt ctgcgctcgg cccttccggc tggctggttt    1140
   attgctgata aatctggagc cggtgagcgt gggtcccgcg gtattattgc agccctgggg 1200
   ccagatggta agccctcccg tatcgtagtt atctacacga cggggagcca ggcaactatg 1260
   gacgaacgta atcgccagat cgctgagata ggtgcctccc tgattaagca ttggtaagcc 1320
   cttccgcttc ctcgctcact gactcgctac gctcggtcgt tcgactgcgg cgagcggtgt 1380
   cagctcactc aaaagcggta atacggttat ccacagaatc aggggataaa gccggaaaga 1440
   acatgtgagc aaaaagcaaa gcaccggaag aagccaacgc cgcaggcgtt tttccatagg 1500
   ctccgccccc ctgacgagca tcacaaaaat cgacgctcaa gccagaggtg gcgaaacccg 1560
   acaggactat aaagatacca ggcgtttccc cctggaagct ccctcgtgcg ctctcctgtt 1620
   ccgaccctgc cgcttaccgg atacctgtcc gcctttctcc cttcgggaag cgtggcgctt 1680
   tctcatagct cacgctgttg gtatctcagt tcggtgtagg tcgttcgctc caagctgggc 1740
   tgtgtgcacg aaccccccgt tcagcccgac cgctgcgcct tatccggtaa ctatcgtctt 1800
   gagtccaacc cggtaagaca cgacttatcg ccactggcag cagccattgg taactgattt 1860
   agaggacttt gtcttgaagt tatgcacctg ttaaggctaa actgaaagaa cagattttgg 1920
   tgagtgcggt cctccaaccc acttaccttg gttcaaagag ttggtagctc agcgaacctt 1980
   gagaaaacca ccgttggtag cggtggtttt tctttattta tgagatgatg aatcaatcgg 2040
   tctatcaagt caacgaacag ctattccgtt gcc 2073
<210> 12
   <211> 2173
   <212> **DNA**
   <213> Unknown
<220>
   <223> Synthetic
<400> 12
   maattgtgag cggataacaa ttacgagctt catgcacagt gaaatcatga aaaatttatt      60
   tgctttgtga gcggataaca attataatat gtggaattgt gagcgctcac aattccacaa     120
   cggtttccct ctagaaataa ttttgtttaa cttttgccct ataaccagac cgttcagcgt     180
   tttagagcta gaaatagcaa gttaaaataa ggctagtccg ttatcaactt gaaaaagtgg     240
   caccgagtcg gtggtgcgcc ccaggcatca aataaaacga aaggctcagt cgaaagactg     300
   ggcctttcgt tttatctgtt gtttgtcggt gaacgctctc tactagagtc acactggctc     360
   accttcgggt gggcctttct gcgtttatag cccacagcta acaccacgtc gtccctatct     420
   gctgccctag gtctatgagt ggttgctgga taactttacg ggcatgcata aggctcgtaa     480
   tatatattca gggagaccac aacggtttcc ctctacaaat aattttgttt aacttttcac     540
   acaggaaacc tactagatga gtattcaaca tttccgtgtc gcccttattc ccttttttgc    600
   ggcattttgc cttcctgttt ttgctcaccc agaaacgctg gtgaaagtaa aagatgccga     660
   agatcagttg ggtgcacgtg tgggttacat cgaactggac ctcaacagcg gtaagattct      720
   tgagagtttt cgccccgaag aacgtttccc aatgatgagc acttttaaag ttctgctctg      780
   tggcgcggta ttatcccgta ttgacgccgg gcaagagcaa ctcggtcgcc gcatacacta      840
   ttctcagaat gacttggttg agtactcacc agtcacagaa aagcatctta cggacggcat      900
   gacagtacgc gaattatgca gcgctgccat aaccatgagt gataacacgg cggccaactt      960
   acttctgaca acgatcggag gaccgaagga gcttaccgct tttttgcaca acatgggtga     1020
   tcatgtaact cgccttgatc gttgggaacc ggagctgaat gaagccatac caaacgacga     1080
   gcgtgacacc acgatgcctg tagctatggc aacaacgttg cgcaaactct taactggcga     1140
   acttcttact ctcgcttccc ggcaacaatt aatagactgg atggaggcgg ataaagttgc     1200
   aggaccactt ctgcgctcgg cccttccggc tggctggttt attgctgata aatctggagc     1260
   cggtgagcgt gggtcccgcg gtattattgc agccctgggg ccagatggta agccctcccg     1320
   tatcgtagtt atctacacga cggggagcca ggcaactatg gacgaacgta atcgccagat     1380
   cgctgagata ggtgcctccc tgattaagca ttggtaagcc cttccgcttc ctcgctcact     1440
   gactcgctac gctcggtcgt tcgactgcgg cgagcggtgt cagctcactc aaaagcggta     1500
   atacggttat ccacagaatc aggggataaa gccggaaaga acatgtgagc aaaaagcaaa     1560
   gcaccggaag aagccaacgc cgcaggcgtt tttccatagg ctccgccccc ctgacgagca     1620
   tcacaaaaat cgacgctcaa gccagaggtg gcgaaacccg acaggactat aaagatacca     1680
   ggcgtttccc cctggaagct ccctcgtgcg ctctcctgtt ccgaccctgc cgcttaccgg     1740
   atacctgtcc gcctttctcc cttcgggaag cgtggcgctt tctcatagct cacgctgttg     1800
   gtatctcagt tcggtgtagg tcgttcgctc caagctgggc tgtgtgcacg aaccccccgt     1860
   tcagcccgac cgctgcgcct tatccggtaa ctatcgtctt gagtccaacc cggtaagaca     1920
   cgacttatcg ccactggcag cagccattgg taactgattt agaggacttt gtcttgaagt     1980
   tatgcacctg ttaaggctaa actgaaagaa cagattttgg tgagtgcggt cctccaaccc     2040
   acttaccttg gttcaaagag ttggtagctc agcgaacctt gagaaaacca ccgttggtag     2100
   cggtggtttt tctttattta tgagatgatg aatcaatcgg tctatcaagt caacgaacag     2160
   ctattccgtt gcc                                                        2173
<210> 13
   <211> 2448
   <212> DNA
   <213> Unknown
<220>
   <223> Synthetic
<400> 13
   maattgtgag cggataacaa ttacgagctt catgcacagt gaaatcatga aaaatttatt      60
   tgctttgtga gcggataaca attataatat gtggaattgt gagcgctcac aattccacaa     120
   cggtttccct ctagaaataa ttttgtttaa cttttgccgt gaataccacg acgatttcgt     180
   tttagagcta gaaatagcaa gttaaaataa ggctagtccg ttatcaactt gaaaaagtgg     240
   caccgagtcg gtggtgcgcc ccaggcatca aataaaacga aaggctcagt cgaaagactg     300
   ggcctttcgt tttatctgtt gtttgtcggt gaacgctctc tactagagtc acactggctc     360
   accttcgggt gggcctttct gcgtttatag ccttagttag ttagattagc agaaagtcaa     420
   aagcctccga ccggaggctt ttgactaaaa cttcccttgg ggttatcatt ggggctcact     480
   caaaggcggt aatcagataa aaaaaatcct tagctttcgc taaggatgat ttctgccctt      540
   ccgcttcctc gctcactgac tcgctacgct cggtcgttcg actgcggcga gcggtgtcag     600
   ctcactcaaa agcggtaata cggttatcca cagaatcagg ggataaagcc ggaaagaaca     660
   tgtgagcaaa aagcaaagca ccggaagaag ccaacgccgc aggcgttttt ccataggctc     720
   cgcccccctg acgagcatca caaaaatcga cgctcaagcc agaggtggcg aaacccgaca     780
   ggactataaa gataccaggc gtttccccct ggaagctccc tcgtgcgctc tcctgttccg     840
   accctgccgc ttaccggata cctgtccgcc tttctccctt cgggaagcgt ggcgctttct     900
   catagctcac gctgttggta tctcagttcg gtgtaggtcg ttcgctccaa gctgggctgt     960
   gtgcacgaac cccccgttca gcccgaccgc tgcgccttat ccggtaacta tcgtcttgag    1020
   tccaacccgg taagacacga cttatcgcca ctggcagcag ccattggtaa ctgatttaga    1080
   ggactttgtc ttgaagttat gcacctgtta aggctaaact gaaagaacag attttggtga    1140
   gtgcggtcct ccaacccact taccttggtt caaagagttg gtagctcagc gaaccttgag    1200
   aaaaccaccg ttggtagcgg tggtttttct ttatttatga gatgatgaat caatcggtct    1260
   atcaagtcaa cgaacagcta ttccgttgcc ctgcgctagc atgcctattt gtttattttt    1320
   ctaaatacat tcaaatatgt atccgctcat gagacaataa ccctgataaa tgcttcaata    1380
   atattgaaaa aggaacagta tgagtattca acatttccgt gtcgccctta ttcccttttt    1440
   tgcggcattt tgccttcctg tttttgctca cccagaaacg ctggtgaaag taaaagatgc    1500
   cgaagatcag ttgggtgcac gtgtgggtta catcgaactg gacctcaaca gcggtaagat    1560
   tcttgagagt tttcgccccg aagaacgttt cccaatgatg agcactttta aagttctgct    1620
   ctgtggcgcg gtattatccc gtattgacgc cgggcaagag caactcggtc gccgcataca    1680
   ctattctcag aatgacttgg ttgagtactc accagtcaca gaaaagcatc ttacggacgg    1740
   catgacagta cgcgaattat gcagcgctgc cataaccatg agtgataaca cggcggccaa    1800
   cttacttctg acaacgatcg gaggaccgaa ggagcttacc gcttttttgc acaacatggg     1860
   tgatcatgta actcgccttg atcgttggga accggagctg aatgaagcca taccaaacga    1920
   cgagcgtgac accacgatgc ctgtagctat ggcaacaacg ttgcgcaaac tcttaactgg 1980
   cgaacttctt actctcgctt cccggcaaca attaatagac tggatggagg cggataaagt 2040
   tgcaggacca cttctgcgct cggcccttcc ggctggctgg tttattgctg ataaatctgg 2100
   agccggtgag cgtgggtccc gcggtattat tgcagccctg gggccagatg gtaagccctc 2160
   ccgtatcgta gttatctaca cgacggggag ccaggcaact atggacgaac gtaatcgcca 2220
   gatcgctgag ataggtgcct ccctgattaa gcattggtaa gcctccggca aaaaaacggg 2280
   caaggtgtca ccaccctgcc ctttttcttt aaaaccgaaa agattacttc gcgtttgcca 2340
   cctgacgtct aagaaaagga atattcagca atttgcccgt gccgaagaaa ggcccacccg 2400
   tgaaggtgag ccagtgagtt gattgctacg taattagtta gttaggcc 2448
<210> 14
   <211> 2337
   <212> DNA
   <213> Unknown
<220>
   <223> Synthetic
<400> 14
   mtccctatca gtgatagaga ttgacatccc tatcagtgat agagatactg agcacgccgg      60
   gcacgggcag cttaccgggt tttagagcta gaaatagcaa gttaaaataa ggctagtccg     120
   ttatcaactt gaaaaagtgg caccgagtcg gtggtgcgcc ccaggcatca aataaaacga     180
   aaggctcagt cgaaagactg ggcctttcgt tttatctgtt gtttgtcggt gaacgctctc     240
   tactagagtc acactggctc accttcgggt gggcctttct gcgtttatag ccttagttag     300
   ttagattagc agaaagtcaa aagcctccga ccggaggctt ttgactaaaa cttcccttgg     360
   ggttatcatt ggggctcact caaaggcggt aatcagataa aaaaaatcct tagctttcgc     420
   taaggatgat ttctgccgat caaaggatct tcttgagatc ctttttttct gcgcgtaatc    480
   ttttgccctg taaacgaaaa aaccacctgg ggaggtggtt tgatcgaagg ttaagtcagt     540
   tggggaactg cttaaccgtg gtaactggct ttcgcagagc acagcaacca aatctgtcct     600
   tccagtgtag ccggactttg gcgcacactt caagagcaac cgcgtgttta gctaaacaaa     660
   tcctctgcga actcccagtt accaatggct gctgccagtg gcgttttacc gtgcttttcc     720
   gggttggact caagtgaaca gttaccggat aaggcgcagc agtcgggctg aacggggagt     780
   tcttgcttac agcccagctt ggagcgaacg acctacaccg agccgagata ccagtgtgtg     840
   agctatgaga aagcgccaca cttcccgtaa gggagaaagg cggaacaggt atccggtaaa     900
   cggcagggtc ggaacaggag agcgcaagag ggagcgaccc gccggaaacg gtggggatct     960
   ttaagtcctg tcgggtttcg cccgtactgt cagattcatg gttgagcctc acggctccca    1020
   cagatgcacc ggaaaagcgt ctgtttatgt gaactctggc aggagggcgg agcctatgga 1080
   aaaacgccac cggcgcggcc ctgctgtttt gcctcacatg ttagtcccct gcttatccac 1140
   ggaatctgtg ggtaactttg tatgtgtccg cagcgcgccc tgcgctagca tgcctatttg 1200
   tttatttttc taaatacatt caaatatgta tccgctcatg agacaataac cctgataaat 1260
   gcttcaataa tattgaaaaa ggaacagtat gagtattcaa catttccgtg tcgcccttat 1320
   tccctttttt gcggcatttt gccttcctgt ttttgctcac ccagaaacgc tggtgaaagt 1380
   aaaagatgcc gaagatcagt tgggtgcacg tgtgggttac atcgaactgg acctcaacag 1440
   cggtaagatt cttgagagtt ttcgccccga agaacgtttc ccaatgatga gcacttttaa 1500
   agttctgctc tgtggcgcgg tattatcccg tattgacgcc gggcaagagc aactcggtcg 1560
   ccgcatacac tattctcaga atgacttggt tgagtactca ccagtcacag aaaagcatct 1620
   tacggacggc atgacagtac gcgaattatg cagcgctgcc ataaccatga gtgataacac 1680
   ggcggccaac ttacttctga caacgatcgg aggaccgaag gagcttaccg cttttttgca 1740
   caacatgggt gatcatgtaa ctcgccttga tcgttgggaa ccggagctga atgaagccat 1800
   accaaacgac gagcgtgaca ccacgatgcc tgtagctatg gcaacaacgt tgcgcaaact 1860
   cttaactggc gaacttctta ctctcgcttc ccggcaacaa ttaatagact ggatggaggc 1920
   ggataaagtt gcaggaccac ttctgcgctc ggcccttccg gctggctggt ttattgctga 1980
   taaatctgga gccggtgagc gtgggtcccg cggtattatt gcagccctgg ggccagatgg 2040
   taagccctcc cgtatcgtag ttatctacac gacggggagc caggcaacta tggacgaacg 2100
   taatcgccag atcgctgaga taggtgcctc cctgattaag cattggtaag cctccggcaa 2160
   aaaaacgggc aaggtgtcac caccctgccc tttttcttta aaaccgaaaa gattacttcg 2220
   cgtttgccac ctgacgtcta agaaaaggaa tattcagcaa tttgcccgtg ccgaagaaag 2280
   gcccacccgt gaaggtgagc cagtgagttg attgctacgt aattagttag ttaggcc 2337
<210> 15
   <211> 2337
   <212> DNA
   <213> Unknown
<220>
   <223> Synthetic
<400> 15
   mtccctatca gtgatagaga ttgacatccc tatcagtgat agagatactg agcacgccgt      60
   gaataccacg acgatttcgt tttagagcta gaaatagcaa gttaaaataa ggctagtccg     120
   ttatcaactt gaaaaagtgg caccgagtcg gtggtgcgcc ccaggcatca aataaaacga     180
   aaggctcagt cgaaagactg ggcctttcgt tttatctgtt gtttgtcggt gaacgctctc     240
   tactagagtc acactggctc accttcgggt gggcctttct gcgtttatag ccttagttag     300
   ttagattagc agaaagtcaa aagcctccga ccggaggctt ttgactaaaa cttcccttgg 360
   ggttatcatt ggggctcact caaaggcggt aatcagataa aaaaaatcct tagctttcgc 420
   taaggatgat ttctgccgat caaaggatct tcttgagatc ctttttttct gcgcgtaatc 480
   ttttgccctg taaacgaaaa aaccacctgg ggaggtggtt tgatcgaagg ttaagtcagt 540
   tggggaactg cttaaccgtg gtaactggct ttcgcagagc acagcaacca aatctgtcct 600
   tccagtgtag ccggactttg gcgcacactt caagagcaac cgcgtgttta gctaaacaaa 660
   tcctctgcga actcccagtt accaatggct gctgccagtg gcgttttacc gtgcttttcc 720
   gggttggact caagtgaaca gttaccggat aaggcgcagc agtcgggctg aacggggagt 780
   tcttgcttac agcccagctt ggagcgaacg acctacaccg agccgagata ccagtgtgtg 840
   agctatgaga aagcgccaca cttcccgtaa gggagaaagg cggaacaggt atccggtaaa 900
   cggcagggtc ggaacaggag agcgcaagag ggagcgaccc gccggaaacg gtggggatct 960
   ttaagtcctg tcgggtttcg cccgtactgt cagattcatg gttgagcctc acggctccca 1020
   cagatgcacc ggaaaagcgt ctgtttatgt gaactctggc aggagggcgg agcctatgga 1080
   aaaacgccac cggcgcggcc ctgctgtttt gcctcacatg ttagtcccct gcttatccac 1140
   ggaatctgtg ggtaactttg tatgtgtccg cagcgcgccc tgcgctagca tgcctatttg 1200
   tttatttttc taaatacatt caaatatgta tccgctcatg agacaataac cctgataaat 1260
   gcttcaataa tattgaaaaa ggaacagtat gagtattcaa catttccgtg tcgcccttat 1320
   tccctttttt gcggcatttt gccttcctgt ttttgctcac ccagaaacgc tggtgaaagt 1380
   aaaagatgcc gaagatcagt tgggtgcacg tgtgggttac atcgaactgg acctcaacag 1440
   cggtaagatt cttgagagtt ttcgccccga agaacgtttc ccaatgatga gcacttttaa 1500
   agttctgctc tgtggcgcgg tattatcccg tattgacgcc gggcaagagc aactcggtcg 1560
   ccgcatacac tattctcaga atgacttggt tgagtactca ccagtcacag aaaagcatct 1620
   tacggacggc atgacagtac gcgaattatg cagcgctgcc ataaccatga gtgataacac 1680
   ggcggccaac ttacttctga caacgatcgg aggaccgaag gagcttaccg cttttttgca 1740
   caacatgggt gatcatgtaa ctcgccttga tcgttgggaa ccggagctga atgaagccat 1800
   accaaacgac gagcgtgaca ccacgatgcc tgtagctatg gcaacaacgt tgcgcaaact 1860
   cttaactggc gaacttctta ctctcgcttc ccggcaacaa ttaatagact ggatggaggc 1920
   ggataaagtt gcaggaccac ttctgcgctc ggcccttccg gctggctggt ttattgctga 1980
   taaatctgga gccggtgagc gtgggtcccg cggtattatt gcagccctgg ggccagatgg 2040
   taagccctcc cgtatcgtag ttatctacac gacggggagc caggcaacta tggacgaacg 2100
   taatcgccag atcgctgaga taggtgcctc cctgattaag cattggtaag cctccggcaa 2160
   aaaaacgggc aaggtgtcac caccctgccc tttttcttta aaaccgaaaa gattacttcg     2220
   cgtttgccac ctgacgtcta agaaaaggaa tattcagcaa tttgcccgtg ccgaagaaag     2280
   gcccacccgt gaaggtgagc cagtgagttg attgctacgt aattagttag ttaggcc        2337
<210> 16
   <211> 2337
   <212> DNA
   <213> Unknown
<220>
   <223> Synthetic
<400> 16
   mtccctatca gtgatagaga ttgacatccc tatcagtgat agagatactg agcacgccct      60
   ataaccagac cgttcagcgt tttagagcta gaaatagcaa gttaaaataa ggctagtccg     120
   ttatcaactt gaaaaagtgg caccgagtcg gtggtgcgcc ccaggcatca aataaaacga     180
   aaggctcagt cgaaagactg ggcctttcgt tttatctgtt gtttgtcggt gaacgctctc     240
   tactagagtc acactggctc accttcgggt gggcctttct gcgtttatag ccttagttag     300
   ttagattagc agaaagtcaa aagcctccga ccggaggctt ttgactaaaa cttcccttgg     360
   ggttatcatt ggggctcact caaaggcggt aatcagataa aaaaaatcct tagctttcgc     420
   taaggatgat ttctgccgat caaaggatct tcttgagatc ctttttttct gcgcgtaatc    480
   ttttgccctg taaacgaaaa aaccacctgg ggaggtggtt tgatcgaagg ttaagtcagt     540
   tggggaactg cttaaccgtg gtaactggct ttcgcagagc acagcaacca aatctgtcct     600
   tccagtgtag ccggactttg gcgcacactt caagagcaac cgcgtgttta gctaaacaaa     660
   tcctctgcga actcccagtt accaatggct gctgccagtg gcgttttacc gtgcttttcc     720
   gggttggact caagtgaaca gttaccggat aaggcgcagc agtcgggctg aacggggagt    780
   tcttgcttac agcccagctt ggagcgaacg acctacaccg agccgagata ccagtgtgtg     840
   agctatgaga aagcgccaca cttcccgtaa gggagaaagg cggaacaggt atccggtaaa     900
   cggcagggtc ggaacaggag agcgcaagag ggagcgaccc gccggaaacg gtggggatct     960
   ttaagtcctg tcgggtttcg cccgtactgt cagattcatg gttgagcctc acggctccca    1020
   cagatgcacc ggaaaagcgt ctgtttatgt gaactctggc aggagggcgg agcctatgga    1080
   aaaacgccac cggcgcggcc ctgctgtttt gcctcacatg ttagtcccct gcttatccac    1140
   ggaatctgtg ggtaactttg tatgtgtccg cagcgcgccc tgcgctagca tgcctatttg    1200
   tttatttttc taaatacatt caaatatgta tccgctcatg agacaataac cctgataaat    1260
   gcttcaataa tattgaaaaa ggaacagtat gagtattcaa catttccgtg tcgcccttat    1320
   tccctttttt gcggcatttt gccttcctgt ttttgctcac ccagaaacgc tggtgaaagt    1380
   aaaagatgcc gaagatcagt tgggtgcacg tgtgggttac atcgaactgg acctcaacag    1440
   cggtaagatt cttgagagtt ttcgccccga agaacgtttc ccaatgatga gcacttttaa 1500
   agttctgctc tgtggcgcgg tattatcccg tattgacgcc gggcaagagc aactcggtcg 1560
   ccgcatacac tattctcaga atgacttggt tgagtactca ccagtcacag aaaagcatct 1620
   tacggacggc atgacagtac gcgaattatg cagcgctgcc ataaccatga gtgataacac 1680
   ggcggccaac ttacttctga caacgatcgg aggaccgaag gagcttaccg cttttttgca 1740
   caacatgggt gatcatgtaa ctcgccttga tcgttgggaa ccggagctga atgaagccat 1800
   accaaacgac gagcgtgaca ccacgatgcc tgtagctatg gcaacaacgt tgcgcaaact 1860
   cttaactggc gaacttctta ctctcgcttc ccggcaacaa ttaatagact ggatggaggc 1920
   ggataaagtt gcaggaccac ttctgcgctc ggcccttccg gctggctggt ttattgctga 1980
   taaatctgga gccggtgagc gtgggtcccg cggtattatt gcagccctgg ggccagatgg 2040
   taagccctcc cgtatcgtag ttatctacac gacggggagc caggcaacta tggacgaacg 2100
   taatcgccag atcgctgaga taggtgcctc cctgattaag cattggtaag cctccggcaa 2160
   aaaaacgggc aaggtgtcac caccctgccc tttttcttta aaaccgaaaa gattacttcg 2220
   cgtttgccac ctgacgtcta agaaaaggaa tattcagcaa tttgcccgtg ccgaagaaag 2280
   gcccacccgt gaaggtgagc cagtgagttg attgctacgt aattagttag ttaggcc 2337
<210> 17
   <211> 13367
   <212> DNA
   <213> Unknown
<220>
   <223> Synthetic
<400> 17
   aattgtgagc ggataacaat tacgagcttc atgcacagtg aaatcatgaa aaatttattt      60
   gctttgtgag cggataacaa ttataatatg tggaattgtg agcgctcaca attccacaac     120
   ggtttccctc tagaaataat tttgtttaac ttttgcccac cggggtggtg cccatccgtt     180
   ttagagctag aaatagcaag ttaaaataag gctagtccgt tatcaacttg aaaaagtggc     240
   accgagtcgg tggtgcgccc caggcatcaa ataaaacgaa aggctcagtc gaaagactgg     300
   gcctttcgtt ttatctgttg tttgtcggtg aacgctctct actagagtca cactggctca     360
   ccttcgggtg ggcctttctg cgtttatagc ccacagcaaa caccacgtcg accctatcag     420
   ctgcgtgctt tctatgagtc gttgctgcat aacttgacaa ttaatcatcc ggctcgtaat     480
   gtttgtggag ccgggcccaa gttcacttaa aaaggagatc aacaatgaaa gcaattttcg     540
   tactgaaaca tcttaatcat gctaaggagg ttttctaatg gtaagcaagg gcgaggagaa     600
   taacatggcc atcatcaagg agttcatgcg cttcaaggtg cacatggagg gcagtgtgaa     660
   cggccacgag ttcgagatcg agggcgaggg cgagggccgt ccctacgagg cctttcagac 720
   cgctaagctg aaggtgacca agggtggccc cctgcccttc gcctgggaca tcctgtcccc 780
   tcagttcatg tacggctcca aggtctacat taagcaccca gccgacatcc ccgactactt 840
   caagctgtcc ttccccgagg gcttcaggtg ggagcgcgtg atgaacttcg aggacggcgg 900
   cattattcac gttaaccagg acagttccct gcaagacggc gtgttcatct acaaggtgaa 960
   gctgcgcggc accaacttcc ccagtgacgg ccccgtaatg cagaagaaga ccatgggctg 1020
   ggaggccagt gaggagcgga tgtaccccga ggacggcgcc ctgaagtctg agatcaaaaa 1080
   gaggctgaag ctgaaggacg gcggccacta cgccgccgag gtcaagacca cctacaaggc 1140
   caagaagccc gtgcagctgc ccggcgccta catcgtcgac atcaagttgg acatcgtgtc 1200
   ccacaacgag gactacacca tcgtggaaca gtacgaacgc gccgagggcc gccactccac 1260
   cggcggcatg gacgagctgt acaaggccga caagaaatac tcgatagggc tggatatcgg 1320
   gaccaactcc gtcggttggg ctgttatcac ggatgaatat aaggtgccca gcaaaaagtt 1380
   caaggtgcta ggtaacaccg accggcacag tatcaaaaaa aacttgatag gagcgttgct 1440
   gtttgacagt ggtgagaccg ctgaagctac gcgccttaaa aggaccgcgc gccgtagata 1500
   tacccgcagg aagaaccgca tttgctatct ccaagagatt ttttcgaacg agatggctaa 1560
   agtagatgat agtttctttc atcgattgga agaaagcttt ttagttgaag aagacaagaa 1620
   gcatgaacgc catccaattt ttggtaacat agtagatgaa gtggcgtatc atgagaaata 1680
   tccgacgatc tatcatctac gaaaaaaatt agtagatagc acggataaag ctgatttacg 1740
   gctgatctat ttagcactgg ctcatatgat taagtttcgc ggccattttt taattgaggg 1800
   tgatctgaac ccagataaca gtgatgttga caaactcttt atccaattag tacagactta 1860
   caaccagctg tttgaagaaa atccaattaa tgccagtggt gtagatgcga aagctatttt 1920
   gagcgcccga ttaagtaaat cgcgtcgact ggaaaacctt attgcgcaac ttcctggcga 1980
   gaagaaaaac gggctgtttg gaaaccttat tgcgttatcg ttaggcttaa ctccaaactt 2040
   taaatcgaac tttgatttag ccgaagatgc gaaactgcaa ttgtcgaaag atacgtacga 2100
   tgatgatctg gataacctgt tagctcagat tggtgatcag tatgcggatt tatttttagc 2160
   cgcgaagaac ctgtcggatg cgattctgtt gtcggatatc ctccgtgtaa acacggaaat 2220
   aacgaaggcg cctctctcgg cgtcgatgat taaacggtac gatgaacatc atcaggactt 2280
   aacgttgctg aaagcgctgg tgcgacagca gttgccggaa aagtataaag aaatcttttt 2340
   tgatcagtcg aaaaatggtt atgccggcta tattgatgga ggtgcgtccc aggaagaatt 2400
   ttataaattt atcaaaccga ttctggaaaa aatggatggc acggaggaac tgttggttaa 2460
   actcaaccgc gaagatttgc tacggaagca gaggactttt gacaatggga gcattcctca 2520
   tcagattcac ttgggcgagc tacatgcgat tttgcgtcgt caggaagact tttatccgtt 2580
   tctgaaagac aaccgcgaga agattgaaaa aatcttgacg tttcgaatcc catattatgt 2640
   gggcccgtta gctcgcggga acagtcgctt tgcctggatg acgaggaagt cggaagaaac 2700
   cattactccg tggaactttg aagaagtggt cgataaaggc gcgtcggcgc agtcgtttat 2760
   tgaacggatg accaattttg ataaaaactt gccgaacgaa aaagtactcc cgaaacatag 2820
   tttattgtat gagtatttta cagtgtataa tgaattaacc aaggtcaaat atgtgacgga 2880
   aggtatgcga aaaccggcct ttttgtcggg cgaacaaaag aaagcaattg tggatctgct 2940
   tttcaaaacc aaccgaaaag taactgtgaa gcagctgaaa gaagattatt tcaaaaaaat 3000
   agaatgcttt gatagtgtgg aaatttcggg tgtggaagat cgttttaacg cgtcgctggg 3060
   cacttaccat gatttactca aaattattaa agataaagat tttttagata acgaagaaaa 3120
   cgaagatatc ctggaggata ttgtgctgac cttaactctg tttgaagata gagagatgat 3180
   tgaggaacgt ttgaaaacct atgcgcacct ttttgatgat aaggttatga aacaattgaa 3240
   acgccggcgc tatacgggct ggggtcgctt aagccgaaaa ttaattaacg gcattagaga 3300
   taagcagagc gggaaaacca tactggattt tttaaaatcg gatggctttg caaaccggaa 3360
   ctttatgcaa ctaatccatg atgatagttt aacctttaaa gaagacattc agaaagccca 3420
   ggttagcggt cagggggata gtctgcatga acatattgcc aacctggcgg gctccccagc 3480
   gattaaaaaa ggcattctgc aaacggtaaa agtggtggat gaattagtca aagtaatggg 3540
   aaggcataag ccggaaaaca tcgtgattga aatggcccgc gaaaaccaaa ccacgcagaa 3600
   ggggcaaaaa aactcacgag agcgcatgaa acgaatcgaa gaaggcatca aagaactggg 3660
   tagtcaaatt ttgaaagagc atccagtgga aaacacgcag ttacagaacg aaaagcttta 3720
   tctttattat cttcagaacg gtcgtgacat gtatgttgac caggaactgg atattaaccg 3780
   cctgagtgat tatgatgtcg atcacattgt gccgcagagt ttcttgaaag acgattcgat 3840
   agacaacaag gtcctgacac gcagcgataa aaaccgcggc aaatcagata atgtgccgag 3900
   tgaagaagta gtcaaaaaga tgaaaaatta ttggcgtcag ttgctcaatg caaagctgat 3960
   cacgcagcgc aagtttgata acctgacaaa agcggaacgc ggtggcttaa gtgaattgga 4020
   taaagcgggc tttatcaaac ggcagttagt ggaaacgcgg cagatcacga agcatgttgc 4080
   ccagatttta gatagtcgga tgaacacgaa atacgatgaa aacgataaat tgattcgaga 4140
   ggtgaaagtt attactctga aaagcaaact ggtgagcgac ttccgaaaag atttccagtt 4200
   ctataaagta cgcgagatta ataactacca tcatgcacat gatgcttatc tcaacgcagt 4260
   cgtgggtacg gcgttaatta agaaatatcc gaaattggaa tcagagtttg tctatggcga 4320
   ttataaagtg tatgatgtgc gcaaaatgat tgcgaagtcg gagcaggaaa tagggaaagc 4380
   cactgccaaa tatttctttt acagcaacat catgaatttc ttcaaaaccg aaattacctt 4440
   ggccaacggt gagattcgga aacggccact catcgaaacg aacggagaaa cgggtgaaat 4500
   tgtctgggat aaaggacgag attttgcaac cgttcggaaa gtattgtcca tgcctcaggt 4560
   caacattgtc aagaaaaccg aagtacaaac cgggggtttc tccaaggagt cgattctgcc 4620
   gaaacgcaac tcagacaagt tgattgcgcg caaaaaagac tgggatccga aaaaatatgg 4680
   cggcttcgat tccccgacag tagcgtactc ggtcctcgtc gtggcgaagg tcgaaaaagg 4740
   gaaatccaag aagctgaaat ccgtgaaaga gctgctcggg atcaccatca tggagcgctc 4800
   ctccttcgag aagaacccca tcgacttcct ggaggcgaag ggctacaagg aggtgaagaa 4860
   ggacctgatc atcaagctcc ccaagtactc cctctttgag ctggagaacg gccgcaagag 4920
   gatgctcgca agtgcaggtg aattacaaaa aggtaatgaa ctagcgctac cgtccaaata 4980
   tgttaacttt ctgtatctgg cgagtcatta tgaaaagtta aagggcagtc cggaagataa 5040
   tgaacagaaa cagttatttg ttgagcaaca taagcattat ctggatgaga ttattgagca 5100
   gatcagtgaa tttagcaagc gcgtgattct ggccgatgca aacctggata aagtgttgag 5160
   tgcctataat aaacatcgtg acaaaccgat acgcgaacag gccgaaaaca ttattcatct 5220
   gtttacatta acaaacttgg gtgcgcctgc ggcgtttaaa tattttgata ccaccattga 5280
   tcgcaaacga tatacaagca ccaaagaagt gctggatgca acgttgatcc atcagtctat 5340
   cacgggcttg tatgaaaccc ggattgattt aagtcaactc ggtggcgact aagcccggcc 5400
   gctactagta acaaaaaacc cctagccccc cgttttatat cactgcccgc tttccagtcg 5460
   ggaaacctgt cgtgccagct gcattaatga atcggccaac gcgcggggag aggcggtttg 5520
   cgtattgggc gccagggtgg tttttctttt caccagtgag actggcaaca gctgattgcc 5580
   cttcaccgcc tggccctgag agagttgcag caagcggtcc acgctggttt gccccagcag 5640
   gcgaaaatcc tgtttgatgg tggttaacgg cgggatataa catgagctat cttcggtatc 5700
   gtcgtatccc actaccgaga tatccgcacc aacgcgcagc ccggactcgg taatggcgcg 5760
   cattgcgccc agcgccatct gatcgttggc aaccagcatc gcagtgggaa cgatgccctc 5820
   attcagcatt tgcatggttt gttgaaaacc ggacatggca ctccagtcgc cttcccgttc 5880
   cgctatcggc tgaatttgat tgcgagtgag atatttatgc cagccagcca gacgcagacg 5940
   cgccgagaca gaacttaatg ggcccgctaa cagcgcgatt tgctggtgac ccaatgcgac 6000
   cagatgctcc acgcccagtc gcgtaccgtc ctcatgggag aaaataatac tgttgatggg 6060
   tgtctggtca gagacatcaa gaaataacgc cggaacatta gtgcaggcag cttccacagc 6120
   aatggcatcc tggtcatcca gcggatagtt aatgatcagc ccactgacgc gttgcgcgag 6180
   aagattgtgc accgccgctt tacaggcttc gacgccgctt cgttctacca tcgacaccac 6240
   cacgctggca cccagttgat cggcgcgaga tttaatcgcc gcgacaattt gcgacggcgc 6300
   gtgcagggcc agactggagg tggcaacgcc aatcagcaac gactgtttgc ccgccagttg 6360
   ttgtgccacg cggttgggaa tgtaattcag ctccgccatc gccgcttcca ctttttcccg 6420
   cgttttcgca gaaacgtggc tggcctggtt caccacgcgg gaaacggtct gataagagac 6480
   accggcatac tctgcgacat cgtataacgt tactggtttc atattcacca ccctgaattg 6540
   actgtcttcc gggcgctatc atgccatacc gcgaaaggtt ttgcgccatt cgatggcgcg 6600
   ccgcgccctg cagcccgggg gatccactag ttctagagcg gccgccaccg cggtggagct 6660
   ccaattcgcc ctatagtgag tcgtattacg cgcgctcact ggccgtcgtt ttacaacgtc 6720
   gtgactggga aaaccctggc gttacccaac ttaatcgcct tgcagcacat ccccctttcg 6780
   ccagctggcg taatagcgaa gaggcccgca ccgatcgccc ttcccaacag ttgcgcagcc 6840
   tgaatggcga atgggacgcg ccctgtagcg gcgcattaag cgcggcgggt gtggtggtta 6900
   cgcgcagcgt gaccgctaca cttgccagcg ccctagcgcc cgctcctttc gctttcttcc 6960
   cttcctttct cgccacgttc gccggctttc cccgtcaagc tctaaatcgg gggctccctt 7020
   tagggttccg atttagtgct ttacggcacc tcgaccccaa aaaacttgat tagggtgatg 7080
   gttcacgtag tgggccatcg ccctgataga cggtttttcg ccctttgacg ttggagtcca 7140
   cgttctttaa tagtggactc ttgttccaaa ctggaacaac actcaaccct atctcggtct 7200
   attcttttga tttataaggg attttgccga tttcggccta ttggttaaaa aatgagctga 7260
   tttaacaaaa atttaacgcg aattttaaca aaatattaac gcttacaatt taggtggcac 7320
   ttttcgggga aatgtgcgcg gaacccctat ttgtttattt ttctaaatac attcaaatat 7380
   gtatccgctc atgagacaat aaccctgata aatgcttcaa taatattgaa aaaggaagag 7440
   tatgagtatt caacatttcc gtgtcgccct tattcccttt tttgcggcat tttgccttcc 7500
   tgtttttgct cacccagaaa cgctggtgaa agtaaaagat gctgaagatc agttgggtgc 7560
   acgagtgggt tacatcgaac tggatctcaa cagcggtaag atccttgaga gttttcgccc 7620
   cgaagaacgt tttccaatga tgagcacttt taaagttctg ctatgtggcg cggtattatc 7680
   ccgtattgac gccgggcaag agcaactcgg tcgccgcata cactattctc agaatgactt 7740
   ggttgagtac tcaccagtca cagaaaagca tcttacggat ggcatgacag taagagaatt 7800
   atgcagtgct gccataacca tgagtgataa cactgcggcc aacttacttc tgacaacgat 7860
   cggaggaccg aaggagctaa ccgctttttt gcacaacatg ggggatcatg taactcgcct 7920
   tgatcgttgg gaaccggagc tgaatgaagc cataccaaac gacgagcgtg acaccacgat 7980
   gcctgtagca atggcaacaa cgttgcgcaa actattaact ggcgaactac ttactctagc 8040
   ttcccggcaa caattaatag actggatgga ggcggataaa gttgcaggac cacttctgcg 8100
   ctcggccctt ccggctggct ggtttattgc tgataaatct ggagccggtg agcgtgggtc 8160
   tcgcggtatc attgcagcac tggggccaga tggtaagccc tcccgtatcg tagttatcta 8220
   cacgacgggg agtcaggcaa ctatggatga acgaaataga cagatcgctg agataggtgc 8280
   ctcactgatt aagcattggt aactgtcaga ccaagtttac tcatatatac tttagattga 8340
   tttaaaactt catttttaat ttaaaaggat ctaggtgaag atcctttttg ataatctcat 8400
   gaccaaaatc ccttaacgtg agttttcgtt ccactgagcg tcagaccccg tagaaaagat 8460
   caaaggatct tcttgagatc ctttttttct gcgcgtaatc tgctgcttgc aaacaaaaaa 8520
   accaccgcta ccagcggtgg tttgtttgcc ggatcaagag ctaccaactc tttttccgaa 8580
   ggtaactggc ttcagcagag cgcagatacc aaatactgtc cttctagtgt agccgtagtt 8640
   aggccaccac ttcaagaact ctgtagcacc gcctacatac ctcgctctgc taatcctgtt 8700
   accagtggct gctgccagtg gcgataagtc gtgtcttacc gggttggact caagacgata 8760
   gttaccggat aaggcgcagc ggtcgggctg aacggggggt tcgtgcacac agcccagctt 8820
   ggagcgaacg acctacaccg aactgagata cctacagcgt gagctatgag aaagcgccac 8880
   gcttcccgaa gggagaaagg cggacaggta tccggtaagc ggcagggtcg gaacaggaga 8940
   gcgcacgagg gagcttccag ggggaaacgc ctggtatctt tatagtcctg tcgggtttcg 9000
   ccacctctga cttgagcgtc gatttttgtg atgctcgtca ggggggcgga gcctatggaa 9060
   aaacgccagc aacgcggcct ttttacggtt cctggccttt tgctggcctt ttgctcacat 9120
   gttctttcct gcgttatccc ctgattctgt ggataaccgt attaccgcct ttgagtgagc 9180
   tgataccgct cgccgcagcc gaacgaccga gcgcagcgag tcagtgagcg aggaagcgga 9240
   agagcgccca atacgcaaac cgcctctccc cgcgcgttgg ccgattcatt aatgcagctg 9300
   gcacgacagg tttcccgact ggaaagcggg cagtgagcgc aacgcaatta atgtgagtta 9360
   gctcactcat taggcacccc aggctttaca ctttatgctt ccggctcgta tgttgtgtgg 9420
   aattgtgagc ggataacaat ttcacacagg aaacagctat gaccatgatt acgccaagcg 9480
   cgcaattaac cctcactaaa gggaacaaaa gctgggtacc gggccccccc tcgaggtcga 9540
   cggatctttt ccgctgcata accctgcttc ggggtcatta tagcgatttt ttcggtatat 9600
   ccatcctttt tcgcacgata tacaggattt tgccaaaggg ttcgtgtaga ctttccttgg 9660
   tgtatccaac ggcgtcagcc gggcaggata ggtgaagtag gcccacccgc gagcgggtgt 9720
   tccttcttca ctgtccctta ttcgcacctg gcggtgctca acgggaatcc tgctctgcga 9780
   ggctggccgg ctaccgccgg cgtaacagat gagggcaagc ggatggctga tgaaaccaag 9840
   ccaaccagga agggcagccc acctatcaag gtgtactgcc ttccagacga acgaagagcg 9900
   attgaggaaa aggcggcggc ggccggcatg agcctgtcgg cctacctgct ggccgtcggc 9960
   cagggctaca aaatcacggg cgtcgtggac tatgagcacg tccgcgagct ggcccgcatc 10020
   aatggcgacc tgggccgcct gggcggcctg ctgaaactct ggctcaccga cgacccgcgc 10080
   acggcgcggt tcggtgatgc cacgatcctc gccctgctgg cgaagatcga agagaagcag 10140
   gacgagcttg gcaaggtcat gatgggcgtg gtccgcccga gggcagagcc atgacttttt 10200
   tagccgctaa aacggccggg gggtgcgcgt gattgccaag cacgtcccca tgcgctccat 10260
   caagaagagc gacttcgcgg agctggtgaa gtacatcacc gacgagcaag gcaagaccga 10320
   tcccccgata agcttcgacg gtatcgataa gcttcgttgt gtctcaaaat ctctgatgtt 10380
   acattgcaca agataaaaat atatcatcat gaacaataaa actgtctgct tacataaaca 10440
   gtaatacaag gggtgttatg agccatattc aacgggaaac gtcttgctcg aggccgcgat 10500
   taaattccaa catggatgct gatttatatg ggtataaatg ggctcgcgat aatgtcgggc 10560
   aatcaggtgc gacaatctat cgattgtatg ggaagcccga tgcgccagag ttgtttctga 10620
   aacatggcaa aggtagcgtt gccaatgatg ttacagatga gatggtcaga ctaaactggc 10680
   tgacggaatt tatgcctctt ccgaccatca agcattttat ccgtactcct gatgatgcat 10740
   ggttactcac cactgcgatc cccggaaaaa cagcattcca ggtattagaa gaatatcctg 10800
   attcaggtga aaatattgtt gatgcgctgg cagtgttcct gcgccggttg cattcgattc 10860
   ctgtttgtaa ttgtcctttt aacagcgatc gcgtatttcg tctcgctcag gcgcaatcac 10920
   gaatgaataa cggtttggtt gatgcgagtg attttgatga cgagcgtaat ggctggcctg 10980
   ttgaacaagt ctggaaagaa atgcataaac ttttgccatt ctcaccggat tcagtcgtca 11040
   ctcatggtga tttctcactt gataacctta tttttgacga ggggaaatta ataggttgta 11100
   ttgatgttgg acgagtcgga atcgcagacc gataccagga tcttgccatc ctatggaact 11160
   gcctcggtga gttttctcct tcattacaga aacggctttt tcaaaaatat ggtattgata 11220
   atcctgatat gaataaattg cagtttcatt tgatgctcga tgagtttttc taatcagaat 11280
   tggttaattg gttgtaacac tggcaagctt gacggaagcc gatactgcat cttcatggtt 11340
   tcaccgccga cacagccagc cggggagtga cggctcacag gtgccgctcc gcgtcctgtg 11400
   cgctatgcag gacgcggaca atctccagcc agtctccccg gtcgcggaaa tagatcacat 11460
   gcgagccggt ggggcatttg cgatagccaa ggcgaacatt cgtcgcccgc ccctgcttcc 11520
   agcccgatgc cagcgcatgg caggcgtccc ggatttcgtc ggtgtagcgg tcggcttgat 11580
   ctggtcccca gttctcagcg ctgtaatccc agataccgtc aatgtcggct tctgcccgag 11640
   gcgagaagga aagagccttc atctgccctc atactccgcg cgcttccgct tcttgaatgc 11700
   ctcgaagtca aagggctgcg gttcgccgga ttcctcgcct tcgatcaacg ccgcttccag 11760
   cgcctttacc ttggcctcat gctcttgcag cagccgcagc cctgcccgca ccacatcgct 11820
   ggccgatccg tagcgcccgg cctgcacctg cgtgtcaatg aacgtggtga aatggtcccc 11880
   gagggacacg gacgtattgc gggccatcgc cgcccccttc ctgttctaag cagattccca 11940
   aaatatacca ataagtacca agaggcaatc tggctgctgt cggtgccagc ggtcgcgctt 12000
   ccgcgacggc aagcggcgca gggcgcttcg caccctgcgc gcaccgcacc cgtcttaccc 12060
   ttttgggtaa gggtcttgat cctcggaatt ggcacaggct ctagtgttat tagtgttacc 12120
   tacggagtaa gttacgtccc ccataagcat ctgtgaaaaa tggcataaac cggtattttg 12180
   gttccgaaag ggtcaaactt tggttccgaa agggtcaaac tttggttccg aaagggtcaa 12240
   actgcctttc catcgacaac cgttcctgat aggtcaaaca agatagttcc tgataggtca 12300
   aagttgcgcc gtatgagcct gcttccagat cggcacccaa acctagattt cttcgtgctc 12360
   gacatcgcgg atgcggtgcc gaaggatgac atggcgtcga tggagcatcc gctgttttcg 12420
   ttggcaacca agccggacat gcggcaccta gaatatcgta acggcgacaa cgttctgaaa 12480
   atccgaccct ctgggctcgg cctgccaacg atcttcgaca aggacattct aatcttcacc 12540
   atcagccagt tgatggcccg gaaaaatcgt ggcgaaccga ttggcgacac ggtccgcttc 12600
   tcggcgcgag agctgtctgt tgcgacgaac cggcctatcg gtgggaacca ctacaagcgc 12660
   cttgaggatg ccttcgcccg cctgcaaggg gcgcagttcg tcaccaacat caagtccggg 12720
   gggaaaatcg aaacccggat attctccctg atcgacgagg gtggttttgt ccgcactgac 12780
   gatgagcgat tccgcctcga ctactgcgag gtcaagctgt cgcgctggct gatgcgggcg 12840
   atagagactg accaggtggt gacgatcacc catgactact tccgcctgcg caggcccttg 12900
   gagcgccgcc tgtatgagat cgcccgtaag cactgcggca actccccgaa gtggcagatc 12960
   ggacttgcaa acctccagaa caagacaggc agcaatgccc cggcaaagcg ctttcgccac 13020
   aacctgcgcg agatcatgcg agcagatgtg acaccgttct ataagttcaa tatcgacgaa 13080
   aacgacatgg tgacagtgcg ccctcgctct acgccggtcg atccttcttc accaatcatc 13140
   atcccagaat gggccgagga gcaggccaga gcacacgctc gaaccctcgg ctgggattac 13200
   tacaccctac ggtctaactg gatggcatac gctcacgagg aaaccgccaa gggcaacccc 13260
   ccgaaaaatc ccggcgcggc atttgtggca tactgcaaaa aacaagagaa gctgcgctga 13320
   cagcgcaagg actggacgcg aacaggcgaa cagtccacga attcgcc 13367
<210> 18
   <211> 13367
   <212> DNA
   <213> unknown
<220>
   <223> Synthetic
<400> 18
   aattgtgagc ggataacaat tacgagcttc atgcacagtg aaatcatgaa aaatttattt 60
   gctttgtgag cggataacaa ttataatatg tggaattgtg agcgctcaca attccacaac 120
   ggtttccctc tagaaataat tttgtttaac ttttgcccta taaccagacc gttcagcgtt 180
   ttagagctag aaatagcaag ttaaaataag gctagtccgt tatcaacttg aaaaagtggc 240
   accgagtcgg tggtgcgccc caggcatcaa ataaaacgaa aggctcagtc gaaagactgg      300
   gcctttcgtt ttatctgttg tttgtcggtg aacgctctct actagagtca cactggctca      360
   ccttcgggtg ggcctttctg cgtttatagc ccacagcaaa caccacgtcg accctatcag      420
   ctgcgtgctt tctatgagtc gttgctgcat aacttgacaa ttaatcatcc ggctcgtaat      480
   gtttgtggag ccgggcccaa gttcacttaa aaaggagatc aacaatgaaa gcaattttcg      540
   tactgaaaca tcttaatcat gctaaggagg ttttctaatg gtaagcaagg gcgaggagaa      600
   taacatggcc atcatcaagg agttcatgcg cttcaaggtg cacatggagg gcagtgtgaa      660
   cggccacgag ttcgagatcg agggcgaggg cgagggccgt ccctacgagg cctttcagac      720
   cgctaagctg aaggtgacca agggtggccc cctgcccttc gcctgggaca tcctgtcccc      780
   tcagttcatg tacggctcca aggtctacat taagcaccca gccgacatcc ccgactactt      840
   caagctgtcc ttccccgagg gcttcaggtg ggagcgcgtg atgaacttcg aggacggcgg      900
   cattattcac gttaaccagg acagttccct gcaagacggc gtgttcatct acaaggtgaa      960
   gctgcgcggc accaacttcc ccagtgacgg ccccgtaatg cagaagaaga ccatgggctg     1020
   ggaggccagt gaggagcgga tgtaccccga ggacggcgcc ctgaagtctg agatcaaaaa     1080
   gaggctgaag ctgaaggacg gcggccacta cgccgccgag gtcaagacca cctacaaggc     1140
   caagaagccc gtgcagctgc ccggcgccta catcgtcgac atcaagttgg acatcgtgtc     1200
   ccacaacgag gactacacca tcgtggaaca gtacgaacgc gccgagggcc gccactccac     1260
   cggcggcatg gacgagctgt acaaggccga caagaaatac tcgatagggc tggatatcgg     1320
   gaccaactcc gtcggttggg ctgttatcac ggatgaatat aaggtgccca gcaaaaagtt     1380
   caaggtgcta ggtaacaccg accggcacag tatcaaaaaa aacttgatag gagcgttgct     1440
   gtttgacagt ggtgagaccg ctgaagctac gcgccttaaa aggaccgcgc gccgtagata     1500
   tacccgcagg aagaaccgca tttgctatct ccaagagatt ttttcgaacg agatggctaa     1560
   agtagatgat agtttctttc atcgattgga agaaagcttt ttagttgaag aagacaagaa     1620
   gcatgaacgc catccaattt ttggtaacat agtagatgaa gtggcgtatc atgagaaata     1680
   tccgacgatc tatcatctac gaaaaaaatt agtagatagc acggataaag ctgatttacg     1740
   gctgatctat ttagcactgg ctcatatgat taagtttcgc ggccattttt taattgaggg     1800
   tgatctgaac ccagataaca gtgatgttga caaactcttt atccaattag tacagactta     1860
   caaccagctg tttgaagaaa atccaattaa tgccagtggt gtagatgcga aagctatttt     1920
   gagcgcccga ttaagtaaat cgcgtcgact ggaaaacctt attgcgcaac ttcctggcga     1980
   gaagaaaaac gggctgtttg gaaaccttat tgcgttatcg ttaggcttaa ctccaaactt     2040
   taaatcgaac tttgatttag ccgaagatgc gaaactgcaa ttgtcgaaag atacgtacga     2100
   tgatgatctg gataacctgt tagctcagat tggtgatcag tatgcggatt tatttttagc 2160
   cgcgaagaac ctgtcggatg cgattctgtt gtcggatatc ctccgtgtaa acacggaaat 2220
   aacgaaggcg cctctctcgg cgtcgatgat taaacggtac gatgaacatc atcaggactt 2280
   aacgttgctg aaagcgctgg tgcgacagca gttgccggaa aagtataaag aaatcttttt 2340
   tgatcagtcg aaaaatggtt atgccggcta tattgatgga ggtgcgtccc aggaagaatt 2400
   ttataaattt atcaaaccga ttctggaaaa aatggatggc acggaggaac tgttggttaa 2460
   actcaaccgc gaagatttgc tacggaagca gaggactttt gacaatggga gcattcctca 2520
   tcagattcac ttgggcgagc tacatgcgat tttgcgtcgt caggaagact tttatccgtt 2580
   tctgaaagac aaccgcgaga agattgaaaa aatcttgacg tttcgaatcc catattatgt 2640
   gggcccgtta gctcgcggga acagtcgctt tgcctggatg acgaggaagt cggaagaaac 2700
   cattactccg tggaactttg aagaagtggt cgataaaggc gcgtcggcgc agtcgtttat 2760
   tgaacggatg accaattttg ataaaaactt gccgaacgaa aaagtactcc cgaaacatag 2820
   tttattgtat gagtatttta cagtgtataa tgaattaacc aaggtcaaat atgtgacgga 2880
   aggtatgcga aaaccggcct ttttgtcggg cgaacaaaag aaagcaattg tggatctgct 2940
   tttcaaaacc aaccgaaaag taactgtgaa gcagctgaaa gaagattatt tcaaaaaaat 3000
   agaatgcttt gatagtgtgg aaatttcggg tgtggaagat cgttttaacg cgtcgctggg 3060
   cacttaccat gatttactca aaattattaa agataaagat tttttagata acgaagaaaa 3120
   cgaagatatc ctggaggata ttgtgctgac cttaactctg tttgaagata gagagatgat 3180
   tgaggaacgt ttgaaaacct atgcgcacct ttttgatgat aaggttatga aacaattgaa 3240
   acgccggcgc tatacgggct ggggtcgctt aagccgaaaa ttaattaacg gcattagaga 3300
   taagcagagc gggaaaacca tactggattt tttaaaatcg gatggctttg caaaccggaa 3360
   ctttatgcaa ctaatccatg atgatagttt aacctttaaa gaagacattc agaaagccca 3420
   ggttagcggt cagggggata gtctgcatga acatattgcc aacctggcgg gctccccagc 3480
   gattaaaaaa ggcattctgc aaacggtaaa agtggtggat gaattagtca aagtaatggg 3540
   aaggcataag ccggaaaaca tcgtgattga aatggcccgc gaaaaccaaa ccacgcagaa 3600
   ggggcaaaaa aactcacgag agcgcatgaa acgaatcgaa gaaggcatca aagaactggg 3660
   tagtcaaatt ttgaaagagc atccagtgga aaacacgcag ttacagaacg aaaagcttta 3720
   tctttattat cttcagaacg gtcgtgacat gtatgttgac caggaactgg atattaaccg 3780
   cctgagtgat tatgatgtcg atcacattgt gccgcagagt ttcttgaaag acgattcgat 3840
   agacaacaag gtcctgacac gcagcgataa aaaccgcggc aaatcagata atgtgccgag 3900
   tgaagaagta gtcaaaaaga tgaaaaatta ttggcgtcag ttgctcaatg caaagctgat 3960
   cacgcagcgc aagtttgata acctgacaaa agcggaacgc ggtggcttaa gtgaattgga 4020
   taaagcgggc tttatcaaac ggcagttagt ggaaacgcgg cagatcacga agcatgttgc 4080
   ccagatttta gatagtcgga tgaacacgaa atacgatgaa aacgataaat tgattcgaga 4140
   ggtgaaagtt attactctga aaagcaaact ggtgagcgac ttccgaaaag atttccagtt 4200
   ctataaagta cgcgagatta ataactacca tcatgcacat gatgcttatc tcaacgcagt 4260
   cgtgggtacg gcgttaatta agaaatatcc gaaattggaa tcagagtttg tctatggcga 4320
   ttataaagtg tatgatgtgc gcaaaatgat tgcgaagtcg gagcaggaaa tagggaaagc 4380
   cactgccaaa tatttctttt acagcaacat catgaatttc ttcaaaaccg aaattacctt 4440
   ggccaacggt gagattcgga aacggccact catcgaaacg aacggagaaa cgggtgaaat 4500
   tgtctgggat aaaggacgag attttgcaac cgttcggaaa gtattgtcca tgcctcaggt 4560
   caacattgtc aagaaaaccg aagtacaaac cgggggtttc tccaaggagt cgattctgcc 4620
   gaaacgcaac tcagacaagt tgattgcgcg caaaaaagac tgggatccga aaaaatatgg 4680
   cggcttcgat tccccgacag tagcgtactc ggtcctcgtc gtggcgaagg tcgaaaaagg 4740
   gaaatccaag aagctgaaat ccgtgaaaga gctgctcggg atcaccatca tggagcgctc 4800
   ctccttcgag aagaacccca tcgacttcct ggaggcgaag ggctacaagg aggtgaagaa 4860
   ggacctgatc atcaagctcc ccaagtactc cctctttgag ctggagaacg gccgcaagag 4920
   gatgctcgca agtgcaggtg aattacaaaa aggtaatgaa ctagcgctac cgtccaaata 4980
   tgttaacttt ctgtatctgg cgagtcatta tgaaaagtta aagggcagtc cggaagataa 5040
   tgaacagaaa cagttatttg ttgagcaaca taagcattat ctggatgaga ttattgagca 5100
   gatcagtgaa tttagcaagc gcgtgattct ggccgatgca aacctggata aagtgttgag 5160
   tgcctataat aaacatcgtg acaaaccgat acgcgaacag gccgaaaaca ttattcatct 5220
   gtttacatta acaaacttgg gtgcgcctgc ggcgtttaaa tattttgata ccaccattga 5280
   tcgcaaacga tatacaagca ccaaagaagt gctggatgca acgttgatcc atcagtctat 5340
   cacgggcttg tatgaaaccc ggattgattt aagtcaactc ggtggcgact aagcccggcc 5400
   gctactagta acaaaaaacc cctagccccc cgttttatat cactgcccgc tttccagtcg 5460
   ggaaacctgt cgtgccagct gcattaatga atcggccaac gcgcggggag aggcggtttg 5520
   cgtattgggc gccagggtgg tttttctttt caccagtgag actggcaaca gctgattgcc 5580
   cttcaccgcc tggccctgag agagttgcag caagcggtcc acgctggttt gccccagcag 5640
   gcgaaaatcc tgtttgatgg tggttaacgg cgggatataa catgagctat cttcggtatc 5700
   gtcgtatccc actaccgaga tatccgcacc aacgcgcagc ccggactcgg taatggcgcg 5760
   cattgcgccc agcgccatct gatcgttggc aaccagcatc gcagtgggaa cgatgccctc 5820
   attcagcatt tgcatggttt gttgaaaacc ggacatggca ctccagtcgc cttcccgttc 5880
   cgctatcggc tgaatttgat tgcgagtgag atatttatgc cagccagcca gacgcagacg 5940
   cgccgagaca gaacttaatg ggcccgctaa cagcgcgatt tgctggtgac ccaatgcgac 6000
   cagatgctcc acgcccagtc gcgtaccgtc ctcatgggag aaaataatac tgttgatggg 6060
   tgtctggtca gagacatcaa gaaataacgc cggaacatta gtgcaggcag cttccacagc 6120
   aatggcatcc tggtcatcca gcggatagtt aatgatcagc ccactgacgc gttgcgcgag 6180
   aagattgtgc accgccgctt tacaggcttc gacgccgctt cgttctacca tcgacaccac 6240
   cacgctggca cccagttgat cggcgcgaga tttaatcgcc gcgacaattt gcgacggcgc 6300
   gtgcagggcc agactggagg tggcaacgcc aatcagcaac gactgtttgc ccgccagttg 6360
   ttgtgccacg cggttgggaa tgtaattcag ctccgccatc gccgcttcca ctttttcccg 6420
   cgttttcgca gaaacgtggc tggcctggtt caccacgcgg gaaacggtct gataagagac 6480
   accggcatac tctgcgacat cgtataacgt tactggtttc atattcacca ccctgaattg 6540
   actgtcttcc gggcgctatc atgccatacc gcgaaaggtt ttgcgccatt cgatggcgcg 6600
   ccgcgccctg cagcccgggg gatccactag ttctagagcg gccgccaccg cggtggagct 6660
   ccaattcgcc ctatagtgag tcgtattacg cgcgctcact ggccgtcgtt ttacaacgtc 6720
   gtgactggga aaaccctggc gttacccaac ttaatcgcct tgcagcacat ccccctttcg 6780
   ccagctggcg taatagcgaa gaggcccgca ccgatcgccc ttcccaacag ttgcgcagcc 6840
   tgaatggcga atgggacgcg ccctgtagcg gcgcattaag cgcggcgggt gtggtggtta 6900
   cgcgcagcgt gaccgctaca cttgccagcg ccctagcgcc cgctcctttc gctttcttcc 6960
   cttcctttct cgccacgttc gccggctttc cccgtcaagc tctaaatcgg gggctccctt 7020
   tagggttccg atttagtgct ttacggcacc tcgaccccaa aaaacttgat tagggtgatg 7080
   gttcacgtag tgggccatcg ccctgataga cggtttttcg ccctttgacg ttggagtcca 7140
   cgttctttaa tagtggactc ttgttccaaa ctggaacaac actcaaccct atctcggtct 7200
   attcttttga tttataaggg attttgccga tttcggccta ttggttaaaa aatgagctga 7260
   tttaacaaaa atttaacgcg aattttaaca aaatattaac gcttacaatt taggtggcac 7320
   ttttcgggga aatgtgcgcg gaacccctat ttgtttattt ttctaaatac attcaaatat 7380
   gtatccgctc atgagacaat aaccctgata aatgcttcaa taatattgaa aaaggaagag 7440
   tatgagtatt caacatttcc gtgtcgccct tattcccttt tttgcggcat tttgccttcc 7500
   tgtttttgct cacccagaaa cgctggtgaa agtaaaagat gctgaagatc agttgggtgc 7560
   acgagtgggt tacatcgaac tggatctcaa cagcggtaag atccttgaga gttttcgccc 7620
   cgaagaacgt tttccaatga tgagcacttt taaagttctg ctatgtggcg cggtattatc 7680
   ccgtattgac gccgggcaag agcaactcgg tcgccgcata cactattctc agaatgactt 7740
   ggttgagtac tcaccagtca cagaaaagca tcttacggat ggcatgacag taagagaatt 7800
   atgcagtgct gccataacca tgagtgataa cactgcggcc aacttacttc tgacaacgat 7860
   cggaggaccg aaggagctaa ccgctttttt gcacaacatg ggggatcatg taactcgcct 7920
   tgatcgttgg gaaccggagc tgaatgaagc cataccaaac gacgagcgtg acaccacgat 7980
   gcctgtagca atggcaacaa cgttgcgcaa actattaact ggcgaactac ttactctagc 8040
   ttcccggcaa caattaatag actggatgga ggcggataaa gttgcaggac cacttctgcg 8100
   ctcggccctt ccggctggct ggtttattgc tgataaatct ggagccggtg agcgtgggtc 8160
   tcgcggtatc attgcagcac tggggccaga tggtaagccc tcccgtatcg tagttatcta 8220
   cacgacgggg agtcaggcaa ctatggatga acgaaataga cagatcgctg agataggtgc 8280
   ctcactgatt aagcattggt aactgtcaga ccaagtttac tcatatatac tttagattga 8340
   tttaaaactt catttttaat ttaaaaggat ctaggtgaag atcctttttg ataatctcat 8400
   gaccaaaatc ccttaacgtg agttttcgtt ccactgagcg tcagaccccg tagaaaagat 8460
   caaaggatct tcttgagatc ctttttttct gcgcgtaatc tgctgcttgc aaacaaaaaa 8520
   accaccgcta ccagcggtgg tttgtttgcc ggatcaagag ctaccaactc tttttccgaa 8580
   ggtaactggc ttcagcagag cgcagatacc aaatactgtc cttctagtgt agccgtagtt 8640
   aggccaccac ttcaagaact ctgtagcacc gcctacatac ctcgctctgc taatcctgtt 8700
   accagtggct gctgccagtg gcgataagtc gtgtcttacc gggttggact caagacgata 8760
   gttaccggat aaggcgcagc ggtcgggctg aacggggggt tcgtgcacac agcccagctt 8820
   ggagcgaacg acctacaccg aactgagata cctacagcgt gagctatgag aaagcgccac 8880
   gcttcccgaa gggagaaagg cggacaggta tccggtaagc ggcagggtcg gaacaggaga 8940
   gcgcacgagg gagcttccag ggggaaacgc ctggtatctt tatagtcctg tcgggtttcg 9000
   ccacctctga cttgagcgtc gatttttgtg atgctcgtca ggggggcgga gcctatggaa 9060
   aaacgccagc aacgcggcct ttttacggtt cctggccttt tgctggcctt ttgctcacat 9120
   gttctttcct gcgttatccc ctgattctgt ggataaccgt attaccgcct ttgagtgagc 9180
   tgataccgct cgccgcagcc gaacgaccga gcgcagcgag tcagtgagcg aggaagcgga 9240
   agagcgccca atacgcaaac cgcctctccc cgcgcgttgg ccgattcatt aatgcagctg 9300
   gcacgacagg tttcccgact ggaaagcggg cagtgagcgc aacgcaatta atgtgagtta 9360
   gctcactcat taggcacccc aggctttaca ctttatgctt ccggctcgta tgttgtgtgg 9420
   aattgtgagc ggataacaat ttcacacagg aaacagctat gaccatgatt acgccaagcg 9480
   cgcaattaac cctcactaaa gggaacaaaa gctgggtacc gggccccccc tcgaggtcga 9540
   cggatctttt ccgctgcata accctgcttc ggggtcatta tagcgatttt ttcggtatat 9600
   ccatcctttt tcgcacgata tacaggattt tgccaaaggg ttcgtgtaga ctttccttgg 9660
   tgtatccaac ggcgtcagcc gggcaggata ggtgaagtag gcccacccgc gagcgggtgt 9720
   tccttcttca ctgtccctta ttcgcacctg gcggtgctca acgggaatcc tgctctgcga 9780
   ggctggccgg ctaccgccgg cgtaacagat gagggcaagc ggatggctga tgaaaccaag 9840
   ccaaccagga agggcagccc acctatcaag gtgtactgcc ttccagacga acgaagagcg 9900
   attgaggaaa aggcggcggc ggccggcatg agcctgtcgg cctacctgct ggccgtcggc 9960
   cagggctaca aaatcacggg cgtcgtggac tatgagcacg tccgcgagct ggcccgcatc 10020
   aatggcgacc tgggccgcct gggcggcctg ctgaaactct ggctcaccga cgacccgcgc 10080
   acggcgcggt tcggtgatgc cacgatcctc gccctgctgg cgaagatcga agagaagcag 10140
   gacgagcttg gcaaggtcat gatgggcgtg gtccgcccga gggcagagcc atgacttttt 10200
   tagccgctaa aacggccggg gggtgcgcgt gattgccaag cacgtcccca tgcgctccat 10260
   caagaagagc gacttcgcgg agctggtgaa gtacatcacc gacgagcaag gcaagaccga 10320
   tcccccgata agcttcgacg gtatcgataa gcttcgttgt gtctcaaaat ctctgatgtt 10380
   acattgcaca agataaaaat atatcatcat gaacaataaa actgtctgct tacataaaca 10440
   gtaatacaag gggtgttatg agccatattc aacgggaaac gtcttgctcg aggccgcgat 10500
   taaattccaa catggatgct gatttatatg ggtataaatg ggctcgcgat aatgtcgggc 10560
   aatcaggtgc gacaatctat cgattgtatg ggaagcccga tgcgccagag ttgtttctga 10620
   aacatggcaa aggtagcgtt gccaatgatg ttacagatga gatggtcaga ctaaactggc 10680
   tgacggaatt tatgcctctt ccgaccatca agcattttat ccgtactcct gatgatgcat 10740
   ggttactcac cactgcgatc cccggaaaaa cagcattcca ggtattagaa gaatatcctg 10800
   attcaggtga aaatattgtt gatgcgctgg cagtgttcct gcgccggttg cattcgattc 10860
   ctgtttgtaa ttgtcctttt aacagcgatc gcgtatttcg tctcgctcag gcgcaatcac 10920
   gaatgaataa cggtttggtt gatgcgagtg attttgatga cgagcgtaat ggctggcctg 10980
   ttgaacaagt ctggaaagaa atgcataaac ttttgccatt ctcaccggat tcagtcgtca 11040
   ctcatggtga tttctcactt gataacctta tttttgacga ggggaaatta ataggttgta 11100
   ttgatgttgg acgagtcgga atcgcagacc gataccagga tcttgccatc ctatggaact 11160
   gcctcggtga gttttctcct tcattacaga aacggctttt tcaaaaatat ggtattgata 11220
   atcctgatat gaataaattg cagtttcatt tgatgctcga tgagtttttc taatcagaat 11280
   tggttaattg gttgtaacac tggcaagctt gacggaagcc gatactgcat cttcatggtt 11340
   tcaccgccga cacagccagc cggggagtga cggctcacag gtgccgctcc gcgtcctgtg 11400
   cgctatgcag gacgcggaca atctccagcc agtctccccg gtcgcggaaa tagatcacat 11460
   gcgagccggt ggggcatttg cgatagccaa ggcgaacatt cgtcgcccgc ccctgcttcc 11520
   agcccgatgc cagcgcatgg caggcgtccc ggatttcgtc ggtgtagcgg tcggcttgat 11580
   ctggtcccca gttctcagcg ctgtaatccc agataccgtc aatgtcggct tctgcccgag 11640
   gcgagaagga aagagccttc atctgccctc atactccgcg cgcttccgct tcttgaatgc 11700
   ctcgaagtca aagggctgcg gttcgccgga ttcctcgcct tcgatcaacg ccgcttccag 11760
   cgcctttacc ttggcctcat gctcttgcag cagccgcagc cctgcccgca ccacatcgct 11820
   ggccgatccg tagcgcccgg cctgcacctg cgtgtcaatg aacgtggtga aatggtcccc 11880
   gagggacacg gacgtattgc gggccatcgc cgcccccttc ctgttctaag cagattccca 11940
   aaatatacca ataagtacca agaggcaatc tggctgctgt cggtgccagc ggtcgcgctt 12000
   ccgcgacggc aagcggcgca gggcgcttcg caccctgcgc gcaccgcacc cgtcttaccc 12060
   ttttgggtaa gggtcttgat cctcggaatt ggcacaggct ctagtgttat tagtgttacc 12120
   tacggagtaa gttacgtccc ccataagcat ctgtgaaaaa tggcataaac cggtattttg 12180
   gttccgaaag ggtcaaactt tggttccgaa agggtcaaac tttggttccg aaagggtcaa 12240
   actgcctttc catcgacaac cgttcctgat aggtcaaaca agatagttcc tgataggtca 12300
   aagttgcgcc gtatgagcct gcttccagat cggcacccaa acctagattt cttcgtgctc 12360
   gacatcgcgg atgcggtgcc gaaggatgac atggcgtcga tggagcatcc gctgttttcg 12420
   ttggcaacca agccggacat gcggcaccta gaatatcgta acggcgacaa cgttctgaaa 12480
   atccgaccct ctgggctcgg cctgccaacg atcttcgaca aggacattct aatcttcacc 12540
   atcagccagt tgatggcccg gaaaaatcgt ggcgaaccga ttggcgacac ggtccgcttc 12600
   tcggcgcgag agctgtctgt tgcgacgaac cggcctatcg gtgggaacca ctacaagcgc 12660
   cttgaggatg ccttcgcccg cctgcaaggg gcgcagttcg tcaccaacat caagtccggg 12720
   gggaaaatcg aaacccggat attctccctg atcgacgagg gtggttttgt ccgcactgac 12780
   gatgagcgat tccgcctcga ctactgcgag gtcaagctgt cgcgctggct gatgcgggcg 12840
   atagagactg accaggtggt gacgatcacc catgactact tccgcctgcg caggcccttg 12900
   gagcgccgcc tgtatgagat cgcccgtaag cactgcggca actccccgaa gtggcagatc 12960
   ggacttgcaa acctccagaa caagacaggc agcaatgccc cggcaaagcg ctttcgccac 13020
   aacctgcgcg agatcatgcg agcagatgtg acaccgttct ataagttcaa tatcgacgaa 13080
   aacgacatgg tgacagtgcg ccctcgctct acgccggtcg atccttcttc accaatcatc 13140
   atcccagaat gggccgagga gcaggccaga gcacacgctc gaaccctcgg ctgggattac 13200
   tacaccctac ggtctaactg gatggcatac gctcacgagg aaaccgccaa gggcaacccc 13260
   ccgaaaaatc ccggcgcggc atttgtggca tactgcaaaa aacaagagaa gctgcgctga 13320
   cagcgcaagg actggacgcg aacaggcgaa cagtccacga attcgcc 13367
<210> 19
   <211> 11077
   <212> DNA
   <213> unknown
<220>
   <223> Synthetic
<400> 19
   aattgtgagc ggataacaat tacgagcttc atgcacagtg aaatcatgaa aaatttattt      60
   gctttgtgag cggataacaa ttataatatg tggaattgtg agcgctcaca attccacaac     120
   ggtttccctc tagaaataat tttgtttaac ttttgcccac cggggtggtg cccatccgtt     180
   ttagagctag aaatagcaag ttaaaataag gctagtccgt tatcaacttg aaaaagtggc     240
   accgagtcgg tggtgcgccc caggcatcaa ataaaacgaa aggctcagtc gaaagactgg     300
   gcctttcgtt ttatctgttg tttgtcggtg aacgctctct actagagtca cactggctca     360
   ccttcgggtg ggcctttctg cgtttatagc ccacagcaaa caccacgtcg accctatcag     420
   ctgcgtgctt tctatgagtc gttgctgcat aacttgacaa ttaatcatcc ggctcgtaat     480
   gtttgtggag ccgggcccaa gttcacttaa aaaggagatc aacaatgaaa gcaattttcg     540
   tactgaaaca tcttaatcat gctaaggagg ttttctaatg gtaagcaagg gcgaggagaa     600
   taacatggcc atcatcaagg agttcatgcg cttcaaggtg cacatggagg gcagtgtgaa     660
   cggccacgag ttcgagatcg agggcgaggg cgagggccgt ccctacgagg cctttcagac     720
   cgctaagctg aaggtgacca agggtggccc cctgcccttc gcctgggaca tcctgtcccc     780
   tcagttcatg tacggctcca aggtctacat taagcaccca gccgacatcc ccgactactt     840
   caagctgtcc ttccccgagg gcttcaggtg ggagcgcgtg atgaacttcg aggacggcgg     900
   cattattcac gttaaccagg acagttccct gcaagacggc gtgttcatct acaaggtgaa     960
   gctgcgcggc accaacttcc ccagtgacgg ccccgtaatg cagaagaaga ccatgggctg    1020
   ggaggccagt gaggagcgga tgtaccccga ggacggcgcc ctgaagtctg agatcaaaaa    1080
   gaggctgaag ctgaaggacg gcggccacta cgccgccgag gtcaagacca cctacaaggc    1140
   caagaagccc gtgcagctgc ccggcgccta catcgtcgac atcaagttgg acatcgtgtc    1200
   ccacaacgag gactacacca tcgtggaaca gtacgaacgc gccgagggcc gccactccac    1260
   cggcggcatg gacgagctgt acaaggccga caagaaatac tcgatagggc tggatatcgg    1320
   gaccaactcc gtcggttggg ctgttatcac ggatgaatat aaggtgccca gcaaaaagtt    1380
   caaggtgcta ggtaacaccg accggcacag tatcaaaaaa aacttgatag gagcgttgct    1440
   gtttgacagt ggtgagaccg ctgaagctac gcgccttaaa aggaccgcgc gccgtagata    1500
   tacccgcagg aagaaccgca tttgctatct ccaagagatt ttttcgaacg agatggctaa    1560
   agtagatgat agtttctttc atcgattgga agaaagcttt ttagttgaag aagacaagaa    1620
   gcatgaacgc catccaattt ttggtaacat agtagatgaa gtggcgtatc atgagaaata    1680
   tccgacgatc tatcatctac gaaaaaaatt agtagatagc acggataaag ctgatttacg 1740
   gctgatctat ttagcactgg ctcatatgat taagtttcgc ggccattttt taattgaggg 1800
   tgatctgaac ccagataaca gtgatgttga caaactcttt atccaattag tacagactta 1860
   caaccagctg tttgaagaaa atccaattaa tgccagtggt gtagatgcga aagctatttt 1920
   gagcgcccga ttaagtaaat cgcgtcgact ggaaaacctt attgcgcaac ttcctggcga 1980
   gaagaaaaac gggctgtttg gaaaccttat tgcgttatcg ttaggcttaa ctccaaactt 2040
   taaatcgaac tttgatttag ccgaagatgc gaaactgcaa ttgtcgaaag atacgtacga 2100
   tgatgatctg gataacctgt tagctcagat tggtgatcag tatgcggatt tatttttagc 2160
   cgcgaagaac ctgtcggatg cgattctgtt gtcggatatc ctccgtgtaa acacggaaat 2220
   aacgaaggcg cctctctcgg cgtcgatgat taaacggtac gatgaacatc atcaggactt 2280
   aacgttgctg aaagcgctgg tgcgacagca gttgccggaa aagtataaag aaatcttttt 2340
   tgatcagtcg aaaaatggtt atgccggcta tattgatgga ggtgcgtccc aggaagaatt 2400
   ttataaattt atcaaaccga ttctggaaaa aatggatggc acggaggaac tgttggttaa 2460
   actcaaccgc gaagatttgc tacggaagca gaggactttt gacaatggga gcattcctca 2520
   tcagattcac ttgggcgagc tacatgcgat tttgcgtcgt caggaagact tttatccgtt 2580
   tctgaaagac aaccgcgaga agattgaaaa aatcttgacg tttcgaatcc catattatgt 2640
   gggcccgtta gctcgcggga acagtcgctt tgcctggatg acgaggaagt cggaagaaac 2700
   cattactccg tggaactttg aagaagtggt cgataaaggc gcgtcggcgc agtcgtttat 2760
   tgaacggatg accaattttg ataaaaactt gccgaacgaa aaagtactcc cgaaacatag 2820
   tttattgtat gagtatttta cagtgtataa tgaattaacc aaggtcaaat atgtgacgga 2880
   aggtatgcga aaaccggcct ttttgtcggg cgaacaaaag aaagcaattg tggatctgct 2940
   tttcaaaacc aaccgaaaag taactgtgaa gcagctgaaa gaagattatt tcaaaaaaat 3000
   agaatgcttt gatagtgtgg aaatttcggg tgtggaagat cgttttaacg cgtcgctggg 3060
   cacttaccat gatttactca aaattattaa agataaagat tttttagata acgaagaaaa 3120
   cgaagatatc ctggaggata ttgtgctgac cttaactctg tttgaagata gagagatgat 3180
   tgaggaacgt ttgaaaacct atgcgcacct ttttgatgat aaggttatga aacaattgaa 3240
   acgccggcgc tatacgggct ggggtcgctt aagccgaaaa ttaattaacg gcattagaga 3300
   taagcagagc gggaaaacca tactggattt tttaaaatcg gatggctttg caaaccggaa 3360
   ctttatgcaa ctaatccatg atgatagttt aacctttaaa gaagacattc agaaagccca 3420
   ggttagcggt cagggggata gtctgcatga acatattgcc aacctggcgg gctccccagc 3480
   gattaaaaaa ggcattctgc aaacggtaaa agtggtggat gaattagtca aagtaatggg 3540
   aaggcataag ccggaaaaca tcgtgattga aatggcccgc gaaaaccaaa ccacgcagaa 3600
   ggggcaaaaa aactcacgag agcgcatgaa acgaatcgaa gaaggcatca aagaactggg 3660
   tagtcaaatt ttgaaagagc atccagtgga aaacacgcag ttacagaacg aaaagcttta 3720
   tctttattat cttcagaacg gtcgtgacat gtatgttgac caggaactgg atattaaccg 3780
   cctgagtgat tatgatgtcg atcacattgt gccgcagagt ttcttgaaag acgattcgat 3840
   agacaacaag gtcctgacac gcagcgataa aaaccgcggc aaatcagata atgtgccgag 3900
   tgaagaagta gtcaaaaaga tgaaaaatta ttggcgtcag ttgctcaatg caaagctgat 3960
   cacgcagcgc aagtttgata acctgacaaa agcggaacgc ggtggcttaa gtgaattgga 4020
   taaagcgggc tttatcaaac ggcagttagt ggaaacgcgg cagatcacga agcatgttgc 4080
   ccagatttta gatagtcgga tgaacacgaa atacgatgaa aacgataaat tgattcgaga 4140
   ggtgaaagtt attactctga aaagcaaact ggtgagcgac ttccgaaaag atttccagtt 4200
   ctataaagta cgcgagatta ataactacca tcatgcacat gatgcttatc tcaacgcagt 4260
   cgtgggtacg gcgttaatta agaaatatcc gaaattggaa tcagagtttg tctatggcga 4320
   ttataaagtg tatgatgtgc gcaaaatgat tgcgaagtcg gagcaggaaa tagggaaagc 4380
   cactgccaaa tatttctttt acagcaacat catgaatttc ttcaaaaccg aaattacctt 4440
   ggccaacggt gagattcgga aacggccact catcgaaacg aacggagaaa cgggtgaaat 4500
   tgtctgggat aaaggacgag attttgcaac cgttcggaaa gtattgtcca tgcctcaggt 4560
   caacattgtc aagaaaaccg aagtacaaac cggggggttc tccaaggagt cgattctgcc 4620
   gaaacgcaac tcagacaagt tgattgcgcg caaaaaagac tgggatccga aaaaatatgg 4680
   cggcttcgat tccccgacag tagcgtactc ggtcctcgtc gtggcgaagg tcgaaaaagg 4740
   gaaatccaag aagctgaaat ccgtgaaaga gctgctcggg atcaccatca tggagcgctc 4800
   ctccttcgag aagaacccca tcgacttcct ggaggcgaag ggctacaagg aggtgaagaa 4860
   ggacctgatc atcaagctcc ccaagtactc cctctttgag ctggagaacg gccgcaagag 4920
   gatgctcgca agtgcaggtg aattacaaaa aggtaatgaa ctagcgctac cgtccaaata 4980
   tgttaacttt ctgtatctgg cgagtcatta tgaaaagtta aagggcagtc cggaagataa 5040
   tgaacagaaa cagttatttg ttgagcaaca taagcattat ctggatgaga ttattgagca 5100
   gatcagtgaa tttagcaagc gcgtgattct ggccgatgca aacctggata aagtgttgag 5160
   tgcctataat aaacatcgtg acaaaccgat acgcgaacag gccgaaaaca ttattcatct 5220
   gtttacatta acaaacttgg gtgcgcctgc ggcgtttaaa tattttgata ccaccattga 5280
   tcgcaaacga tatacaagca ccaaagaagt gctggatgca acgttgatcc atcagtctat 5340
   cacgggcttg tatgaaaccc ggattgattt aagtcaactc ggtggcgact aagcccggcc 5400
   gctactagta acaaaaaacc cctagccccc cgttttatat cactgcccgc tttccagtcg 5460
   ggaaacctgt cgtgccagct gcattaatga atcggccaac gcgcggggag aggcggtttg 5520
   cgtattgggc gccagggtgg tttttctttt caccagtgag actggcaaca gctgattgcc 5580
   cttcaccgcc tggccctgag agagttgcag caagcggtcc acgctggttt gccccagcag 5640
   gcgaaaatcc tgtttgatgg tggttaacgg cgggatataa catgagctat cttcggtatc 5700
   gtcgtatccc actaccgaga tatccgcacc aacgcgcagc ccggactcgg taatggcgcg 5760
   cattgcgccc agcgccatct gatcgttggc aaccagcatc gcagtgggaa cgatgccctc 5820
   attcagcatt tgcatggttt gttgaaaacc ggacatggca ctccagtcgc cttcccgttc 5880
   cgctatcggc tgaatttgat tgcgagtgag atatttatgc cagccagcca gacgcagacg 5940
   cgccgagaca gaacttaatg ggcccgctaa cagcgcgatt tgctggtgac ccaatgcgac 6000
   cagatgctcc acgcccagtc gcgtaccgtc ctcatgggag aaaataatac tgttgatggg 6060
   tgtctggtca gagacatcaa gaaataacgc cggaacatta gtgcaggcag cttccacagc 6120
   aatggcatcc tggtcatcca gcggatagtt aatgatcagc ccactgacgc gttgcgcgag 6180
   aagattgtgc accgccgctt tacaggcttc gacgccgctt cgttctacca tcgacaccac 6240
   cacgctggca cccagttgat cggcgcgaga tttaatcgcc gcgacaattt gcgacggcgc 6300
   gtgcagggcc agactggagg tggcaacgcc aatcagcaac gactgtttgc ccgccagttg 6360
   ttgtgccacg cggttgggaa tgtaattcag ctccgccatc gccgcttcca ctttttcccg 6420
   cgttttcgca gaaacgtggc tggcctggtt caccacgcgg gaaacggtct gataagagac 6480
   accggcatac tctgcgacat cgtataacgt tactggtttc atattcacca ccctgaattg 6540
   actgtcttcc gggcgctatc atgccatacc gcgaaaggtt ttgcgccatt cgatggcgcg 6600
   ccgcgccctg cgctagcatg cctatttgtt tatttttcta aatacattca aatatgtatc 6660
   cgctcatgag acaataaccc tgataaatgc ttcaataata ttgaaaaagg aacagtatga 6720
   gccatattca acgggaaacg tcttgctcca ggccgcgatt aaattccaac atggatgctg 6780
   atttatatgg gtataaatgg gctcgcgata atgtcgggca atcaggtgcg acaatctatc 6840
   gattgtatgg gaagcccgat gcgccagagt tgtttctgaa acatggcaaa ggtagcgttg 6900
   ccaatgatgt tacagatgag atggtcagac taaactggct gacggaattt atgcctctcc 6960
   cgaccatcaa gcattttatc cgtactcctg atgatgcatg gttactcacc actgcgatcc 7020
   ccgggaaaac agcattccag gtattagaag aatatcctga ttcaggtgaa aatattgttg 7080
   atgcgctggc agtgttcctg cgccggttgc attcgattcc tgtttgtaat tgtcctttta 7140
   acagcgatcg cgtatttcgt ctcgctcagg cgcaatcacg aatgaataac ggtttggttg 7200
   atgcgagtga ttttgatgac gagcgtaatg gctggcctgt tgaacaagtc tggaaagaaa 7260
   tgcataagct tttgccattc tcaccggatt cagtcgtcac tcatggtgat ttctcacttg 7320
   ataaccttat ttttgacgag gggaaattaa taggttgtat tgatgttgga cgagtcggaa 7380
   tcgcagaccg ataccaggat cttgccatcc tatggaactg cctcggtgag ttttctcctt 7440
   cattacagaa acggcttttt caaaaatatg gtattgataa tcctgatatg aataaattgc 7500
   agtttcattt gatgctcgat gagtttttct aagccttagt tagttagatt agcagaaagt 7560
   caaaagcctc cgaccggagg cttttgacta aaacttccct tggggttatc attggggctc 7620
   actcaaaggc ggtaatcaga taaaaaaaat ccttagcttt cgctaaggat gatttctgcc 7680
   agatgtgtat aagagacagc tgggcgcgcc ccatgtcagc cgttaagtgt tcctgtgtca 7740
   ctcaaaattg ctttgagagg ctctaagggc ttctcagtgc gttacatccc tggcttgttg 7800
   tccacaaccg ttaaacctta aaggctttaa aagccttata tattcttttt tttcttataa 7860
   aacttaaaac cttagaggct atttaagttg ctgatttata ttaattttat tgttcaaaca 7920
   tgagagctta gtacgtgaaa catgagagct tagtacgtta gccatgagag cttagtacgt 7980
   tagccatgag ggtttagttc gttaaacatg agagcttagt acgttaaaca tgagagctta 8040
   gtacgtgaaa catgagagct tagtacgtac tatcaacagg ttgaactgct gatcttcaga 8100
   tcggcgcgcc ggccggccta cggccagcct cgcagagcag gattcccgtt gagcaccgcc 8160
   aggtgcgaat aagggacagt gaagaaggaa cacccgctcg cgggtgggcc tacttcacct 8220
   atcctgcccg gctgacgccg ttggatacac caaggaaagt ctacacgaac cctttggcaa 8280
   aatcctgtat atcgtgcgaa aaaggatgga tataccgaaa aaatcgctat aatgaccccg 8340
   aagcagggtt atgcagcgga aaaggacaag gccctgcgct agcatgccta tttgtttatt 8400
   tttctaaata cattcaaata tgtatccgct catgagacaa taaccctgat aaatgcttca 8460
   ataatattga aaaaggaaca gtatgagtat tcaacatttc cgtgtcgccc ttattccctt 8520
   ttttgcggca ttttgccttc ctgtttttgc tcacccagaa acgctggtga aagtaaaaga 8580
   tgccgaagat cagttgggtg cacgtgtggg ttacatcgaa ctggacctca acagcggtaa 8640
   gattcttgag agttttcgcc ccgaagaacg tttcccaatg atgagcactt ttaaagttct 8700
   gctctgtggc gcggtattat cccgtattga cgccgggcaa gagcaactcg gtcgccgcat 8760
   acactattct cagaatgact tggttgagta ctcaccagtc acagaaaagc atcttacgga 8820
   cggcatgaca gtacgcgaat tatgcagcgc tgccataacc atgagtgata acacggcggc 8880
   caacttactt ctgacaacga tcggaggacc gaaggagctt accgcttttt tgcacaacat 8940
   gggtgatcat gtaactcgcc ttgatcgttg ggaaccggag ctgaatgaag ccataccaaa 9000
   cgacgagcgt gacaccacga tgcctgtagc tatggcaaca acgttgcgca aactcttaac 9060
   tggcgaactt cttactctcg cttcccggca acaattaata gactggatgg aggcggataa 9120
   agttgcagga ccacttctgc gctcggccct tccggctggc tggtttattg ctgataaatc 9180
   tggagccggt gagcgtgggt cccgcggtat tattgcagcc ctggggccag atggtaagcc 9240
   ctcccgtatc gtagttatct acacgacggg gagccaggca actatggacg aacgtaatcg      9300
   ccagatcgct gagataggtg cctccctgat taagcattgg taagccttag attttaatgc      9360
   cctgcgccat caggtctttc gcggccagaa agccatccag tttgctttgc agcgcttccc      9420
   aaccttccca cagcgcaccc cagctcgcaa tgccggtacg tttgctatcc ataaagccgc      9480
   ccagacgcgc aatcgccata tacgcccatt gcaggctgcc cgctttttct ttgcgtttgc      9540
   gtttgccttt atccagatag cccagcagtt ggcattcatc cggggtcagc acggtttccg      9600
   cgctctggct ttcaacgtgt tccgcttctt tcagcaggcc ctgcgcacgc agtgcttgcg      9660
   gcggagtaaa agattcacgc agttgcagca gacgcaccgc cacaaagctc agaatgctca      9720
   ccatacgttc caggttatcc ggttcttcca tacgctgacg ttccgcaccc gcacccgttt      9780
   tccacgcttt gtgaaattct tcaatgcgcc aacgatgggt ataaatatca atcacacgca      9840
   gcgcttgggc cagactttcc accggctcgc tggtcagcag cagccatttc agcggggttt      9900
   cgcctttcgg cggattaatt tcttcggcca gcaccgcgtt cagggtaatg ttgccctgtt      9960
   tcagggtaat acggccgcta cgcaggctca ggctcgcttt acgcgccgga cggtttttac     10020
   gtttgccacg tttatccacc acgcctttct gcggaatgct aatctgatag ccgcccagtt     10080
   ccggctggtt tttcaggtga tcatacagat acaggccgct ttccacatct ttacgcggat     10140
   gtttgctacg caccacaaaa cgttcgttat gggccagttt atcttgcaga tacgcatgaa     10200
   tatccgcttc acgatcgcac accgcaatca cgttgctcat catgctgccc attctcagac     10260
   gcgaagttgc agcagcggcc agccatttgc cgctttcttt ttcatccgca tccgccggat     10320
   catccggacg catccaccat tcttgatgca gcaggcccac ggtacgaaag gtggtcgctt     10380
   ccagcagcag cacgctatgc acccaccaac cacggctttt atcctgaatg ctacccagtt     10440
   tgcccagttc ttccgccacc tgatgacgat agctcagaga ggtggtatct tcaattgcca     10500
   gcagttccgg aaattcctgg gccagtttca cggtctgcat ggcacccgct ttacgaatcg     10560
   cttccgcgct cacgttcgga ttacgaataa aacgatacgc gccttcctgc atggctttgc     10620
   tgccttcgct gctaatggta atgcttttgc cgctatattt ggccagttgc gccgcaacat     10680
   tcaccagacg cgcggtacga cgcggatcac ccagcgcagc actagaaaac acgcttttcg     10740
   cccaatccgc cgcacgatgc agtgcactgg taatcatgaa cgttaccatg ttaggaggtc     10800
   acatggaaca tcagattctg gaaaacggga aaggttccgt tcaggacgct acttgtgtag     10860
   tttaaaccag ctgtctctta tacacatctg cctccggcaa aaaaacgggc aaggtgtcac     10920
   caccctgccc tttttcttta aaaccgaaaa gattacttcg cgtttgccac ctgacgtcta     10980
   agaaaaggaa tattcagcaa tttgcccgtg ccgaagaaag gcccacccgt gaaggtgagc     11040
   cagtgagttg attgctacgt aattagttag ttaggcc                              11077
<210> 20
   <211> 11077
   <212> DNA
   <213> unknown
<220>
   <223> Synthetic
<400> 20
   aattgtgagc ggataacaat tacgagcttc atgcacagtg aaatcatgaa aaatttattt 60
   gctttgtgag cggataacaa ttataatatg tggaattgtg agcgctcaca attccacaac 120
   ggtttccctc tagaaataat tttgtttaac ttttgcccta taaccagacc gttcagcgtt 180
   ttagagctag aaatagcaag ttaaaataag gctagtccgt tatcaacttg aaaaagtggc 240
   accgagtcgg tggtgcgccc caggcatcaa ataaaacgaa aggctcagtc gaaagactgg 300
   gcctttcgtt ttatctgttg tttgtcggtg aacgctctct actagagtca cactggctca 360
   ccttcgggtg ggcctttctg cgtttatagc ccacagcaaa caccacgtcg accctatcag 420
   ctgcgtgctt tctatgagtc gttgctgcat aacttgacaa ttaatcatcc ggctcgtaat 480
   gtttgtggag ccgggcccaa gttcacttaa aaaggagatc aacaatgaaa gcaattttcg 540
   tactgaaaca tcttaatcat gctaaggagg ttttctaatg gtaagcaagg gcgaggagaa 600
   taacatggcc atcatcaagg agttcatgcg cttcaaggtg cacatggagg gcagtgtgaa 660
   cggccacgag ttcgagatcg agggcgaggg cgagggccgt ccctacgagg cctttcagac 720
   cgctaagctg aaggtgacca agggtggccc cctgcccttc gcctgggaca tcctgtcccc 780
   tcagttcatg tacggctcca aggtctacat taagcaccca gccgacatcc ccgactactt 840
   caagctgtcc ttccccgagg gcttcaggtg ggagcgcgtg atgaacttcg aggacggcgg 900
   cattattcac gttaaccagg acagttccct gcaagacggc gtgttcatct acaaggtgaa 960
   gctgcgcggc accaacttcc ccagtgacgg ccccgtaatg cagaagaaga ccatgggctg 1020
   ggaggccagt gaggagcgga tgtaccccga ggacggcgcc ctgaagtctg agatcaaaaa 1080
   gaggctgaag ctgaaggacg gcggccacta cgccgccgag gtcaagacca cctacaaggc 1140
   caagaagccc gtgcagctgc ccggcgccta catcgtcgac atcaagttgg acatcgtgtc 1200
   ccacaacgag gactacacca tcgtggaaca gtacgaacgc gccgagggcc gccactccac 1260
   cggcggcatg gacgagctgt acaaggccga caagaaatac tcgatagggc tggatatcgg 1320
   gaccaactcc gtcggttggg ctgttatcac ggatgaatat aaggtgccca gcaaaaagtt 1380
   caaggtgcta ggtaacaccg accggcacag tatcaaaaaa aacttgatag gagcgttgct 1440
   gtttgacagt ggtgagaccg ctgaagctac gcgccttaaa aggaccgcgc gccgtagata 1500
   tacccgcagg aagaaccgca tttgctatct ccaagagatt ttttcgaacg agatggctaa 1560
   agtagatgat agtttctttc atcgattgga agaaagcttt ttagttgaag aagacaagaa 1620
   gcatgaacgc catccaattt ttggtaacat agtagatgaa gtggcgtatc atgagaaata 1680
   tccgacgatc tatcatctac gaaaaaaatt agtagatagc acggataaag ctgatttacg 1740
   gctgatctat ttagcactgg ctcatatgat taagtttcgc ggccattttt taattgaggg 1800
   tgatctgaac ccagataaca gtgatgttga caaactcttt atccaattag tacagactta 1860
   caaccagctg tttgaagaaa atccaattaa tgccagtggt gtagatgcga aagctatttt 1920
   gagcgcccga ttaagtaaat cgcgtcgact ggaaaacctt attgcgcaac ttcctggcga 1980
   gaagaaaaac gggctgtttg gaaaccttat tgcgttatcg ttaggcttaa ctccaaactt 2040
   taaatcgaac tttgatttag ccgaagatgc gaaactgcaa ttgtcgaaag atacgtacga 2100
   tgatgatctg gataacctgt tagctcagat tggtgatcag tatgcggatt tatttttagc 2160
   cgcgaagaac ctgtcggatg cgattctgtt gtcggatatc ctccgtgtaa acacggaaat 2220
   aacgaaggcg cctctctcgg cgtcgatgat taaacggtac gatgaacatc atcaggactt 2280
   aacgttgctg aaagcgctgg tgcgacagca gttgccggaa aagtataaag aaatcttttt 2340
   tgatcagtcg aaaaatggtt atgccggcta tattgatgga ggtgcgtccc aggaagaatt 2400
   ttataaattt atcaaaccga ttctggaaaa aatggatggc acggaggaac tgttggttaa 2460
   actcaaccgc gaagatttgc tacggaagca gaggactttt gacaatggga gcattcctca 2520
   tcagattcac ttgggcgagc tacatgcgat tttgcgtcgt caggaagact tttatccgtt 2580
   tctgaaagac aaccgcgaga agattgaaaa aatcttgacg tttcgaatcc catattatgt 2640
   gggcccgtta gctcgcggga acagtcgctt tgcctggatg acgaggaagt cggaagaaac 2700
   cattactccg tggaactttg aagaagtggt cgataaaggc gcgtcggcgc agtcgtttat 2760
   tgaacggatg accaattttg ataaaaactt gccgaacgaa aaagtactcc cgaaacatag 2820
   tttattgtat gagtatttta cagtgtataa tgaattaacc aaggtcaaat atgtgacgga 2880
   aggtatgcga aaaccggcct tttgtcgggc cgaacaaaag aaagcaattg tggatctgct 2940
   tttcaaaacc aaccgaaaag taactgtgaa gcagctgaaa gaagattatt tcaaaaaaat 3000
   agaatgcttt gatagtgtgg aaatttcggg tgtggaagat cgttttaacg cgtcgctggg 3060
   cacttaccat gatttactca aaattattaa agataaagat tttttagata acgaagaaaa 3120
   cgaagatatc ctggaggata ttgtgctgac cttaactctg tttgaagata gagagatgat 3180
   tgaggaacgt ttgaaaacct atgcgcacct ttttgatgat aaggttatga aacaattgaa 3240
   acgccggcgc tatacgggct ggggtcgctt aagccgaaaa ttaattaacg gcattagaga 3300
   taagcagagc gggaaaacca tactggattt tttaaaatcg gatggctttg caaaccggaa 3360
   ctttatgcaa ctaatccatg atgatagttt aacctttaaa gaagacattc agaaagccca 3420
   ggttagcggt cagggggata gtctgcatga acatattgcc aacctggcgg gctccccagc 3480
   gattaaaaaa ggcattctgc aaacggtaaa agtggtggat gaattagtca aagtaatggg 3540
   aaggcataag ccggaaaaca tcgtgattga aatggcccgc gaaaaccaaa ccacgcagaa 3600
   ggggcaaaaa aactcacgag agcgcatgaa acgaatcgaa gaaggcatca aagaactggg 3660
   tagtcaaatt ttgaaagagc atccagtgga aaacacgcag ttacagaacg aaaagcttta 3720
   tctttattat cttcagaacg gtcgtgacat gtatgttgac caggaactgg atattaaccg 3780
   cctgagtgat tatgatgtcg atcacattgt gccgcagagt ttcttgaaag acgattcgat 3840
   agacaacaag gtcctgacac gcagcgataa aaaccgcggc aaatcagata atgtgccgag 3900
   tgaagaagta gtcaaaaaga tgaaaaatta ttggcgtcag ttgctcaatg caaagctgat 3960
   cacgcagcgc aagtttgata acctgacaaa agcggaacgc ggtggcttaa gtgaattgga 4020
   taaagcgggc tttatcaaac ggcagttagt ggaaacgcgg cagatcacga agcatgttgc 4080
   ccagatttta gatagtcgga tgaacacgaa atacgatgaa aacgataaat tgattcgaga 4140
   ggtgaaagtt attactctga aaagcaaact ggtgagcgac ttccgaaaag atttccagtt 4200
   ctataaagta cgcgagatta ataactacca tcatgcacat gatgcttatc tcaacgcagt 4260
   cgtgggtacg gcgttaatta agaaatatcc gaaattggaa tcagagtttg tctatggcga 4320
   ttataaagtg tatgatgtgc gcaaaatgat tgcgaagtcg gagcaggaaa tagggaaagc 4380
   cactgccaaa tatttctttt acagcaacat catgaatttc ttcaaaaccg aaattacctt 4440
   ggccaacggt gagattcgga aacggccact catcgaaacg aacggagaaa cgggtgaaat 4500
   tgtctgggat aaaggacgag attttgcaac cgttcggaaa gtattgtcca tgcctcaggt 4560
   caacattgtc aagaaaaccg aagtacaaac cgggggtttc tccaaggagt cgattctgcc 4620
   gaaacgcaac tcagacaagt tgattgcgcg caaaaaagac tgggatccga aaaaatatgg 4680
   cggcttcgat tccccgacag tagcgtactc ggtcctcgtc gtggcgaagg tcgaaaaagg 4740
   gaaatccaag aagctgaaat ccgtgaaaga gctgctcggg atcaccatca tggagcgctc 4800
   ctccttcgag aagaacccca tcgacttcct ggaggcgaag ggctacaagg aggtgaagaa 4860
   ggacctgatc atcaagctcc ccaagtactc cctctttgag ctggagaacg gccgcaagag 4920
   gatgctcgca agtgcaggtg aattacaaaa aggtaatgaa ctagcgctac cgtccaaata 4980
   tgttaacttt ctgtatctgg cgagtcatta tgaaaagtta aagggcagtc cggaagataa 5040
   tgaacagaaa cagttatttg ttgagcaaca taagcattat ctggatgaga ttattgagca 5100
   gatcagtgaa tttagcaagc gcgtgattct ggccgatgca aacctggata aagtgttgag 5160
   tgcctataat aaacatcgtg acaaaccgat acgcgaacag gccgaaaaca ttattcatct 5220
   gtttacatta acaaacttgg gtgcgcctgc ggcgtttaaa tattttgata ccaccattga 5280
   tcgcaaacga tatacaagca ccaaagaagt gctggatgca acgttgatcc atcagtctat 5340
   cacgggcttg tatgaaaccc ggattgattt aagtcaactc ggtggcgact aagcccggcc 5400
   gctactagta acaaaaaacc cctagccccc cgttttatat cactgcccgc tttccagtcg 5460
   ggaaacctgt cgtgccagct gcattaatga atcggccaac gcgcggggag aggcggtttg 5520
   cgtattgggc gccagggtgg tttttctttt caccagtgag actggcaaca gctgattgcc 5580
   cttcaccgcc tggccctgag agagttgcag caagcggtcc acgctggttt gccccagcag 5640
   gcgaaaatcc tgtttgatgg tggttaacgg cgggatataa catgagctat cttcggtatc 5700
   gtcgtatccc actaccgaga tatccgcacc aacgcgcagc ccggactcgg taatggcgcg 5760
   cattgcgccc agcgccatct gatcgttggc aaccagcatc gcagtgggaa cgatgccctc 5820
   attcagcatt tgcatggttt gttgaaaacc ggacatggca ctccagtcgc cttcccgttc 5880
   cgctatcggc tgaatttgat tgcgagtgag atatttatgc cagccagcca gacgcagacg 5940
   cgccgagaca gaacttaatg ggcccgctaa cagcgcgatt tgctggtgac ccaatgcgac 6000
   cagatgctcc acgcccagtc gcgtaccgtc ctcatgggag aaaataatac tgttgatggg 6060
   tgtctggtca gagacatcaa gaaataacgc cggaacatta gtgcaggcag cttccacagc 6120
   aatggcatcc tggtcatcca gcggatagtt aatgatcagc ccactgacgc gttgcgcgag 6180
   aagattgtgc accgccgctt tacaggcttc gacgccgctt cgttctacca tcgacaccac 6240
   cacgctggca cccagttgat cggcgcgaga tttaatcgcc gcgacaattt gcgacggcgc 6300
   gtgcagggcc agactggagg tggcaacgcc aatcagcaac gactgtttgc ccgccagttg 6360
   ttgtgccacg cggttgggaa tgtaattcag ctccgccatc gccgcttcca ctttttcccg 6420
   cgttttcgca gaaacgtggc tggcctggtt caccacgcgg gaaacggtct gataagagac 6480
   accggcatac tctgcgacat cgtataacgt tactggtttc atattcacca ccctgaattg 6540
   actgtcttcc gggcgctatc atgccatacc gcgaaaggtt ttgcgccatt cgatggcgcg 6600
   ccgcgccctg cgctagcatg cctatttgtt tatttttcta aatacattca aatatgtatc 6660
   cgctcatgag acaataaccc tgataaatgc ttcaataata ttgaaaaagg aacagtatga 6720
   gccatattca acgggaaacg tcttgctcca ggccgcgatt aaattccaac atggatgctg 6780
   atttatatgg gtataaatgg gctcgcgata atgtcgggca atcaggtgcg acaatctatc 6840
   gattgtatgg gaagcccgat gcgccagagt tgtttctgaa acatggcaaa ggtagcgttg 6900
   ccaatgatgt tacagatgag atggtcagac taaactggct gacggaattt atgcctctcc 6960
   cgaccatcaa gcattttatc cgtactcctg atgatgcatg gttactcacc actgcgatcc 7020
   ccgggaaaac agcattccag gtattagaag aatatcctga ttcaggtgaa aatattgttg 7080
   atgcgctggc agtgttcctg cgccggttgc attcgattcc tgtttgtaat tgtcctttta 7140
   acagcgatcg cgtatttcgt ctcgctcagg cgcaatcacg aatgaataac ggtttggttg 7200
   atgcgagtga ttttgatgac gagcgtaatg gctggcctgt tgaacaagtc tggaaagaaa 7260
   tgcataagct tttgccattc tcaccggatt cagtcgtcac tcatggtgat ttctcacttg 7320
   ataaccttat ttttgacgag gggaaattaa taggttgtat tgatgttgga cgagtcggaa 7380
   tcgcagaccg ataccaggat cttgccatcc tatggaactg cctcggtgag ttttctcctt 7440
   cattacagaa acggcttttt caaaaatatg gtattgataa tcctgatatg aataaattgc 7500
   agtttcattt gatgctcgat gagtttttct aagccttagt tagttagatt agcagaaagt 7560
   caaaagcctc cgaccggagg cttttgacta aaacttccct tggggttatc attggggctc 7620
   actcaaaggc ggtaatcaga taaaaaaaat ccttagcttt cgctaaggat gatttctgcc 7680
   agatgtgtat aagagacagc tgggcgcgcc ccatgtcagc cgttaagtgt tcctgtgtca 7740
   ctcaaaattg ctttgagagg ctctaagggc ttctcagtgc gttacatccc tggcttgttg 7800
   tccacaaccg ttaaacctta aaggctttaa aagccttata tattcttttt tttcttataa 7860
   aacttaaaac cttagaggct atttaagttg ctgatttata ttaattttat tgttcaaaca 7920
   tgagagctta gtacgtgaaa catgagagct tagtacgtta gccatgagag cttagtacgt 7980
   tagccatgag ggtttagttc gttaaacatg agagcttagt acgttaaaca tgagagctta 8040
   gtacgtgaaa catgagagct tagtacgtac tatcaacagg ttgaactgct gatcttcaga 8100
   tcggcgcgcc ggccggccta cggccagcct cgcagagcag gattcccgtt gagcaccgcc 8160
   aggtgcgaat aagggacagt gaagaaggaa cacccgctcg cgggtgggcc tacttcacct 8220
   atcctgcccg gctgacgccg ttggatacac caaggaaagt ctacacgaac cctttggcaa 8280
   aatcctgtat atcgtgcgaa aaaggatgga tataccgaaa aaatcgctat aatgaccccg 8340
   aagcagggtt atgcagcgga aaaggacaag gccctgcgct agcatgccta tttgtttatt 8400
   tttctaaata cattcaaata tgtatccgct catgagacaa taaccctgat aaatgcttca 8460
   ataatattga aaaaggaaca gtatgagtat tcaacatttc cgtgtcgccc ttattccctt 8520
   ttttgcggca ttttgccttc ctgtttttgc tcacccagaa acgctggtga aagtaaaaga 8580
   tgccgaagat cagttgggtg cacgtgtggg ttacatcgaa ctggacctca acagcggtaa 8640
   gattcttgag agttttcgcc ccgaagaacg tttcccaatg atgagcactt ttaaagttct 8700
   gctctgtggc gcggtattat cccgtattga cgccgggcaa gagcaactcg gtcgccgcat 8760
   acactattct cagaatgact tggttgagta ctcaccagtc acagaaaagc atcttacgga 8820
   cggcatgaca gtacgcgaat tatgcagcgc tgccataacc atgagtgata acacggcggc 8880
   caacttactt ctgacaacga tcggaggacc gaaggagctt accgcttttt tgcacaacat 8940
   gggtgatcat gtaactcgcc ttgatcgttg ggaaccggag ctgaatgaag ccataccaaa 9000
   cgacgagcgt gacaccacga tgcctgtagc tatggcaaca acgttgcgca aactcttaac 9060
   tggcgaactt cttactctcg cttcccggca acaattaata gactggatgg aggcggataa 9120
   agttgcagga ccacttctgc gctcggccct tccggctggc tggtttattg ctgataaatc 9180
   tggagccggt gagcgtgggt cccgcggtat tattgcagcc ctggggccag atggtaagcc 9240
   ctcccgtatc gtagttatct acacgacggg gagccaggca actatggacg aacgtaatcg 9300
   ccagatcgct gagataggtg cctccctgat taagcattgg taagccttag attttaatgc 9360
   cctgcgccat caggtctttc gcggccagaa agccatccag tttgctttgc agcgcttccc 9420
   aaccttccca cagcgcaccc cagctcgcaa tgccggtacg tttgctatcc ataaagccgc 9480
   ccagacgcgc aatcgccata tacgcccatt gcaggctgcc cgctttttct ttgcgtttgc 9540
   gtttgccttt atccagatag cccagcagtt ggcattcatc cggggtcagc acggtttccg 9600
   cgctctggct ttcaacgtgt tccgcttctt tcagcaggcc ctgcgcacgc agtgcttgcg 9660
   gcggagtaaa agattcacgc agttgcagca gacgcaccgc cacaaagctc agaatgctca 9720
   ccatacgttc caggttatcc ggttcttcca tacgctgacg ttccgcaccc gcacccgttt 9780
   tccacgcttt gtgaaattct tcaatgcgcc aacgatgggt ataaatatca atcacacgca 9840
   gcgcttgggc cagactttcc accggctcgc tggtcagcag cagccatttc agcggggttt 9900
   cgcctttcgg cggattaatt tcttcggcca gcaccgcgtt cagggtaatg ttgccctgtt 9960
   tcagggtaat acggccgcta cgcaggctca ggctcgcttt acgcgccgga cggtttttac 10020
   gtttgccacg tttatccacc acgcctttct gcggaatgct aatctgatag ccgcccagtt 10080
   ccggctggtt tttcaggtga tcatacagat acaggccgct ttccacatct ttacgcggat 10140
   gtttgctacg caccacaaaa cgttcgttat gggccagttt atcttgcaga tacgcatgaa 10200
   tatccgcttc acgatcgcac accgcaatca cgttgctcat catgctgccc attctcagac 10260
   gcgaagttgc agcagcggcc agccatttgc cgctttcttt ttcatccgca tccgccggat 10320
   catccggacg catccaccat tcttgatgca gcaggcccac ggtacgaaag gtggtcgctt 10380
   ccagcagcag cacgctatgc acccaccaac cacggctttt atcctgaatg ctacccagtt 10440
   tgcccagttc ttccgccacc tgatgacgat agctcagaga ggtggtatct tcaattgcca 10500
   gcagttccgg aaattcctgg gccagtttca cggtctgcat ggcacccgct ttacgaatcg 10560
   cttccgcgct cacgttcgga ttacgaataa aacgatacgc gccttcctgc atggctttgc 10620
   tgccttcgct gctaatggta atgcttttgc cgctatattt ggccagttgc gccgcaacat 10680
   tcaccagacg cgcggtacga cgcggatcac ccagcgcagc actagaaaac acgcttttcg 10740
   cccaatccgc cgcacgatgc agtgcactgg taatcatgaa cgttaccatg ttaggaggtc 10800
   acatggaaca tcagattctg gaaaacggga aaggttccgt tcaggacgct acttgtgtag 10860
   tttaaaccag ctgtctctta tacacatctg cctccggcaa aaaaacgggc aaggtgtcac 10920
   caccctgccc tttttcttta aaaccgaaaa gattacttcg cgtttgccac ctgacgtcta 10980
   agaaaaggaa tattcagcaa tttgcccgtg ccgaagaaag gcccacccgt gaaggtgagc 11040
   cagtgagttg attgctacgt aattagttag ttaggcc 11077

## Description

### TECHNICAL FIELD

The disclosure relates to systems, mechanisms and methods to develop an engineered genetic system that can be introduced into a cell such as a probiotic for gene editing. The enginerred probiotic can be used in, for example, the treatment of gastrointestinal, skin or urinary tract diseases and infections, combatting the spread of antibiotic resistance, and decontamination of environmental pathogens.

### BACKGROUND

Next generation sequencing technologies are allowing researchers to rapidly and accurately interrogate the genomic content of microbiomes and catalog both commensal and pathogenic microbes. Advances in our understanding of the mammalian microbiome are likely to lead to the use of next generation sequencing as a diagnostic tool to identify the existence and precise genotype of pathogens and virulence genes and distinguish between the microbiome composition and structure of healthy and diseased individuals.

Likewise, our ability to engineer microbes using synthetic biology and metabolic engineering tools and technologies has advanced to the point where we can begin to consider applying engineered microbes to restore healthy microbiome states. Engineered organisms are already used widely in human and veterinary health for therapeutic production, biomedical research, and more recently as products themselves. It is now possible to merge the fields of microbiome discovery and synthetic biology to develop new strategies to modulate human and animal health and disease.

One area of growing opportunity in this field is translating microbiome-based discovery into therapeutic impact by employing probiotics to modulate diseases and infections with a microbial component. Although not totally understood, many skin disorders are believed to have a microbial component. Lesions resulting from atopic dermatitis often become infected with pathogens like *Staphylococcus aureus.* Seborrhoeic dermatitis is believed to have a fungal component since treatment with fungicides is effective. Burn wounds are often infected with *Streptococcus pyogenes, Enterococcus* spp., or *Pseudomonas aeruginosa.*

Currently, many gastrointestinal disease states have been associated with changes in the composition of faecal and intestinal mucosal communities, including inflammatory bowel diseases (IBD and IBS), obesity and the metabolic syndrome. Probiotics, or beneficial microbes, are used to improve symbiosis between enteric microbiota and the host or to restore states of dysbiosis. Probiotics may modulate immune responses, provide key nutrients, or suppress the proliferation and virulence of infectious agents. In particular, the enteric microbiota are known to impact gastrointestinal health and the disruption of this homeostasis is associated with many disease states such as diarrhea. Diarrhea is defined by the WHO as the condition of having three or more loose or liquid bowel movements per day. The disease can be acute-usually due to an infectious agent-or chronic-usually associated with other medical conditions affecting the intestine such as IBD, IBS, and Crohn's Disease. Loss of microbial balance in the gastrointestinal tract is commonly associated with all forms of diarrhea. Thus, probiotics have garnered clinical attention as potential therapeutic or preventative treatments of the disease.

Unfortunately, the evidence of probiotic efficacy in clinical settings is only modest for the prevention of diarrhea and contradictory results are common likely due to differences in populations studied, the type of probiotic, duration of treatment and dosage [Guandalini, 2011]. Additionally, many probiotics are hindered by inherent physiological and technological weaknesses and often the most clinically promising strains are not suitable as therapeutics. The most common probiotics tested for their impact on diarrhea are *Lactobacillus, Bifidobacterium lactis,* and *Streptococcus,* either alone or in combination with each other. Because of these variables, it is unlikely that the current wild type probiotics will be viable candidates for successful therapeutic interventions for diarrhea.

The treatment of gastrointestinal infections has been further complicated by the rise of antibiotic resistance. Over 70% of hospital bacterial infections harbor resistance to one or more classes of antibiotics. The prevalence of antibiotic-resistant pathogenic microbial infection stems from a confluence of practices and policies. To date, the rise of drug resistant pathogens has been addressed by improved containment practices, judicious use of antibiotics, and government-sponsored antibiotic research and development programs. Despite these efforts, the spread of antibiotic resistance continues to be a significant and growing threat.

Urinary tract infections affect 50% of women and 12% of men at least once in their lifetimes, with 80% of these infections caused by a group of *Escherichia coli* known as uropathogenic *E. coli* (UPEC) [Brumbaugh, 2012]. Similar to gastrointestinal infections, UPEC infections are often complicated by resistance to multiple antibiotics. 25% of women with urinary tract infections suffer from a recurrent infection within 6 to 12 months of the initial infection, and 3% of all women suffer from persistently recurring urinary tract infections. Prophylactic antibiotics are the current course of treatment for women with persistently recurring urinary tract infections; rising rates of antibiotic resistance are already driving physicians to abandon first- and second-line antibiotics. In addition to the complications of persistent UPEC infections, comorbidities such as secondary yeast infections and gastrointestinal infections increase the importance of developing new treatments.

Accordingly, new strategies for the treatment of skin, gastrointestinal or urinary tract disease and infection, including those stemming of drug resistant microbes, are needed. Furthermore, the ability to tailor a probiotic to target a specific pathogen or toxin would offer a novel therapy for skin, gastrointestinal or urinary tract disease and/or infection. In addition, the ability to endow the probiotic with the ability to target drug resistant microbes would be of significant therapeutic value.

Beyond the microbiome, there is also a need to decontaminate areas that harbor antibiotic resistant or otherwise pathogenic bacteria. Animal feed has been identified as a source of drug resistant microbes entering the food supply [Allen, 2014]. Subtherapeutic levels of antibiotics are commonly used as animal feed additives; this practice has exacerbated the spread of antibiotic resistant microbes in agriculture and in clinical settings [Silbergeld 2008]. Engineered probiotics targeting drug resistant microbes in livestock or animal feed would be a new strategy to controlling the spread of antibiotic resistance genes in the food supply.

Strategies to treat animal feed with beneficial probiotics can likewise be adapted to the decontamination of other environments that harbor pathogenic bacteria. Probiotics can be designed to target pathogenic bacteria that have been used as biological weapons, such as Bacillus anthracis. These probiotics could be precisely targeted to select agents via topical application and/or ingestion of the probiotic, and by designing the the probiotic to target gene sequences unique to the select agent of concern. These approaches could also be adapted to the decontamination of environmental sites that were contaminated by select agent bacteria.

### SUMMARY

Systems and methods of the present disclosure provide for engineered genetic systems with many applications, such as for use in the treatment of diseases and infections using engineered probiotics. Furthermore, systems and methods of the present disclosure can be used to reduce or eliminate antibiotic resistance, the spread of antibiotic resistance, and/or the spread of pathogenic elements, within or beyond a microbial community.

In one aspect, an engineered genetic system is provided, comprising: a nuclease module designed to specifically target and degrade a nucleic acid of interest encoding a virulence factor, toxin, effector, pathogenic component and/or antibiotic resistance trait; and a synthetic mobile genetic element (MGE) module capable of dispersing the system from one host cell to another; wherein the nuclease module comprises a nuclease encoded by a gene located in the MGE module, wherein the nuclease module comprises: i) a Cas protein and one or more synthetic crRNAs wherein each crRNA comprises a spacer having a target sequence derived from the nucleic acid of interest; ii) a Transcription Activator-Like Effector Nuclease (TALEN) designed to target and degrade the nucleic acid of interest; iii) a Zinc Finger Nuclease (ZFN) designed to target and degrade the nucleic acid of interest; or iv) a meganuclease designed to target and degrade the nucleic acid of interest; and an antibiotic-free maintenance module, wherein the antibiotic-free maintenance module comprises one or more nucleic acid molecules comprising a carbon utilization operon. The engineered genetic system can be used to target and degrade the nucleic acid of interest within an organism such as a bacterial cell.

In some embodiments, the nuclease module comprises a Cas protein and one or more synthetic crRNAs wherein each crRNA comprises a spacer having a target sequence derived from the nucleic acid of interest. The Cas protein can be expressed constitutively or inducibly. The Cas protein may, in one example, be expressed from SEQ ID NO:1. In one example, the Cas protein is *Streptomyces pyogenes* Cas9 nuclease. The crRNA(s) can be transcribed and processed from a CRISPR array which may be placed under the control of an inducible promoter or a constitutive promoter. In one example, the CRISPR array has SEQ ID NO:3. In some embodiments, the nuclease module can further include a tracrRNA that forms a complex with the Cas protein and crRNA. The tracrRNA may be placed under the control of an inducible promoter or a constitutive promoter. In one example, the tracrRNA is transcribed from SEQ ID NO:2. In certain embodiments, the tracrRNA and crRNA can be provided in a single guide RNA. In certain embodiments, the system can include multiple guide RNAs. These guide RNAs may target a single gene at multiple nucleotide positions, or they may target multiple genes of interest for degradation.

The nucleic acid of interest can be DNA or RNA. In some embodiments, the target sequence can be immediately adjacent to a Protospacer Associated Motif (PAM) in the nucleic acid of interest. When the Cas protein is *Streptomyces pyogenes* Cas9 nuclease, the PAM can have the NGG sequence that is 3' of the target sequence.

In various embodiments, the nuclease can include a Transcription Activator-Like Effector Nuclease (TALEN) designed to target and degrade the nucleic acid of interest, a Zinc Finger Nuclease (ZFN) designed to target and degrade the nucleic acid of interest, and/or a meganuclease designed to target and degrade the nucleic acid of interest.

In some embodiments, the virulence factor, toxin, effector, pathogenic component and/or antibiotic resistance trait are selected from those listed in Tables 1 and 2. For example, the virulence factor can be a colonization or fitness factor.

The MGE module, in some embodiments, comprises a gene encoding a transposase and a MGE selected from a bacteriophage, conjugative plasmid, or conjugative transposon. For example, the MGE can be derived from Tn916, RK2, PI, Tn5280, or Tn4651.

In some embodiments, one or more CRISPR elements may be combined with an MGE in one plasmid to facilitate transfer between bacterial cells. The plasmid may further be designed as in SEQ ID NO:17 or SEQ ID NO:18.

In some embodiments, CRISPR elements may be combined with an MGE to facilitate transfer between bacterial cells, including a transposase that allows transfer of the CRISPR elements to the genome of the recipient cell. For example, the transposase can be derived from the Tn3 or Tn5 transposable elements. Two such designs are provided as SEQ ID NO:19 and SEQ ID NO:20.

The present disclosure also provides an engineered organism comprising the engineered genetic system disclosed herein, for use in the prevention and/or treatment of a disease or infection, the prevention and/or treatment of antibiotic resistance, limiting the spread of antibiotic resistance, and/or decontamination of environmental pathogens. In some embodiments, the engineered genetic system is introduced into a host selected from a bacterial cell, archaea cell and/or yeast cell.

In yet another aspect, an engineered probiotic comprising the engineered organism described herein is provided. The engineered probiotic can be an oral probiotic for use in the gastrointestinal tract, a probiotic for use in the urinary tract, and/or a topical probiotic for use on the skin.

In various embodiments, the engineered probiotic for use in the gastrointestinal tract and/or in the urinary tract can be based on a host selected from Bacteroidetes, Firmicutes, Proteobacteria, Actinobacteria, Verrucomicrobia or Fusobacteria divisions of Bacteria. For example, the host may be selected from *Bacteroides* species including *Bacteroides* AFS519, *Bacteroides* sp. CCUG 39913, *Bacteroides* sp. Smarlab 3301186, *Bacteroides ovatus, Bacteroides salyersiae, Bacteroides* sp. MPN isolate group 6, *Bacteroides* DSM 12148, *Bacteroides merdae, Bacteroides distasonis, Bacteroides stercosis, Bacteroides splanchnicus, Bacteroides* WM2, *Bacteroides uniformis, Bacteroides* WH302, *Bacteroides fragilis, Bacteroides caccae, Bacteroides thetaiotamicron, Bacteroides vulgatus,* and *Bacteroides capillosus.* The host can also be selected from *Clostridium* species including *Clostridium leptum, Clostridium boltaea, Clostridium bartlettii, Clostridium symbiosum, Clostridium* sp. DSM 6877(FS41), *Clostridium* A2-207, *Clostridium scindens, Clostridium spiroforme, Clostridium* sp. A2-183, *Clostridium* sp. SL6/1/1, *Clostridium* sp. GM2/1, *Clostridium* sp. A2-194, *Clostridium* sp. A2-166, *Clostridium* sp. A2-175, *Clostridium* sp. SR1/1, *Clostridium* sp. L1-83, *Clostridium* sp. L2-6, *Clostridium* sp. A2-231, *Clostridium* sp. A2-165 and *Clostridium* sp. SS2/1. The host may also be selected from *Eubacterium* species including *Eubacterium plautii, Eubacterium ventriosum, Eubacterium halii, Eubacterium siraeum, Eubacterium eligens,* and *Eubacterium rectale.* In some embodiments, the host is selected from *Alistipesfinegoldii, Alistipes putredinis, Anaerotruncus colihominis, Allisonella histaminiformans, Bulleida moorei, Peptostreptococcus* sp. oral clone CK035, *Anaerococcus vaginalis, Ruminococcus bromii, Anaerofustis stercorihominis, Streptococcus mitis, Ruminococcus callidus, Streptococcus parasanguinis, Coprococcus eutactus, Gemella haemolysans, Peptostreptococcus micros, Ruminococcus gnavus, Coprococcus catus, Roseburia intestinalis, Roseburiafaecalis, Ruminococcus obeum, Catenibacterium mitsuokai, Ruminococcus torques, Subdoligranulum variabile, Dorea formicigenerans, Dialister* sp. E2_20, *Dorea longicatena, Faecalibacterium prausnitzii, Akkermansia muciniphila, Fusobacterium* sp. oral clone R002, *Escherichia coli, Haemophilus parainfluenziae, Bilophila wadsworthii, Desulfovibrio piger, Cornyebacterium durum, Bifidobacterium adolescentis, Actinomyces graevenitzii, Cornyebacterium sundsvallense, Actinomyces odontolyticus,* and *Collinsella aerofaciens.* In certain embodiments, the host is selected from the genus *Lactobacillus, Bifidobacterium,* and/or *Streptococcus.* For example, the host can be selected from *Lactobacillus casei, Lactobacillus lactis, Lactobacillus reuteri, Lactobacillus rhamnosus, Lactobacillus acidophilus, Lactobacillus plantarum, Lactobacillus paracasei, Lactobacillus bulgaricus, Lactobacillus fermentum* and *Lactobacillus johnsonii.* The host may also be selected from *Bacillus coagulans* GBI-30, 6086, *Bifidobacterium animalis* subsp. *lactis* BB-12, *Bifidobacterium longum* subsp. *infantis* 35624, *Lactobacillus paracasei* St11 (or NCC2461), *Lactobacillus johnsonii* La1 *(Lactobacillus johnsonii* NCC533), *Lactobacillus plantarum* 299v, *Lactobacillus reuteri* ATCC 55730, *Lactobacillus reuteri* DSM 17938, *Lactobacillus reuteri* ATCC PTA 5289, *Saccharomyces boulardii, Lactobacillus rhamnosus* GR-1, *Lactobacillus reuteri* RC-14, *Lactobacillus acidophilus* CL1285, *Lactobacillus casei* LBC80R, *Lactobacillus plantarum* HEAL 9, *Lactobacillus paracasei* 8700:2, *Streptococcus thermophilus, Lactobacillus paracasei* LMG P 22043, *Lactobacillus johnsonii* BFE 6128, *Lactobacillus fermentum* ME-3, *Lactobacillus plantarum* BFE 1685, *Bifidobacterium longum* BB536 and *Lactobacillus rhamnosus* LB21 NCIMB 40564. In one embodiment, the host is selected from an *Escherichia coli* strain, such as *E. coli* HS, *E. coli* SE11, *E. coli* SE15, *E. coli* W, and *E. coli* Nissle 1917. In various embodiments, the host may be a clinical or environmental isolate of a bacterial strain.

In some embodiments, the engineered probiotic for use on the skin can be based on a host which is selected from the genera *Staphylococcus, Propionibacterium, Malassezia, Corynebacterium, Brevibacterium, Lactococcus, Lactobacillus, Micrococcus, Debaryomyces,* and *Cryptococcus.* For example, the host may be selected from *Staphylococcus epidermis, Staphylococcus saprophyticus, Propionibacterium acnes, Propionibacterium avidum, Lactococcus lactis, Lactobacillus reuteri* and *Lactobacillus plantarum.*

A method for prevention and/or treatment of a disease or infection, for prevention and/or treatment of antibiotic resistance, and/or for limiting the spread of antibiotic resistance may include administering an effective amount of the engineered probiotic described herein to a subject in need thereof. The subject can be a human or an animal.

In still another aspect, the above systems and methods can be used to limit the occurrence or spread of virulence factors, pathogenic elements and/or antibiotic resistance genes in a microbial population at an environmental site. In some embodiments, the environmental site is animal feed, farm or other material or location where animals or livestock frequent. In other embodiments, the environmental site is a building or location where humans frequent, such as a hospital or other clinical settings.

Also provided herein is a population of cells, comprising at least one engineered organism or engineered probiotic disclosed herein, wherein the MGE module in the at least one engineered organism or probiotic is capable of spreading the engineered genetic system into other cells in the population. Overtime, the population of cells will be subject to the engineered genetic system which can target and degrade the nucleic acid of interest in the population of cells. This way, "vaccination" of a population of cells with one engineered cell or a small group of cells can effectively combat or eleminate undesirable trais of the population of cells, thereby achieving, for example, the treatment of gastrointestinal, skin or urinary tract diseases and infections, prevention of the spread of antibiotic resistance, and/or decontamination of emvironmental pathogens

### BRIEF DESCRIPTION OF THE FIGURES

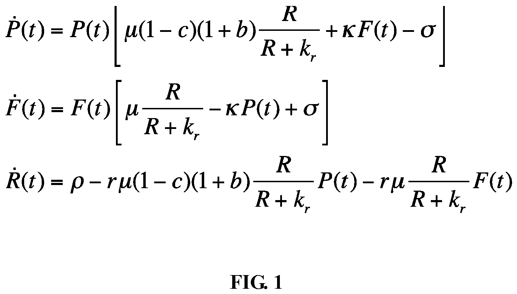

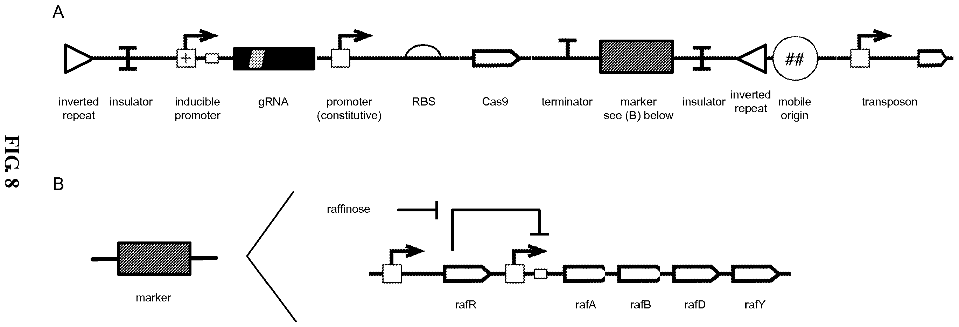

FIG. 1 lists three equations that comprise a basic model to describe the spread of the gene targeting and degradation system from the engineered probiotic to other members of a microbial community. P(t) and F(t) denotes the subpopulations with and without the gene targeting and degradation system, respectively. R(t) denotes the pool of growth resources available. Other variables are defined in Table 11.

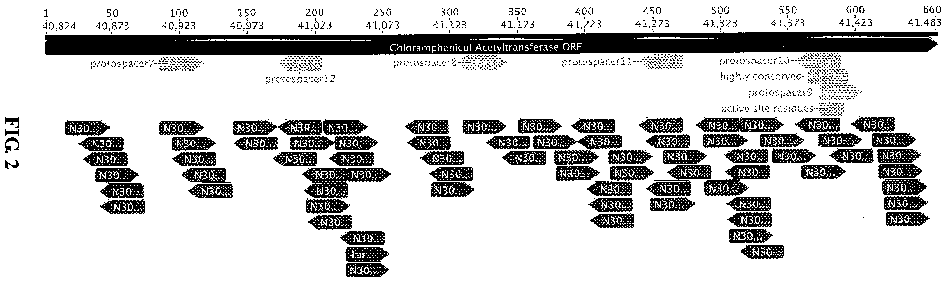

FIG. 2 depicts a schematic of the *Yersinia pestis* biovar Orientalis str IP275 chloramphenicol acetyltransferase coding sequence (CAT, Genbank accession NC_009141 40824...41483). Sites suitable for targeting with the *S. pyogenes* Cas9 nuclease are annotated in dark gray. Selected target sites and gene features of interest are annotated in light gray.

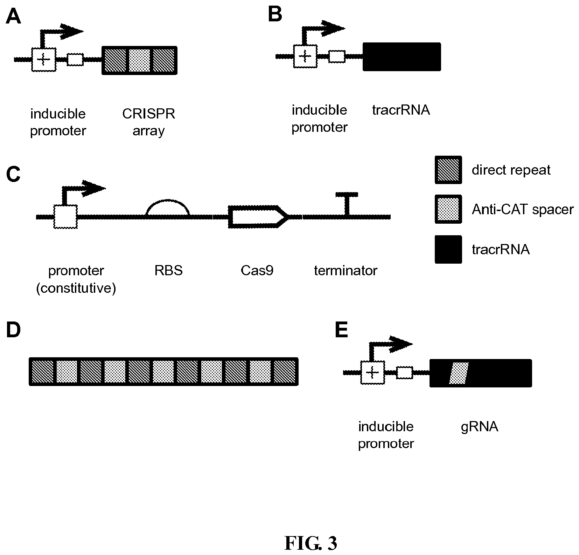

FIGS. 3A-3E depict exemplary designs of the three elements of a CRISPR/Cas gene targeting system: the CRISPR array transcription cassette (FIG. 3A), the tracrRNA transcription cassette (FIG. 3B), and the Cas9 expression cassette (FIG. 3C). The CRISPR array may contain one or more spacers: a one spacer design is shown in (FIG. 3A) while a five spacer design is shown in (FIG. 3D). In an alternative design, the tracrRNA and target spacer are combined into a single guide RNA or gRNA transcription cassette (FIG. 3E).

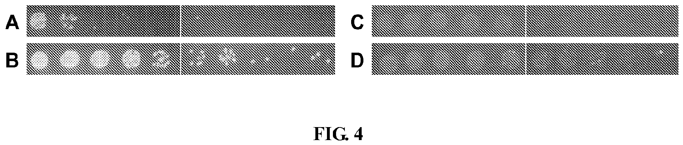

FIGS. 4A-4D show the results of challenging an engineered probiotic strain versus a control strain with a plasmid encoding an antibiotic resistance trait of interest *(Yersinia pestis* biovar Orientalis str IP275 chloramphenicol acetyltransferase coding sequence or CAT, SEQ ID NO:4). The challenge plasmid is also designed to encode a fluorescent protein for convenience of observation. The engineered probiotic strain encodes a CRISPR/Cas gene targeting and degradation system comprising a Cas9 expression cassette (SEQ ID NO:1), a tracrRNA (SEQ ID NO:2) and a CRISPR array (SEQ ID NO:3). The system is designed to target the CAT gene. The control strain is similar to the engineered probiotic strain but it omits the CRISPR array (SEQ ID NO:3). The engineered probiotic strain and control strain are challenged via transformation. The engineered probiotic has a reduced number of colonies after transformation with the plasmid and growth on chloramphenicol plates (FIG. 4A) relative to the control strain (FIG. 4B) indicating the efficacy of the gene targeting and degradation system. In FIG. 4C and FIG. 4D, results from a similar experiment are shown except that transformed cells were grown on kanamycin plates which selects for one (but not all) of the CRISPR components. Cell viability is not reduced, and no fluorescent colonies are visible in FIG. 4C indicating that the engineered probiotic is 100% effective in the absence of selection of the transformed plasmid.

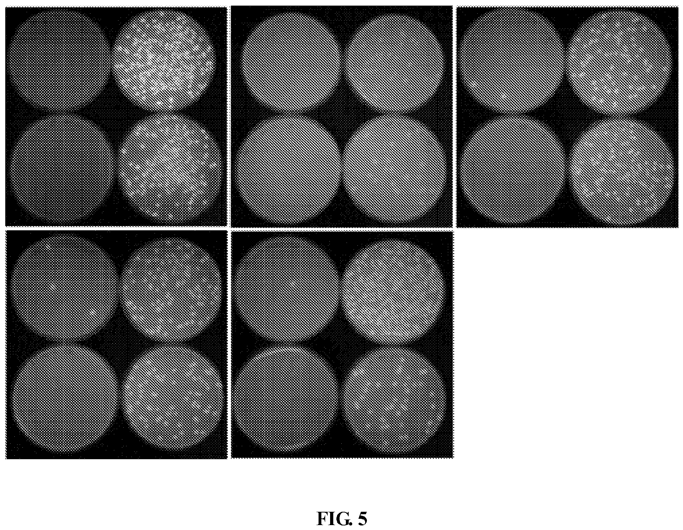

FIG. 5 shows the results of challenging an engineered probiotic strain versus a control strain with a set of five different plasmids each encoding an antibiotic resistance trait of interest *(Yersinia pestis* biovar Orientalis str IP275 chloramphenicol acetyltransferase coding sequence or CAT, SEQ ID NOs:4-8). All of the challenge plasmids are also designed to encode various fluorescent proteins for convenience. The engineered probiotic strain encodes a CRISPR/Cas gene targeting and degradation system comprising a Cas9 expression cassette (SEQ ID NO:1), a tracrRNA (SEQ ID NO:2) and a CRISPR array (SEQ ID NO:3). The system is designed to target the CAT gene. The control strain is similar to the engineered probiotic strain but it omits the CRISPR array (SEQ ID NO:3). The engineered probiotic strain and control strain are challenged via transformation (top row of panels corresponds to SEQ ID NO:4-6, bottom row of panels corresponds to SEQ ID NOs:7-8). The engineered probiotic has a reduced number of colonies after transformation with the plasmid and growth on chloramphenicol plates (left in each panel) than the control strain (right in each panel). Each panel includes replicate results for each challenge experiment (top and bottom of each panel).

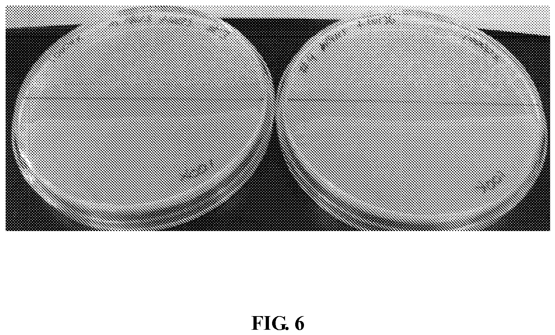

FIG. 6 shows the results of challenging an engineered probiotic strain versus a control strain with a plasmid that encodes an antibiotic resistance trait of interest that is not the target of the engineered probiotic. The engineered probiotic strain encodes a CRISPR/Cas gene targeting and degradation system comprising a Cas9 expression cassette (SEQ ID NO:1), a tracrRNA (SEQ ID NO:2) and a CRISPR array (SEQ ID NO:3). The system is designed to target the *Yersinia pestis* biovar Orientalis str IP275 chloramphenicol acetyltransferase coding sequence. The control strain is similar to the engineered probiotic strain but it omits the CRISPR array (SEQ ID NO:3). The engineered probiotic strain and control strain are challenged via transformation with a plasmid encoding a tetracycline resistance gene (SEQ ID NO:9). The engineered probiotic strain (left) and control strain (right) show no observable difference in the number of colonies after transformation with the plasmid and growth on tetracycline plates. The top half of each plate is a 1:1000 dilution of the transformation mix plated on the bottom half of each plate. The similar colony densities per unit area indicates that the engineered probiotic does not suffer from reduced cell viability or competence relative to the control strain.

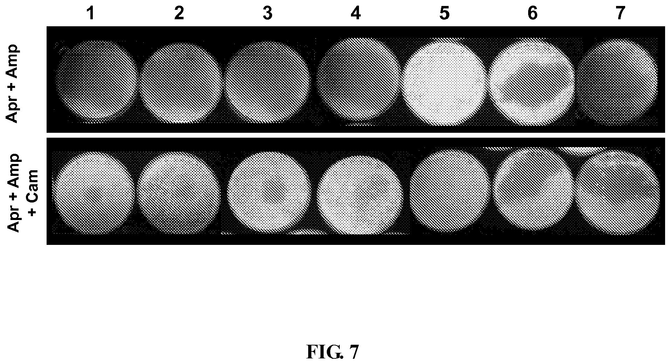

FIG. 7 shows the results of challenging a target strain with various guide RNAs (gRNAs) targeted at difference sequences to test the ability of CRISPR/Cas gene targeting and degradation systems to remove preexisting undesirable genes. The target strain comprises a low copy plasmid encoding a Cas9 expression cassette (SEQ ID NO:1) and a high copy plasmid encoding a *Yersinia pestis* biovar Orientalis str IP275 chloramphenicol acetyltransferase coding sequence (CAT) and a fluorescent protein (SEQ ID NO:4). Each column shows challenge via transformation results from a different gRNA construct. The gRNAs in columns 1-4 (SEQ ID NO:10-13) and 6-7 (SEQ ID NO:15-16) were targeted against the CAT gene whereas the gRNA (SEQ ID NO:14) in column 5 was targeted against an off-target gene not present in the target strain. Columns 1-4 and 6-7 vary in the identity of the promoter driving transcription of the gRNA, the plasmid, and the target site of the gRNA. The top row shows results from transformants plated on apramycin and ampicillin which selected for the Cas9 and gRNA plasmids, respectively. The bottom row shows results from transformants plated on apramycin, ampicillin, and chloramphenicol. Uneven distributions of colony growth reflect variations in antibiotic concentration across the agar plate.
FIGA. 8A-8B depict schematics of exemplary designs of an engineered probiotic. FIG. 8A depicts the complete mobilizable gene targeting and degradation system including the antibiotic-free selection and containment mechanism (denoted by dark gray box and labeled marker). FIG. 8B depicts an examplary design of the selection and containment mechanism derived from the *raf* operon that is controlled by raffinose.

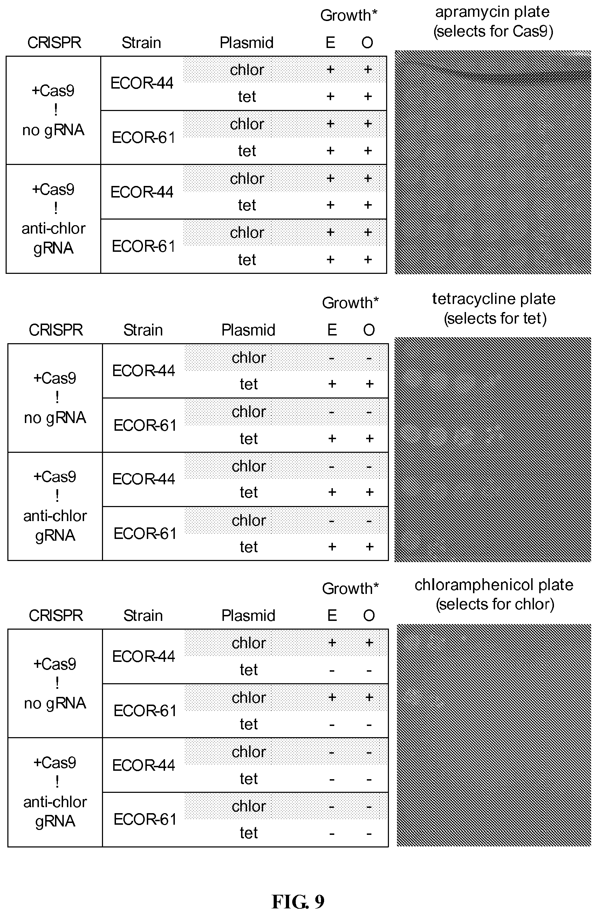

FIG. 9 depicts the performance of the device when deployed in commensal strains of *E. coli,* specifically selected strains from the *E. coli* Collection of Reference (ECOR). These strains have not undergone extensive laboratory evolution, and are therefore closely related to the *E. coli* found in the healthy human gut. FIG. 9 demonstrates that the device prevents uptake of CAT plasmids in this context. ^{∗}Growth is characterized in two columns: the "E" column shows the expected growth phenotype based on previous experiments with laboratory strains of *Escherichia coli,* while the "O" column shows the observed growth phenotype in this experiment with commensal strains of *Escherichia coli.* In both columns, the - symbol denotes no growth under the illustrated condition, while the + symbol denotes normal growth. Photos to the right of these columns show the actual growth phenotype of these strains. From these data it is evident that the CRISPR/Cas system behaves similarly in both laboratory and commensal strains of *Escherichia coli.*

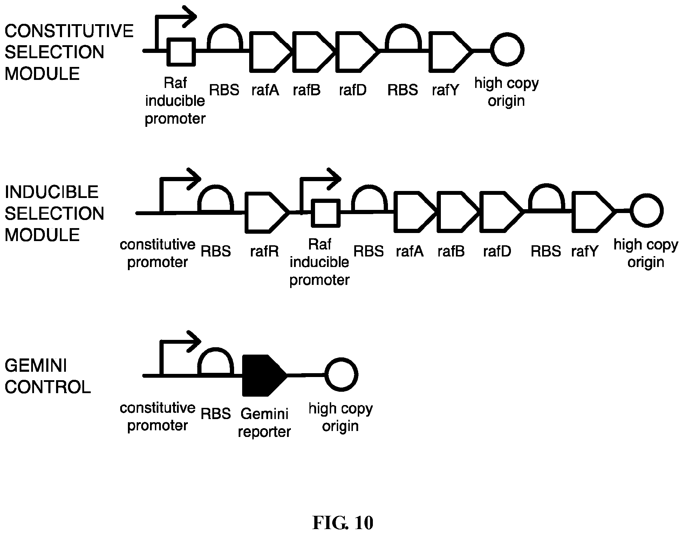

FIG. 10 depicts additional design schemes for selection mechanisms based on the *raf* operon. Also depicted is a control design that expresses the fluorescent Gemini reporter instead of the *raf* operon. For simplicity, the ribosome binding sites controlling the expression of rafB and rafD are not shown in this figure.

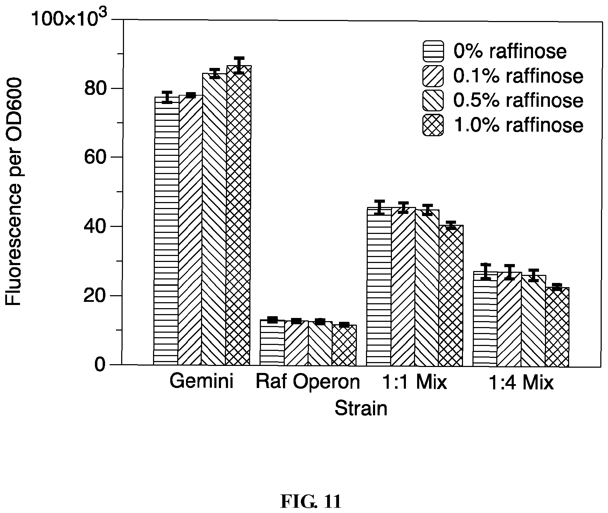

FIG. 11 illustrates the performance of the constitutive raf operon as a selection module. The Gemini column represents *Escherichia coli* cultures containing the Gemini control plasmid shown in FIG. 10. The Raf Operon column represents *Escherichia coli* cultures containig the constitutive Raf selection plasmid shown in FIG. 10. The 1:1 Mix column represents an *Escherichia coli* culture inoculated with equal amounts of the Gemini and Raf Operon *Escherichia coli* strains. The 1:4 Mix column represents an *Escherichia coli* culture inoculated at a ratio of 1 unit of Gemini *Escherichia coli* to every 4 units of Raf Operon *Escherichia coli.* All cultures shown were inoculated with the same sum total amount of *Escherichia coli* and grown in Terrific Broth overnight prior to measurement. Shading of each column illustrates the amount of raffinose applied to each culture. Lower fluorescence indicates that the Raf Operon strain is outcompeting the Gemini strain. The Raf operon provides a significant growth advantage over the Gemini strain at a raffinose concentration of 1.0%. This advantage is potentially larger than suggested by the loss of Gemini fluorescence in the 1:1 and 1:4 mixed samples, as the Gemini strain appears to grow to slightly lower densities in higher concentrations of raffinose, while simultaneously exhibiting a higher fluorescence signal at higher concentrations of raffinose.

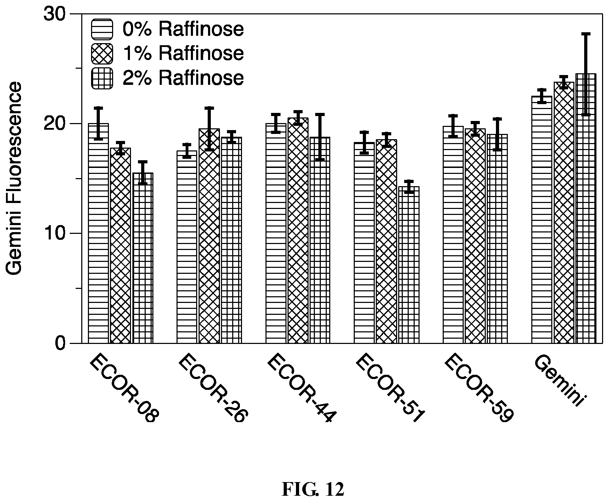

FIG. 12 shows the performance of the constitutive Raf operon in commensal *Escherichia coli* strains from the ECOR collection. The assay was set up as in FIG. 11, with the ECOR strain in each column mixed at a 1:1 ratio with the laboratory *Escherichia coli* strain hosting the Gemini control plasmid. Both ECOR-08 and ECOR-51 display a clear growth advantage in the presence of raffinose.

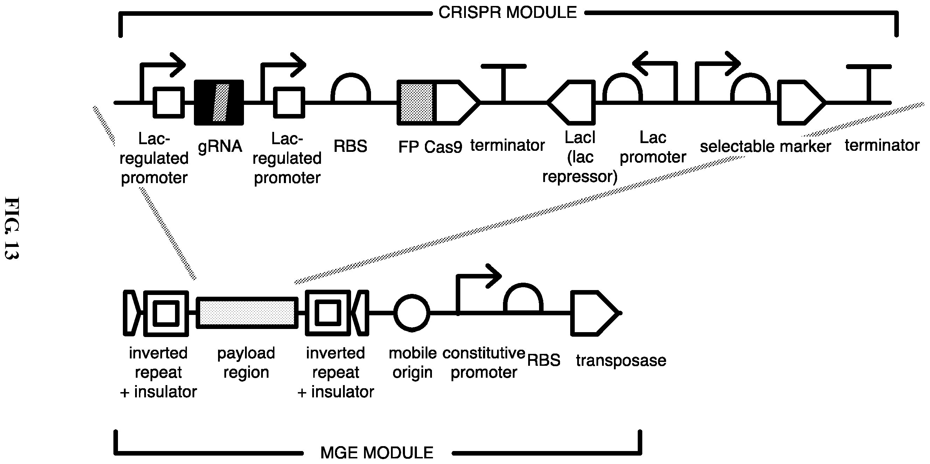

FIG. 13 depicts an updated single-plasmid design for the device. The CRISPR module has been updated to be regulated by the Lac repressor, to prevent activation of the CRISPR device in the absence of lactose or lactose analogs such as Isopropyl β-D-1-thiogalactopyranoside or 5-bromo-4-chloro-3-indolyl-β-D-galactopyranoside. The Cas9 protein may optionally be fused with a fluorescent protein ("FP" in FIG. 13), such as a deep red fluorescent protein, to enable *in vivo* imaging of Cas9 expression. The CRISPR module is harbored in the payload region of a mobile plasmid (MGE module) capable of conjugation between bacterial cells (SEQ ID NOS:17 and 18); optionally this module includes a constitutive transposase for transfer of the payload region and contained CRISPR module into the genome of bacterial cells (SEQ ID NOS:19 and 20).

### DETAILED DESCRIPTION

The present disclosure relates to methods and systems for developing and using an engineered probiotic as therapeutic treatment for gastrointestinal, skin or urinary tract diseases and/or infections, as agent for combatting the spread of antibiotic resistance, and/or as tool for decontamination of emvironmental pathogens. The invention is as defined in the appended claims.

### Definitions

As used herein, the terms "nucleic acids," "nucleic acid molecule" and "polynucleotide" may be used interchangeably and include both single-stranded (ss) and double-stranded (ds) RNA, DNA and RNA:DNA hybrids. As used herein the terms "nucleic acid", "nucleic acid molecule", "polynucleotide", "oligonucleotide", "oligomer" and "oligo" are used interchangeably and are intended to include, but are not limited to, a polymeric form of nucleotides that may have various lengths, including either deoxyribonucleotides or ribonucleotides, or analogs thereof. For example, oligos may be from 5 to about 200 nucleotides, from 10 to about 100 nucleotides, or from 20 to about 50 nucleotides long. However, shorter or longer oligonucleotides may be used. Oligos for use in the present disclosure can be fully designed. A nucleic acid molecule may encode a full-length polypeptide or a fragment of any length thereof, or may be non-coding.

Nucleic acids can refer to naturally-occurring or synthetic polymeric forms of nucleotides. The oligos and nucleic acid molecules of the present disclosure may be formed from naturally-occurring nucleotides, for example forming deoxyribonucleic acid (DNA) or ribonucleic acid (RNA) molecules. Alternatively, the naturally-occurring oligonucleotides may include structural modifications to alter their properties, such as in peptide nucleic acids (PNA) or in locked nucleic acids (LNA). The terms should be understood to include equivalents, analogs of either RNA or DNA made from nucleotide analogs and as applicable to the embodiment being described, single-stranded or double-stranded polynucleotides. Nucleotides useful in the disclosure include, for example, naturally-occurring nucleotides (for example, ribonucleotides or deoxyribonucleotides), or natural or synthetic modifications of nucleotides, or artificial bases. Modifications can also include phosphorothioated bases for increased stability.

Nucleic acid sequences that are "complementary" are those that are capable of base-pairing according to the standard Watson-Crick complementarity rules. As used herein, the term "complementary sequences" means nucleic acid sequences that are substantially complementary, as may be assessed by the nucleotide comparison methods and algorithms set forth below, or as defined as being capable of hybridizing to the polynucleotides that encode the protein sequences.

As used herein, the term "gene" refers to a nucleic acid that contains information necessary for expression of a polypeptide, protein, or untranslated RNA (e.g., rRNA, tRNA, anti-sense RNA). When the gene encodes a protein, it includes the promoter and the structural gene open reading frame sequence (ORF), as well as other sequences involved in expression of the protein. When the gene encodes an untranslated RNA, it includes the promoter and the nucleic acid that encodes the untranslated RNA.

As used herein, the term "genome" refers to the whole hereditary information of an organism that is encoded in the DNA (or RNA for certain viral species) including both coding and non-coding sequences. The term may include the chromosomal DNA of an organism and/or DNA that is contained in an organelle such as, for example, the mitochondria or chloroplasts and/or extrachromosomal plasmid and/or artificial chromosome. A "native gene" or "endogenous gene" refers to a gene that is native to the host cell with its own regulatory sequences whereas an "exogenous gene" or "heterologous gene" refers to any gene that is not a native gene, comprising regulatory and/or coding sequences that are not native to the host cell. In some embodiments, a heterologous gene may comprise mutated sequences or part of regulatory and/or coding sequences. In some embodiments, the regulatory sequences may be heterologous or homologous to a gene of interest. A heterologous regulatory sequence does not function in nature to regulate the same gene(s) it is regulating in the transformed host cell. "Coding sequence" refers to a DNA sequence coding for a specific amino acid sequence. As used herein, "regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, ribosome binding sites, translation leader sequences, RNA processing site, effector (e.g., activator, repressor) binding sites, stem-loop structures, and so on.

As described herein, a genetic element may be any coding or non-coding nucleic acid sequence. In some embodiments, a genetic element is a nucleic acid that codes for an amino acid, a peptide or a protein. Genetic elements may be operons, genes, gene fragments, promoters, exons, introns, regulatory sequences, or any combination thereof. Genetic elements can be as short as one or a few codons or may be longer including functional components (e.g., encoding proteins) and/or regulatory components. In some embodiments, a genetic element includes an entire open reading frame of a protein, or the entire open reading frame and one or more (or all) regulatory sequences associated therewith. One skilled in the art would appreciate that the genetic elements can be viewed as modular genetic elements or genetic modules. For example, a genetic module can comprise a regulatory sequence or a promoter or a coding sequence or any combination thereof. In some embodiments, the genetic element includes at least two different genetic modules and at least two recombination sites. In eukaryotes, the genetic element can comprise at least three modules. For example, a genetic module can be a regulator sequence or a promoter, a coding sequence, and a polyadenlylation tail or any combination thereof. In addition to the promoter and the coding sequences, the nucleic acid sequence may comprises control modules including, but not limited to a leader, a signal sequence and a transcription terminator. The leader sequence is a non-translated region operably linked to the 5' terminus of the coding nucleic acid sequence. The signal peptide sequence codes for an amino acid sequence linked to the amino terminus of the polypeptide which directs the polypeptide into the cell's secretion pathway.

As generally understood, a codon is a series of three nucleotides (triplets) that encodes a specific amino acid residue in a polypeptide chain or for the termination of translation (stop codons). There are 64 different codons (61 codons encoding for amino acids plus 3 stop codons) but only 20 different translated amino acids. The overabundance in the number of codons allows many amino acids to be encoded by more than one codon. Different organisms (and organelles) often show particular preferences or biases for one of the several codons that encode the same amino acid. The relative frequency of codon usage thus varies depending on the organism and organelle. In some instances, when expressing a heterologous gene in a host organism, it is desirable to modify the gene sequence so as to adapt to the codons used and codon usage frequency in the host. In particular, for reliable expression of heterologous genes it may be preferred to use codons that correlate with the host's tRNA level, especially the tRNA's that remain charged during starvation. In addition, codons having rare cognate tRNA's may affect protein folding and translation rate, and thus, may also be used. Genes designed in accordance with codon usage bias and relative tRNA abundance of the host are often referred to as being "optimized" for codon usage, which has been shown to increase expression level. Optimal codons also help to achieve faster translation rates and high accuracy. In general, codon optimization involves silent mutations that do not result in a change to the amino acid sequence of a protein.

Genetic elements or genetic modules may derive from the genome of natural organisms or from synthetic polynucleotides or from a combination thereof. In some embodiments, the genetic elements modules derive from different organisms. Genetic elements or modules useful for the methods described herein may be obtained from a variety of sources such as, for example, DNA libraries, BAC (bacterial artificial chromosome) libraries, *de novo* chemical synthesis, commercial gene synthesis or excision and modification of a genomic segment. The sequences obtained from such sources may then be modified using standard molecular biology and/or recombinant DNA technology to produce polynucleotide constructs having desired modifications for reintroduction into, or construction of, a large product nucleic acid, including a modified, partially synthetic or fully synthetic genome. Exemplary methods for modification of polynucleotide sequences obtained from a genome or library include, for example, site directed mutagenesis; PCR mutagenesis; inserting, deleting or swapping portions of a sequence using restriction enzymes optionally in combination with ligation; *in vitro* or *in vivo* homologous recombination; and site-specific recombination; or various combinations thereof. In other embodiments, the genetic sequences useful in accordance with the methods described herein may be synthetic oligonucleotides or polynucleotides. Synthetic oligonucleotides or polynucleotides may be produced using a variety of methods known in the art.

In some embodiments, genetic elements share less than 99%, less than 95%, less than 90%, less than 80%, less than 70% sequence identity with a native or natural nucleic acid sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Expression as a percentage of homology, similarity, or identity refers to a function of the number of identical or similar amino acids at positions shared by the compared sequences. Various alignment algorithms and/or programs may be used, including FASTA, BLAST, or ENTREZ FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences. Other techniques for alignment are described [Doolittle, 1996]. Preferably, an alignment program that permits gaps in the sequence is utilized to align the sequences. The Smith-Waterman is one type of algorithm that permits gaps in sequence alignments [Shpaer, 1997]. Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer.

As used herein, the phrase "homologous recombination" refers to the process in which nucleic acid molecules with similar nucleotide sequences associate and exchange nucleotide strands. A nucleotide sequence of a first nucleic acid molecule that is effective for engaging in homologous recombination at a predefined position of a second nucleic acid molecule can therefore have a nucleotide sequence that facilitates the exchange of nucleotide strands between the first nucleic acid molecule and a defined position of the second nucleic acid molecule. Thus, the first nucleic acid can generally have a nucleotide sequence that is sufficiently complementary to a portion of the second nucleic acid molecule to promote nucleotide base pairing. Homologous recombination requires homologous sequences in the two recombining partner nucleic acids but does not require any specific sequences. Homologous recombination can be used to introduce a heterologous nucleic acid and/or mutations into the host genome. Such systems typically rely on sequence flanking the heterologous nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

It should be appreciated that the nucleic acid sequence of interest or the gene of interest may be derived from the genome of natural organisms. In some embodiments, genes of interest may be excised from the genome of a natural organism or from the host genome, for example *E. coli.* It has been shown that it is possible to excise large genomic fragments by *in vitro* enzymatic excision and *in vivo* excision and amplification. For example, the FLP/FRT site specific recombination system and the Cre/loxP site specific recombination systems have been efficiently used for excision large genomic fragments for the purpose of sequencing [Yoon, 1998]. In some embodiments, excision and amplification techniques can be used to facilitate artificial genome or chromosome assembly. In some embodiments, the excised genomic fragments can be assembled with engineered promoters and/or other gene expression elements and inserted into the genome of the host cell.

As used herein, the term "polypeptide" refers to a sequence of contiguous amino acids of any length. The terms "peptide," "oligopeptide," "protein" or "enzyme" may be used interchangeably herein with the term "polypeptide". In certain instances, "enzyme" refers to a protein having catalytic activities.

As used herein, unless otherwise stated, the term "transcription" refers to the synthesis of RNA from a DNA template; the term "translation" refers to the synthesis of a polypeptide from an mRNA template. Translation in general is regulated by the sequence and structure of the 5' untranslated region (5'-UTR) of the mRNA transcript. One regulatory sequence is the ribosome binding site (RBS), which promotes efficient and accurate translation of mRNA. The prokaryotic RBS is the Shine-Dalgarno sequence, a purine-rich sequence of 5'-UTR that is complementary to the UCCU core sequence of the 3'-end of 16S rRNA (located within the 30S small ribosomal subunit). Various Shine-Dalgarno sequences have been found in prokaryotic mRNAs and generally lie about 10 nucleotides upstream from the AUG start codon. Activity of a RBS can be influenced by the length and nucleotide composition of the spacer separating the RBS and the initiator AUG. In eukaryotes, the Kozak sequence lies within a short 5' untranslated region and directs translation of mRNA. An mRNA lacking the Kozak consensus sequence may also be translated efficiently in an *in vitro* systems if it possesses a moderately long 5'-UTR that lacks stable secondary structure. While *E. coli* ribosome preferentially recognizes the Shine-Dalgarno sequence, eukaryotic ribosomes (such as those found in retic lysate) can efficiently use either the Shine-Dalgarno or the Kozak ribosomal binding sites.

As used herein, the terms "promoter," "promoter element," or "promoter sequence" refer to a DNA sequence which when ligated to a nucleotide sequence of interest is capable of controlling the transcription of the nucleotide sequence of interest into mRNA. A promoter is typically, though not necessarily, located 5' (i.e., upstream) of a nucleotide sequence of interest whose transcription into mRNA it controls, and provides a site for specific binding by RNA polymerase and other transcription factors for initiation of transcription. A promoter may be constitutively active ("constitutive promoter") or be controlled by other factors such as a chemical, heat or light. The activity of an "inducible promoter" is induced by the presence or absence or biotic or abiotic factors. Aspects of the disclosure relate to an "autoinducible" or "autoinduction" system where an inducible promoter is used, but addition of exogenous inducer is not required. Commonly used constitutive promoters include CMV, EF1a, SV40, PGK1, Ubc, human beta actin, CAG, Ac5, Polyhedrin, TEF1, GDS, ADH1 (repressed by ethanol), CaMV35S, Ubi, HI, U6, T7 (requires T7 RNA polymerase), and SP6 (requires SP6 RNA polymerase). Common inducible promoters include TRE (inducible by Tetracycline or its derivatives; repressible by TetR repressor), GAL1 & GAL10 (inducible with galactose; repressible with glucose), lac (constitutive in the absence of lac repressor (LacI); can be induced by IPTG or lactose), T7lac (hybrid of T7 and lac; requires T7 RNA polymerase which is also controlled by lac operator; can be induced by IPTG or lactose), araBAD (inducible by arabinose which binds repressor AraC to switch it to activate transcription; repressed catabolite repression in the presence of glucose via the CAP binding site or by competitive binding of the anti-inducer fucose), trp (repressible by tryptophan upon binding with TrpR repressor), tac (hybrid of lac and trp; regulated like the lac promoter; e.g., tacI and tacII), and pL (temperature regulated). The promoter can be prokaryotic or eukaryotic promoter, depending on the host. Common promoters and their sequences are well known in the art.

One should appreciate that promoters have modular architecture and that the modular architecture may be altered. Bacterial promoters typically include a core promoter element and additional promoter elements. The core promoter refers to the minimal portion of the promoter required to initiate transcription. A core promoter includes a Transcription Start Site, a binding site for RNA polymerases and general transcription factor binding sites. The "transcription start site" refers to the first nucleotide to be transcribed and is designated +1. Nucleotides downstream of the start site are numbered +1, +2, etc., and nucleotides upstream of the start site are numbered -1, -2, etc. Additional promoter elements are located 5' (i.e., typically 30-250 bp upstream of the start site) of the core promoter and regulate the frequency of the transcription. The proximal promoter elements and the distal promoter elements constitute specific transcription factor site. In prokaryotes, a core promoter usually includes two consensus sequences, a -10 sequence or a -35 sequence, which are recognized by sigma factors. The -10 sequence (10 bp upstream from the first transcribed nucleotide) is typically about 6 nucleotides in length and is typically made up of the nucleotides adenosine and thymidine (also known as the Pribnow box). The presence of this box is essential to the start of the transcription. The -35 sequence of a core promoter is typically about 6 nucleotides in length. The nucleotide sequence of the -35 sequence is typically made up of the each of the four nucleosides. The presence of this sequence allows a very high transcription rate. In some embodiments, the -10 and the -35 sequences are spaced by about 17 nucleotides. Eukaryotic promoters are more diverse than prokaryotic promoters and may be located several kilobases upstream of the transcription starting site. Some eukaryotic promoters contain a TATA box, which is located typically within 40 to 120 bases of the transcriptional start site. One or more upstream activation sequences (UAS), which are recognized by specific binding proteins can act as activators of the transcription. Theses UAS sequences are typically found upstream of the transcription initiation site. The distance between the UAS sequences and the TATA box is highly variable and may be up to 1 kb.

As used herein, the term "vector" refers to any genetic element, such as a plasmid, phage, transposon, cosmid, chromosome, artificial chromosome, episome, virus, virion, etc., capable of replication when associated with the proper control elements and which can transfer gene sequences into or between cells. The vector may contain a selection module suitable for use in the identification of transformed or transfected cells. For example, selection modules may provide antibiotic resistant, fluorescent, enzymatic, as well as other traits. As a second example, selection modules may complement auxotrophic deficiencies or supply critical nutrients not in the culture media. Types of vectors include cloning and expression vectors. As used herein, the term "cloning vector" refers to a plasmid or phage DNA or other DNA sequence which is able to replicate autonomously in a host cell and which is characterized by one or a small number of restriction endonuclease recognition sites and/or sites for site-specific recombination. A foreign DNA fragment may be spliced into the vector at these sites in order to bring about the replication and cloning of the fragment. The term "expression vector" refers to a vector which is capable of expressing of a gene that has been cloned into it. Such expression can occur after transformation into a host cell, or in IVPS systems. The cloned DNA is usually operably linked to one or more regulatory sequences, such as promoters, activator/repressor binding sites, terminators, enhancers and the like. The promoter sequences can be constitutive, inducible and/or repressible.

As used herein, the term "host" or "host cell" refers to any prokaryotic or eukaryotic single cell (e.g., yeast, bacterial, archaeal, etc.) cell or organism. The host cell can be a recipient of a replicable expression vector, cloning vector or any heterologous nucleic acid molecule. Host cells may be prokaryotic cells such as species of the genus *Escherichia* or *Lactobacillus,* or eukaryotic single cell organism such as yeast. The heterologous nucleic acid molecule may contain, but is not limited to, a sequence of interest, a transcriptional regulatory sequence (such as a promoter, enhancer, repressor, and the like) and/or an origin of replication. As used herein, the terms "host," "host cell," "recombinant host" and "recombinant host cell" may be used interchangeably. For examples of such hosts, see [Sambrook, 2001].

One or more nucleic acid sequences can be targeted for delivery to target prokaryotic or eukaryotic cells via conventional transformation techniques. As used herein, the term "transformation" is intended to refer to a variety of art-recognized techniques for introducing an exogenous nucleic acid sequence (e.g., DNA) into a target cell, including calcium phosphate or calcium chloride co-precipitation, conjugation, electroporation, sonoporation, optoporation, injection and the like. Suitable transformation media include, but are not limited to, water, CaCl₂, cationic polymers, lipids, and the like. Suitable materials and methods for transforming target cells can be found in [Sambrook, 2001], and other laboratory manuals.

As used herein, the term "selection module" or "reporter" refers to a gene, operon, or protein that can be attached to a regulatory sequence of another gene or protein of interest, so that upon expression in a host cell or organism, the reporter can confer certain characteristics that can be relatively easily selected, identified and/or measured. Reporter genes are often used as an indication of whether a certain gene has been introduced into or expressed in the host cell or organism. Examples of commonly used reporters include: antibiotic resistance genes, fluorescent proteins, auxotropic selection modules, β-galactosidase (encoded by the bacterial gene *lacZ),* luciferase (from lightning bugs), chloramphenicol acetyltransferase (CAT; from bacteria), GUS (β-glucuronidase; commonly used in plants) and green fluorescent protein (GFP; from jelly fish). Reporters or selection moduless can be selectable or screenable. A selection module (e.g., antibiotic resistance gene, auxotropic gene) is a gene confers a trait suitable for artificial selection; typically host cells expressing the selectable selection module is protected from a selective agent that is toxic or inhibitory to cell growth. A screenable selection module (e.g., *gfp, lacZ)* generally allows researchers to distinguish between wanted cells (expressing the selection module) and unwanted cells (not expressing the selection module or expressing at insufficient level).

The term "virulence factor", "toxin", "effector" or "pathogenic component" as used herein, refers to molecules that enable otherwise commensal organisms to cause disease or otherwise disrupt a microbial community. The removal of these factors or genetic elements encoding them (including both DNA and RNA) from a commensal bacterial geneome, or the loss of a plasmid or other mobile genetic element encoding them from a commensal bacterial genome is understood to restore the host bacteria to a non-pathogenic state. These factors include but are not limited to any molecule that enables a pathogen to colonize a niche in the host, evade the host's immune system, inhibit the host's immune response, damage the host, enter or exit out of cells, or obtain nutrition from the host. For example, one type of such factor is colonization factors that help the establishment of the pathogen at the appropriate portal of entry. Pathogens usually colonize host tissues that are in contact with the external environment. Sites of entry in human hosts include the urogenital tract, the digestive tract, the respiratory tract and the conjunctiva. Organisms that infect these regions have usually developed tissue adherence mechanisms and some ability to overcome or withstand the constant pressure of the host defenses at the surface, and factors involved therewith have been identified as colonization factors.

The term "pathogenic element", as used herein, refers to genetic elements (including both DNA and RNA) that enable otherwise commensal organisms to cause disease or otherwise disrupt a microbial community. The removal of pathogenic elements from a commensal bacterial geneome, or the loss of a plasmid or other mobile genetic element propagating pathogenic elements from a commensal bacterial genome is understood to restore the host bacteria to a non-pathogenic state. Pathogenic elements include but are not limited to pathogenicity islands. Pathogenic elements (including both DNA and RNA) may encode virulence factors, toxins, effectors or pathogenic components.

The term "mobile genetic element" or "MGE" refers to genetic elements that encode enzymes and other proteins (e.g., transposase) that mediate the movement of DNA within genomes (intracellular mobility) or between cells (intercellular mobility). Examples include transposons, plasmids, bacteriophage, and pathogenicity islands. The MGE can be naturally occurring or engineered. The MGE can be cell-type specific, tissue specific, organism specific, or species specific (e.g., bacteria specific or human specific). The MGE can also be non-specific with respect to cell-type, tissue, organism and/or species.

The term "engineer," "engineering" or "engineered," as used herein, refers to genetic manipulation or modification of biomolecules such as DNA, RNA and/or protein, or like technique commonly known in the biotechnology art.

The locus tags and accession numbers provided throughout this description are derived from the NCBI database (National Ceter for Biotechnology Information) maintained by the National Institute of Health, USA. The accession numbers are provided in the database on January 16, 2014.

Other terms used in the fields of recombinant nucleic acid technology, microbiology, metabolic engineering, and molecular and cell biology as used herein will be generally understood by one of ordinary skill in the applicable arts.

### Target genes for degradation

In one aspect, the present disclosure provides for genes of interest that constitute preferred targets for degradation by the engineered probiotic comprising the engineered genetic system according to the claims. Many microbial species have strains that exist as commensals as part of the natural, healthy microbial flora as well as pathogenic and/or virulent strains capable of causing disease. For example, *Escherichia coli* exists in the human gut as a commensal organism but pathogenic strains are also known. Major categories of *E. coli* pathogens include enteropathogenic *E. coli* (EPEC), enterohemorrhagic *E. coli* (EHEC), enterotoxigenic *E. coli* (ETEC), enteroaggregative *E. coli* (EAEC), enteroinvasive *E. coli* (EIEC), diffusely adherent *E. coli* (DAEC), enteroaggregative *E. coli* ST (EAST) [Kaper, 2004]. Other categories of *E. coli* pathogens are known to be extraintestinal (ExPEC) including uropathogenic *E. coli* (UPEC) and meningitis-associated *E. coli* (MNEC). Despite these varied mechanisms of pathogenesis, each of these diseases are caused by strains that are largely similar to commensal *E. coli;* these strains are differentiated by a small number of specific virulence attributes responsible for each disease. Genetic elements that encode these virulence attributes are frequently found on mobilizable elements that can be readily transferred into new strains to create new virulence factor combinations. Thus, it is these genetic elements themselves, rather than a particular strain or species, that is the basic unit of selection and evolution in a microbial population. In some embodiments, genes that encode virulence attributes are gene targets for the engineered probiotic of this disclosure. Exemplary virulence factors (including both colonization and fitness factors), toxins and effectors are set forth below in Table 1. Note that many of the listed factors and toxins have multiple variants and/or types. A similar set of genes encoding virulence attributes may be compiled for other microbial species that include pathogenic strains.

**Table 1: Virulence factors, toxins and effectors in pathogenic E. coli strains**

| ***Factor*** | ***Strain category*** | ***Activity*/*effect*** |
|---|---|---|
| IcsA (VirG) | EIEC | Nucleation of actin filaments |
| Intimin | EPEC, EHEC | Adhesin, induces T_{H}1 response |
| Dr adhesins | DAEC, UPEC | Adhesin, binds to decay-accelerating factor (DAF), activates phosphatidylinositol 3-kinase (PI-3), induces MHC class I chain-related gene A (MICA) |
| P (Pap) fimbriae | UPEC | Adhesin; induces cytokine expression |
| CFAs, CS, or PCF | ETEC | Adhesin; colonization factor antigens, coli surface antigens, or putative colonization factors |
| Type-1 fimbrae | All | UPEC adhesion; binds to uroplakin |
| F1C fimbriae | UPEC | Adhesin |
| S fimbriae | UPEC, MNEC | Adhesin |
| Bundle-forming pilius (BFP) | EPEC | Type IV pilus |
| Aggregative adherence fimbriae | EAEC | Adhesin |
| Paa | EPEC, EHEC | Adhesin |
| ToxB | EHEC | Adhesin |
| Efa-1/LifA | EHEC | Adhesin |
| Long polar fimbriae (LPF) | EHEC, EPEC | Adhesin |
| Saa | EHEC | Adhesin |
| OmpA | MNEC, EHEC | Adhesin |
| Curli | Various | Adhesin; binds to fibronectin |
| IbeA, B, C | MNEC | Promotes invasion |
| AsIA | MNEC | Promotes invasion |
| Dispersin | EAEC | Promotes colonization; aids mucous penetration |
| K antigen capsules | MNEC | Antiphagocytic |
| Aerobactin | EIEC | Iron acquisition; siderophore |
| Yersiniabactin | Various | Iron acquisition; siderophore |
| IreA | UPEC | Iron acquisition; siderophore receptor |
| IroN | UPEC | Iron acquisition; siderophore receptor |
| | EIEC, | |
| Chu (Shu) | UPEC, MNEC | Iron acquisition; haem transport |
| Flagellin | All | Motility; induces cytokine expression through toll-like receptor TLR5 |
| Lipopolysaccharide | All | Induces cytokine expression through TLR4 |
| Heat-labile enterotoxin (LT) | ETEC | ADP ribosylates and activates adenylate cyclase resulting in ion secretion |
| Shiga toxin (Stx) | EHEC | Depurinates rRNA, inhibiting protein synthesis; induces apoptosis |
| Cytolethal distending toxin (CDT) | Various | DNaseI activity, blocks mitosis in G2/M phase |
| Shigella enterotoxin 1 | EAEC, | Ion secretion |
| (ShET1) | EIEC? | |
| Urease | EHEC | Cleaves urea to ammonia and carbon dioxide |
| EspC | EPEC | Serine protease; ion secretion |
| EspP | EHEC | Serine protease; cleaves coagulation factor V |
| Haemoglobin -binding protease (Tsh) | ExPEC, APEC | Degrades haemoglobin to release haem/iron |
| Pet | EAEC | Serine protease; ion secretion; cytotoxicity |
| | UPEC, | |
| Pic | EAEC, | Protease, mucinase |
| | EIEC? | |
| Sat | UPEC | Vacuolation |
| SepA | EIEC? | Serine protease |
| SigA | EIEC? | Ion secretion |
| Cycle-inhibiting factor (Cif) | EPEC, EHEC | Blocks mitosis in G2/M phase; results in inactivation of Cdkl |
| EspF | EPEC, EHEC | Opens tight junctions, induces apoptosis |
| EspH | EPEC, EHEC | Modulates filopodia and pedestal formation |
| Map | EPEC, EHEC | Stimulates Cdc42-dependent filopodia formation; disrupts mitochondrial membrane potential |
| Tir | EPEC, EHEC | Nucleation of cytoskeletal proteins, loss of microvilli, GTPase-activating protein (GAP)-like activity |
| IpaA | EIEC | Actin depolymerization |
| IpaB | EIEC | Apoptosis, interleukin-1 (IL-1) release; membrane insertion |
| IpaC | EIEC | Actin polymerization, activation of Cdc42 and Rac |
| IpaH | EIEC | Modulates inflammation (?) |
| IpgD | EIEC | Inositol 4-phophatase, membrane blebbing |
| VirA | EIEC | Microtubule destabilization, membrane ruffling |
| StcE | EHEC | Cleaves C1-esterase inhibitor (C1-INH), disrupts complement cascade |
| HlyA | UPEC | Cell lysis |
| Ehx | EHEC | Cell lysis |
| Cytotoxic necrotizing factors (CNF1,CNF2) | MNEC, UPEC, NTEC | Altered cytoskeleton, multinucleation with cell enlargement, necrosis |
| | EPEC, | |
| LifA/Efa | EHEC | Inhibits lymphocyte activation, adhesion |
| Shigella enterotoxin 2 (ShET2) | EIEC, ETEC | Ion secretion |
| Heat-stable enterotoxin a (STa) | ETEC | Activates guanylate cyclase resulting in ion secretion |
| Heat-stable enterotoxin b (STb) | ETEC | Increase intracellular calcium resulting in ion secretion |
| EAST | Various | Activates guanylate cyclase resulting in ion secretion |

Exemplary target genes for degradation asssociated with skin disease and infection include genes encoding toxic shock syndrome toxin-1 (TSST-1, Accession J02615) and staphylococcal superantigen-like protein 11 (SSL11, Accession CP001996 470022..470615). Other virulence factors include staphylococcal enterotoxins such as enterotoxin type G2 *(seg2),* enterotoxin K *(sek),* enterotoxin H *(seh),* enterotoxin type C4 *(sec4),* enterotoxin L *(sel),* and enterotoxin A *(sea);* virulence genes encoded by open reading frames SAV0811, SAV1159, SAV1208, SAV1481, SAV2371, SAV2569, SAV2638, SAV0665, SAV0149, SAV0164, SAV0815, SAV1324, SAV1811, SAV1813, SAV1046, SAV0320, SAV2035, SAV0919, SAV2170, SAV1948, SAV2008, SAV1819, SAV0422, SAV0423, SAV0424, SAV0428, SAV2039, SAV1884, SAV0661 from *Staphylococcus aureus* subsp. Mu50 (Accession BA000017.4). Antibiotic resistance genes common to skin infections include the methicillin resistance gene PBP gene for beta-lactam-inducible penicillin-binding protein *(mecA,* Accession Y00688). Homologs of the listed genes offer additional target genes for degradation.

In many cases, the mechanism of antibiotic resistance is encoded by either a single or small number of genes. Similar to genes encoding virulence attributes, genetic elements that encode antibiotic resistance trait often spread through a mixed species microbial population through horizontal gene transfer. Many genes that confer clinically-relevant antibiotic resistance phenotypes to their host cell have been identified previously. In some embodiments, antibiotic resistance genes constitute gene targets for the engineered probiotic of this disclosure. Exemplary antibiotic resistance genes are set forth below in Table 2.

**Table 2: Genes encoding antibiotic resistance**

| ***Antibiotic*** | ***Resistance Gene(s)*** | ***Species*** | ***Example Locus tag* / *Accession number*** |
|---|---|---|---|
| aminoglycosides | *aadA2* | *Escherichia coli* | pUMNK88_133 |
| aminoglycosides | *aadA* | *E. coli, Yersinia pestis,* | pAR060302_132 |
| | | *Salmonella enterica* | |
| aminoglycosides | *aacC* | *E. coli, S. enterica* | pAR060302_133 |
| aminoglycosides | *aacA1* | *Cornyebacterium resistens* | pJA144188_p28 |
| aminoglycosides | *aphA* | *Y. pestis* | YpIP275_pIP1202_0052 |
| aminoglycosides | *strAB* | *E. coli, Yersinia ruckeri, Y.* | pAR060302_44 and |
| | | *pestis, S. enterica* | pAR060302_43 |
| beta-lactams | *pbp1A* | *C. resistens* | CP002857.1 |
| | | | (2543105..2545177) |
| beta-lactams | *pbp1B* | C. *resistens* | CP002857.1 |
| | | | (2410761..2413031) |
| beta-lactams | *pbp2A* | C. *resistens* | CP002857.1 (48881..50284) |
| beta-lactams | *pbp2B* | C. *resistens* | CP002857.1 |
| | | | (311405..313186) |
| beta-lactams | *pbp2C* | C. *resistens* | CP002857.1 |
| | | | (1512744..1514591) |
| beta-lactams | *dac* | C. *resistens* | CP002857.1 |
| | | | (217296..218633) |
| beta-lactams | *bla*_{CMY-2} | *E. coli, S. enterica* | pAR060302_81 |
| chloramphenicol/florfenicol | *floR* | *E. coli, S. enterica* | pAR060302_39 |
| chloramphenicol | *cmlA* | *E. coli* | pUMNK88_132 |
| chloramphenicol | *cat* | *Enterococcus faecalis, Y.* | YpIP275_pIP1202_0063 |
| | | *pestis* | |
| chloramphenicol | *cmx* | *C. resistens* | pJA144188_p21 |
| erythromycin | *ermA* | *E. faecalis* | AF507977.1 (12938..13675) |
| fluoroquinolones | *gyrA* | *C. resistens* | CP002857.1 (8935..11376) |
| macrolide | *mph2* and *mel* | *E. coli* | pPG010208_34 and pPG010208_35 |
| macrolides and lincosamides | *erm(x)* | *C. resistens* | pJA144188_p06 |
| methicillin | *mecA* | *Staphylococcus aureus* | Y00688 or KC243783.1 |
| streptomycin and | *aadA1a* | *C. resistens* | pJA144188_p26 |
| spectinomycin | | | |
| sulfonamides | *sul1* | *E. coli, Y. pestis, S. enterica* | pAR060302_139 |
| sulfonamides | *sul2* | *E. coli, Y. ruckeri, Y. pestis,* | pAR060302_46 |
| | | *S. enterica* | |
| tetracycline | *tetA* | *E. coli, Y. ruckeri, Y. pestis,* | pAR060302_41 |
| | | *S. enterica* | |
| tetracyclines | *tet(W)* | *C. resistens* | pJA144188_p07 |
| | *bla*_{SHV-1} | *Y. pestis* | YpIP275_pIP1202_0175 |
| trimethoprim | *dhfr* | *Y. ruckeri* | YR71pYR1_0114 |
| vancomycin/teicoplanin | *van(A)* | *Enterococcus faecium* | AM296544.1 (8898..15523) |
| vancomycin | *van(B)* | *E. faecalis* | AB374546.1 (32627..39057) |
| all antibiotic options in | *bla*_{NDM-1} | *E. coli, Acinetobacter* | HQ857107.1 (2193..3005) |
| humans | | *baumanii, Klebsiella* | |
| | | *pneumoniae* | |

In other, non-claimed examples, other undesirable and/or malicious genes and/or genetic elements can also be targeted. For example, in a disease caused by a genetic abnormality such as cancer, such genetic abnormality can be targeted for degradation. As a result, gene therapy can be achieved. The targeting can be cell-type specific or tissue specific (e.g., by using cell-type specific or tissue specific MGE), so as to limit to gene degradation a desired cell type or tissue.

### Targeting and degradation of undesirable genes

In some embodiments, targeting and degradation of undesirable genes can be mediated by CRISPR-an acronym for clustered, regularly interspaced short palindromic repeats. CRISPRs were first described in 1987 [Ishino, 1987]. CRISPRs play a functional role in phage defense in prokaryotes [Barrangou, 2007; Horvath, 2008; Deveau, 2008]. Briefly, CRISPRs work as follows. When exposed to a phage infection or invasive genetic element, some members of the bacterial population incorporate short sequences from the foreign DNA ("spacers") between repeated sequences within the CRISPR locus. The combined unit of repeats and spacers in tandem is referred to as the "CRISPR array." The CRISPR array is transcribed and then processed into short crRNAs (CRISPR RNAs) each containing a single spacer and flanking repeated sequences. Spacers are derived from foreign DNA (which contains corresponding protospacers that can base pair with the spacers) and are generally stably inherited by daughter cells such that when later exposed to a phage or invasive DNA element with the same sequence, the strain is resistant to infection. CRISPRs are known to operate in conjunction with cognate Cas (CRISPR associated) protein(s) that show specificity to the repeat sequences separating the spacers [Heidelberg, 2009; Horvath, 2009; Kunin, 2007]. The Cas protein(s) operate in conjunction with the crRNA to mediate the cleavage of incoming foreign DNA where the crRNA forms an effector complex with the Cas proteins and guides the complex to the foreign DNA, which is then cleaved by the Cas proteins [Bhaya, 2011]. There are several pathways of CRISPR activation, one of which requires a tracrRNA (trans-activating crRNA, also transcribed from the CRISPR array) which plays a role in the maturation of crRNA. Then a crRNA/tracrRNA hybrid forms and acts as a guide for the Cas9 to the foreign DNA [Deltcheva, 2011]. There are also other pathways that do not require tracrRNA.

Synthetic biologists have recently demonstrated that CRISPR-Cas nucleases and associated RNAs can be repurposed to edit the genomes in bacteria, yeast and human cells [DiCarlo, 2013; Jiang, 2013; Cong, 2013; Mali 2013; Jinek, 2013]. These techniques all rely on the use of a Cas9 nuclease to introduce double strand breaks at specific loci. Since the binding specificity of Cas9 is defined by a separate RNA molecule, crRNA, Cas9 can be directed to recognize and cleave nearly all 20-30 base pair sequences. The short sequence requirements for CRISPR targeting allow Cas9 to be re-targeted simply by inserting oligonucleotides of interest into the cognate CRISPR constructs.

In some aspects, the present disclosure provides for a probiotic engineered to include a heterologous, genetic system designed to target gene(s) of interest for degradation. In some embodiments, the heterologous genetic system encodes a synthetic CRISPR-Cas device designed to target a Cas nuclease to one or more gene(s) of interest. The heterologous genetic system comprises a gene encoding a Cas nuclease, a CRISPR array containing one or more spacers derived from the target DNA flanked by CRISPR direct repeats that is transcribed and processed into one or more crRNAs, and optionally, a tracrRNA that forms a complex with the Cas protein and the crRNA. By targeting a Cas nuclease to sequence(s) within target gene(s) of interest (protospacers), the gene(s) of interest may be targeted for cleavage and therefore subsequent degradation thereof in the cell.

Viable target sequences for CRISPR/Cas systems are determined based on the specific Cas nuclease chosen; the sequence of interest (protospacer) must be immediately adjacent to a "Protospacer Associated Motif' (PAM) [Jinek, 2012]. In some embodiments, the *Streptococcus pyogenes* Cas9 nuclease may be used [Jiang, 2013; Cong, 2013; Mali, 2013; Jinek, 2013; Jinek, 2012]. *S. pyogenes* Cas9 requires the PAM "NGG" to be 3' of the sequence of interest, where "N" can be any nucleotide. The "NGG" motif is very common in nucleic acid sequences and thus allows us to select essentially any gene of interest as a target for the engineered probiotic.

CRISPR arrays are highly repetitive due to the requirement for direct repeat sequences adjacent to spacer sequence(s). As such, CRISPR arrays can be unstable due to possible recombination events [Jiang, 2013]. To obviate this problem, it has been shown that the tracrRNA and crRNA may be combined into a single RNA sequence ("guide RNA") that mimics the processed crRNA-tracrRNA complex. Guide RNA based designs have been demonstrated to be effective when employed for genome editing in a variety of hosts [DiCarlo, 2013; Cong, 2013; Mali, 2013]. Thus, in some embodiments, the CRISPR/Cas system of the engineered probiotic includes one or more synthetic guide RNA(s) designed to target the gene(s) of interest for degradation.

In addition to CRISPR/Cas systems, Transcription Activator-Like Effector Nucleases, Zinc Finger Nucleases, or Meganucleases may be used to target genes of interest for degradation. Transcription Activator-Like Effector Nucleases (TALENs) are modular protein nucleases that can be designed to bind and cut specific DNA sequences [Cermak, 2011; Ting, 2011]. Exemplary TALENs are reviewed in [Joung, 2012]. Similarly, Zinc Finger Nucleases (ZFNs) are another class of modular protein nucleases that can be designed to bind and cut specific DNA sequences [Wright, 2006]. Exemplary ZFNs are reviewed in [Urnov, 2010]. Meganucleases can also be used and designed to bind and cut specific DNA sequences. Exemplary meganucleases are reviewed in [Silva, 2011].

In some embodiments, the CRISPR/Cas system may be designed to target RNA molecules. The guide RNA(s) may be designed to target single stranded RNA that is analogous to the guide RNAs designed to target DNA; however, the PAM is provided in *trans* as a DNA oligonucleotide [O'Connell, 2014]. The DNA oligonucleotide hybridizes to the single stranded RNA target sequence and comprises the non-target strand of the RNA:DNA heteroduplex. The RNA target sequence needs not include the PAM sequence as long as the DNA oligonucleotide provides the PAM sequence to facilitate cleavage. Indeed, the absence of the PAM sequence in the single stranded RNA precludes the CRISPR/Cas system from targeting the encoding DNA sequence.

In various embodiments, TALENs or ZFNs or meganucleases may be substituted for CRISPR/Cas nucleases in an engineered probiotic, provided the TALEN or ZFN or meganuclease is designed to target a specific DNA sequence for degradation. As is generally understood to those skilled in the art, TALENs and ZFNs consist of modular protein domains, each domain conferring binding specificity to a specific DNA base pair. Indivdual modular TALEN domains can target "A," "T," "C," or "G" nucleotides. Thus engineered TALENs comprising a fusion protein of modular TALEN domains can be designed to target an arbitrary and specific base pair sequence [Cermak, 2011]. Likewise, individual modular domains of ZFNs target a variety of 3 base pair sequences. Engineered ZFNs are fusion proteins, typically composed of 3 ZFN modules that target a specific 9 base pair sequence [Maeder, 2008]. Meganucleases target DNA sequences of 10 or more base pairs in length; if this recognition sequence exists in the gene of interest and doesn't exist elsewhere in the genomes of the targeted cellular community, then meganucleases may be substituted for CRISPR/Cas nucleases [Silva, 2011].

### Engineered horizontal gene transfer

Horizontal gene transfer is a major mechanism of transfer of virulence attributes and antibiotic resistance phenotypes within microbial populations. For example, metagenomic analysis of human gut flora indicates that horizontal gene transfer is more prevalent in the human microbiome than in external environments [Smillie, 2011]. The high cell density of the human gastrointestinal tract renders it highly conducive to gene transfer [Ley, 2006]. Mobile genetic elements (MGEs)-including transposons, plasmids, bacteriophage, and pathogenicity islands-are responsible for the acquisition of virulence attributes by otherwise commensal microorganisms [Kaper, 2004]. Horizontal gene transfer is primarily accomplished by one of three mechanisms in bacteria. First, transmission of plasmids via conjugation of a donor bacterium to a recipient bacterium. Second, transformation of a cell with free DNA in the form of a plasmid or nucleic acid fragments. Third, transduction as mediated by a bacteriophage.

In some aspects, the present disclosure provides for a probiotic engineered to include a heterologous, genetic system, as defined in the claims, designed to propagate the gene degradation system within a microbial population. The heterologous genetic system comprises a synthetic mobile genetic element (MGE) capable of dispersing the gene degradation system to other microbial strains and species in a microbial population. Thus, the engineered probiotic itself need only persist long enough in the microbial population to remove gene(s) of interest from the population and/or to transfer the MGE to other strains within microbial population. Types of known MGEs include conjugative transposons, conjugative plasmids and bacteriophages. Exemplary mobile genetic elements are set forth below in Table 3.

**Table 3: Mobile genetic elements (MGEs)**

| ***Name*** | ***Type*** | ***Size*** | ***Host range*** |
|---|---|---|---|
| Tn916 | Conjugative transposon | 18.5 kb, 11 genes | Gram-positive bacteria with lower transmissibility in Gram-negative bacteria |
| RK2 | IncP-1 Conjugative plasmid | 60 kb, 56 core genes | Almost all Gram-negative bacteria and some Gram-positive bacteria including Actinobacteria |
| P1 | Bacteriophage | 90 kb, 117 genes | Several Gram-negative bacteria |
| Tn5280 | Class I transposon | 8.5 kb | N/A |
| Tn4651 | Class II transposon | 56 kb | N/A |

Conjugative transposons are compact, self-transmissible mobile elements that combine dispersal and translocative functions on a single DNA fragment [Tsuda, 1999; Salyers, 1995]. Conjugative transposons generally reside on the bacterial genome and can self-excise and transfer to recipient cells via conjugation. Exemplary conjugative transposons include Tn916.

Conjugative plasmids offer an alternative embodiment for the MGE of the present disclosure. In some embodiments, the conjugative plasmid is an IncP-1 plasmid since they are known for both their broad-host range and high efficiency self-transmission [Adamczyk, 2003]. Exemplary IncP-1 plasmids maintained in different hosts include *E. coli* (pRK2013) (a-Proteobacteria), *Ralstonia eutropha* (pSS50) (β-Proteobacteria), and RK2 conjugated into *Pseudonocardia autotrophica* for Gram-positive Actinobacteria. In some embodiments, the MGE is derived from an RK2 compatible conjugative plasmid in which the *pir* and *tra* factors have been moved from the plasmid to the chromosome of the engineered probiotic. Host cells that are *pir*+ and *tra*+ permit transfer of plasmids bearing RK2 *mob* elements to new strains. Since the *pir* and *tra* factors are provided in trans by the host cell, the RK2 plasmid cannot further propagate in recipient strains lacking these factors. Thus, propagation of the RK2 plasmid is limited only to those strains that make direct contact with the engineered probiotic.

Conjugative plasmids may optionally include a transposon that allows a portion of the plasmid to be stably transferred to the genome of the recipient cell. For example, the *tnp* transposase from the Tn5 transposon translocates DNA sequences flanked by IS50 repeat sequences [Phadnis, 1986]. Placement of arbitrary DNA between transposon repeats (referred to as a "payload region" in FIG. 13) between transposon repeats (labeled as "inverted repeat") results in translocation of the payload region to other DNA molecules, including the genome of recipient bacterial cells. A schematic of a typical design including a conjugative plasmid with a transposon is illustrated in FIG. 13. While lac promoter is shown in FIG. 13, one of ordinary skill in the art would understand other inducible promoters can also be used. Exemplary sequences are included as SEQ ID NO:19 and SEQ ID NO:20.

Bacteriophages offer an alternative embodiment for the MGE of the present disclosure. Exemplary bacteriophage includes bacteriophage PI, a temperate phage capable of entering either a lysogenic or lytic state upon infection. Prior published results suggest that PI has a broad host range among the Gram-negatives including *Agrobacterium, Alcaligenes, Citrobacter, Enterobacter, Erwinia, Flavobacterium, Klebsiella, Proteus, Pseudomonas, Salmonella,* and *Serratia* [Murooka, 1979]. Nevertheless, bacteriophages tend to have narrower host ranges than other MGEs like plasmids. Thus, in some embodiments, use of bacteriophage as a transmission vector may necessitate additional engineering of the bacteriophage to broaden its host range. For example, the bacteriophage may be engineered to bypass host restriction-modification systems by eliminating 6 bp palindromic sequences from the MGE and by adding methylase(s) to protect short sites, to expand its replication range by including a broad host range replication origin, and/or to enhance the bacteriophage's ability to penetrate the extracellular matrix by adding display degradative enzymes.

### Organisms or host cells for the engineered probiotic

The host cell or organism, as disclosed herein, may be chosen from eukaryotic or prokaryotic systems capable of surviving, persisting and/or colonizing in the mammalian gastrointestinal system or the mammalian urinary tract, such as bacterial cells (Gram-negative or Gram-positive), archaea and yeast cells. Suitable organisms can include those bacteria belonging to the Bacteroidetes, Firmicutes, Proteobacteria, Actinobacteria, Verrucomicrobia or Fusobacteria divisions (superkingdoms) of Bacteria. In a preferred embodiment, the host cell/organism is culturable in the laboratory. In some embodiments, host cells/organisms can be selected from *Bacteroides* species including *Bacteroides* AFS519, *Bacteroides* sp. CCUG 39913, *Bacteroides* sp. Smarlab 3301186, *Bacteroides ovatus, Bacteroides salyersiae, Bacteroides* sp. MPN isolate group 6, *Bacteroides* DSM 12148, *Bacteroides merdae, Bacteroides distasonis, Bacteroides stercosis, Bacteroides splanchnicus, Bacteroides* WH2, *Bacteroides uniformis, Bacteroides* WH302, *Bacteroides fragilis, Bacteroides caccae, Bacteroides thetaiotamicron, Bacteroides vulgatus,* and *Bacteroides capillosus.* In some embodiments, host cells/organisms can be selected from *Clostridium* species including *Clostridium leptum, Clostridium boltaea, Clostridium bartlettii, Clostridium symbiosum, Clostridium* sp. DSM 6877(FS41), *Clostridium* A2-207, *Clostridium scindens, Clostridium spiroforme, Clostridium* sp. A2-183, *Clostridium* sp. SL6/1/1, *Clostridium* sp. GM2/1, *Clostridium* sp. A2-194, *Clostridium* sp. A2-166, *Clostridium* sp. A2-175, *Clostridium* sp. SR1/1, *Clostridium* sp. L1-83, *Clostridium* sp. L2-6, *Clostridium* sp. A2-231, *Clostridium* sp. A2-165 and *Clostridium* sp. SS2/1. In some embodiments, host cells/organisms can be selected from *Eubacterium* species including *Eubacterium plautii, Eubacterium ventriosum, Eubacterium halii, Eubacterium siraeum, Eubacterium eligens,* and *Eubacterium rectale.* In some embodiments, host cells/organisms can be selected from *Alistipes finegoldii, Alistipes putredinis, Anaerotruncus colihominis, Allisonella histaminiformans, Bulleida moorei, Peptostreptococcus* sp. oral clone CK035, *Anaerococcus vaginalis, Ruminococcus bromii, Anaerofustis stercorihominis, Streptococcus mitis, Ruminococcus callidus, Streptococcus parasanguinis, Coprococcus eutactus, Gemella haemolysans, Peptostreptococcus micros, Ruminococcus gnavus, Coprococcus catus, Roseburia intestinalis, Roseburiafaecalis, Ruminococcus obeum, Catenibacterium mitsuokai, Ruminococcus torques, Subdoligranulum variabile, Dorea formicigenerans, Dialister* sp. E2_20, *Dorea longicatena, Faecalibacterium prausnitzii, Akkermansia muciniphila, Fusobacterium* sp. oral clone R002, *Escherichia coli, Haemophilus parainfluenziae, Bilophila wadsworthii, Desulfovibrio piger, Cornyebacterium durum, Bifidobacterium adolescentis, Actinomyces graevenitzii, Cornyebacterium sundsvallense, Actinomyces odontolyticus,* and *Collinsella aerofaciens.*

In some embodiments, host cells or organisms can be selected from known natural probiotic strains. Exemplary probiotic species include those belonging to the genus *Lactobacillus, Bifidobacterium,* and/or *Streptococcus.* Exemplary probiotic strains include *Bacillus coagulans* GBI-30, 6086, *Bifidobacterium animalis* subsp. *lactis* BB-12, *Bifidobacterium longum* subsp. *infantis* 35624, *Lactobacillus paracasei* St11 (or NCC2461), *Lactobacillus johnsonii* La1 *(Lactobacillus johnsonii* NCC533), *Lactobacillus plantarum* 299v, *Lactobacillus reuteri* ATCC 55730, *Lactobacillus reuteri* DSM 17938, *Lactobacillus reuteri* ATCC PTA 5289, *Saccharomyces boulardii, Lactobacillus rhamnosus* GR-1, *Lactobacillus reuteri* RC-14, *Lactobacillus acidophilus* CL1285, *Lactobacillus casei* LBC80R, *Lactobacillus plantarum* HEAL 9, *Lactobacillus paracasei* 8700:2, *Streptococcus thermophilus, Lactobacillus paracasei* LMG P 22043, *Lactobacillus johnsonii* BFE 6128, *Lactobacillus fermentum* ME-3, *Lactobacillus plantarum* BFE 1685, *Bifidobacterium longum* BB536 and *Lactobacillus rhamnosus* LB21 NCIMB 40564.

In some embodiments, the host cell or organism is derived from a laboratory or commensal *Escherichia coli* strain. Exemplary *Escherichia coli* strains are set forth below (Table 4). Strain W is the laboratory strain believed to most closely resemble commensal strains [Archer, 2011]. Strain Nissle 1917 has long been used as a probiotic in human under the trade name Mutaflor [Grozdanov, 2004]. The *Escherichia coli* Collection Of Reference (ECOR) is a collection of commensal *Escherichia coli* strains that were isolated from the gut of healthy mammals [Ochman 1984]. ECOR strains have not undergone substantial laboratory evolution since their isolation, and are therefore used as model commensal strains.

**Table 4: Escherichia coli strains**

| *Strain* | *Accession* |
|---|---|
| *E. coli* HS | NC_009800 |
| *E. coli* SE11 | NC_0111415 |
| *E. coli* SE15 | AP009378 |
| *E. coli* W | CP002185 |
| *E. coli* Nissle 1917 | AJ586887-9 |
| *E. coli* ECOR-08 | ECOR-08 |
| *E. coli* ECOR-26 | ECOR-26 |
| *E. coli* ECOR-34 | ECOR-34 |
| *E. coli* ECOR-36 | ECOR-36 |
| *E. coli* ECOR-44 | ECOR-44 |
| *E. coli* ECOR-47 | ECOR-47 |
| *E. coli* ECOR-51 | ECOR-51 |
| *E. coli* ECOR-56 | ECOR-56 |
| *E. coli* ECOR-59 | ECOR-59 |
| *E. coli* ECOR-61 | ECOR-61 |

In some embodiments, the host cell or organism is derived from the genus *Lactobacillus.* Exemplary *Lactobacillus* species include *Lactobacillus casei, Lactobacillus lactis, Lactobacillus reuteri, Lactobacillus rhamnosus, Lactobacillus acidophilus, Lactobacillus plantarum, Lactobacillus paracasei, Lactobacillus bulgaricus, Lactobacillus fermentum* and *Lactobacillus johnsonii.*

The host cell or organism, as disclosed herein, may be chosen from eukaryotic or prokaryotic systems capable of surviving, persisting and/or colonizing skin, such as bacterial cells (Gram-negative or Gram-positive), archaea and yeast cells. Suitable organisms can include those bacteria belonging to the Bacteroidetes, Firmicutes, Proteobacteria, and Actinobacteria phyla. In a preferred embodiment, the host cell/organism is culturable in the laboratory. In some embodiments, host cells/organisms can be selected from the genera *Staphylococcus, Propionibacterium, Malassezia, Corynebacterium, Brevibacterium, Lactococcus, Lactobacillus, Debaryomyces,* and *Cryptococcus.* Exemplary species include *Staphylococcus epidermis, Propionibacterium acnes, Lactococcus lactis, Lactobacillus reuteri* and *Lactobacillus plantarum.*

The host cell or organism, as disclosed herein, may be chosen from eukaryotic or prokaryotic systems capable of surviving, persisting and/or colonizing the environment or substance to be decontaminated, such as bacterial cells (Gram-negative or Gram-positive), archaea and yeast cells.

It should be noted that various engineered strains and/or mutations of the organisms or cell lines discussed herein can also be used.

### Antibiotic-free maintenance and containment of the engineered probiotic

In accrodance with the claims, the present disclosure provides for a mechanism to select for the maintenance of the engineered probiotic and/or the heterologous genetic system comprising a mobilizable gene targeting and degradation system. Conventionally, plasmid maintenance in host cells or organisms is selected for through the inclusion of antibiotic resistance cassettes and the application of antibiotics to the microbial population. However, the inclusion of antibiotic resistance cassettes in the engineered probiotic of the present disclosure is undesirable since it may lead to unwanted spread of the cassette. Furthermore, use of antibiotics in, for example, the gastrointestinal microbiome, selects against other commensal strains which can promote re-colonization by pathogenic strains particularly in hospital environments [Fekety, 1981]. In accordance with the claims , the engineered probiotic and/or the heterologous genetic system comprises a nucleic acid encoding one or more genes that confers a selective advantage that is not based on antibiotic resistance.

In accordance with the claims, the nucleic acid encodes one or more nucleic acid molecules comrpsing a carbon utilization operon that confer the ability to utilize particular carbon source(s) not used by the parent, wildtype host cell or organism from which the engineered probiotic is derived. The inclusion of these carbon source utilization gene(s) confers a selective advantage to any cells carrying the heterologous genetic system when grown in the presence of the corresponding carbon source. Other strains in the microbial population will not be selected against, however, since other carbon sources are available for their growth. In the absence of the corresponding carbon source, the burden of replicating, transcribing and translating the carbon source utilization gene(s) has the additional benefit of limiting the fitness of the engineered probiotic. In this way, the engineered probiotic and/or heterologous genetic system comprising a mobilizable gene targeting and degradation system may be selected for maintenance and dispersal under specific conditions (presence of the carbon source) and selected for containment and loss under other conditions (absence of the carbon source). Coadministration of the carbon source with the probiotic can be used as a means to control the propagation and duration of the probiotic treatment.

In some embodiments, the carbon source utilization gene(s) are derived from the *raf* operon. The *raf* operon confers the ability to catabolize the trisaccharide raffinose and has been found on multiple conjugative plasmids [Aslanidis, 1989; Périchon, 2008]. In the *raf* operon, raffinose inhibits repression of raffinose catabolic genes by the RafR repressor [Ulmke, 1997; Aslandis, 1989]. Raffinose utilization genes include *rafA* which encodes an alpha-D-galactosidase, *rafB* which encodes a permease, *rafD* which encodes an invertase and *rafY* which encodes a porin. Exemplary *raf* operon genes are set forth below (Table 5).

**Table 5: raf operon genes**

| ***Gene*** | ***Function*** | ***Accession number*** |
|---|---|---|
| *rafR* | repressor | M29849 (166... 1176) |
| *rafA* | alpha- D-galactosidase | M27273.1 (70...2196) |
| *rafB* | raffinose permease | M27273.1 (2259...3536) |
| *rafD* | raffinose invertase / sucrose hydrolase | M27273.1 (3536...4966) |
| *rafY* | porin | U82290 (302...1696) |

In some embodiments, the carbon source utilization gene(s) are derived from the *csc* operon. The *csc* operon confers the ability to catabolize the sugar sucrose [Archer, 2011]. The *csc* operon comprises *cscR* which encodes a regulator, *cscB* which encodes a sucrose transporter, *cscA* which encodes an invertase, *cscK* which encodes a fructokinase. Exemplary *csc* operon genes are set forth below (Table 6).

**Table 6: csc operon genes**

| ***Gene*** I | ***Function*** I | ***Accession number*** |
|---|---|---|
| cscR | repressor | X81461.2 (7060...8055) |
| cscB | sucrose transporter | X81461.2 (3171...4418) |
| cscA | sucrose invertase / sucrose hydrolase | X81461.2 (5619...7052) |
| cscK | fructokinase | X81461.2 (4480...5403) |

In some embodiments, the carbon source utilization gene(s) are derived from the *xyl* operon. The *xyl* operon confers the ability to catabolize the sugar xylose [Song, 1997]. Exemplary *xyl* operon genes are set forth below (Table 7).

**Table 7: xyl operon genes**

| ***Gene*** | ***Function*** | ***Accession number*** |
|---|---|---|
| xylR | transcriptional activator | NC_007779.1 (3904258..3905436) |
| xylA | xylose isomerase | NC_007779.1 (3909650..3910972) |
| xylB | xyulokinase | NC_007779.1 (3911044..3912498) |
| xylF | xylose ABC transporter subunit | NC_007779.1 (3908292..3909284) |
| xylG | xylose ABC transporter subunit | NC_007779.1 (3906673..3908214) |
| xylH | xylose ABC transporter subunit | NC_007779.1 (3905514..3906695) |

### In some embodiments, the carbon source utilization gene(s) are derived from the ara operon. The ara operon confers the ability to catabolize the sugar arabinose [Miyada, 1984]. Exemplary ara operon genes are set forth below (

Table 8).

**Table 8: ara operon genes**

| ***Gene*** | ***Function*** | ***Accession number*** |
|---|---|---|
| araC | transcriptional activator | NC_000913.3 (70387..71265) |
| araB | L-ribulokinase | NC_000913.3 (68348..70048) |
| araA | L-arabinose isomerase | NC_000913.3 (66835..68337) |
| araD | L-ribulose-5-phosphate 4-epimerase | NC_000913.3 (65855..66550) |
| araF | arabinose ABC transporter subunit | NC_000913.3 (1985139..1986128,) |
| araG | arabinose ABC transporter subunit | NC_000913.3 (1983555..1985069) |
| araH | arabinose ABC transporter subunit | NC_000913.3 (1982554..1983540) |

In some aspects, the present disclosure provides for a mechanism to select against the uptake of additional mobile genetic elements by the engineered probiotic of the present disclosure. Various bacterial strains including *Escherichia coli, Vibrio chlolerae* and *Nitrosomonas europaea* are known to contain one or more toxin-antitoxin system encoded on their chromosomes; preliminary studies suggest that chromosomally integrated toxin-antitoxin systems may serve to protect the cell from foreign DNA including conjugative plasmids [Saavedra De Bast, 2008]. Thus, in some embodiments, the engineered probiotic of the present disclosure comprises a chromosomally integrated toxin-antitoxin system to restrict uptake and maintenance of foreign DNA from other strains in the microbiome. Exemplary toxin-antitoxin systems, the elements targeted by their cognate toxins, and the cellular process disrupted by the toxins are set forth below (Table 9) [Van Melderen, 2009]. Toxin-antitoxin systems produce a toxin protein that target a cellular process; the antitoxin (typically an RNA or protein) prevents the toxin from disrupting the targeted cellular process. For example, in the MazF system, the MazF toxin protein disrupts RNA translation, and the MazE antitoxin protein binds MazF to ameliorate the toxic activity.

**Table 9: Toxin-antitoxin systems**

| ***System*** | ***Target*** | ***Cellular Process*** |
|---|---|---|
| CcdB | DNA gyrase | replication |
| RelE | translating ribosome | translation |
| MazF | RNAs | translation |
| ParE | DNA gyrase | replication |
| Doc | translating ribosome | translation |
| VapC | RNAs | unknown |
| ζ | unknown | unknown |
| HipA | Ef-Tu | translation |
| HigB | translating ribosome | translation |

In some aspects, the present disclosure provides for a mechanism to select for the functional expression of the CRISPR/Cas based gene targeting and degradation system. It has been demonstrated that natural CRISPR/Cas systems exist that degrade endogenous mRNA transcripts while leaving the corresponding genomic DNA intact [Sampson, 2013]. This is accomplished in *Francisella novicida* by a scaRNA molecule that forms a complex with tracrRNA and the FTN_1103 mRNA. Since the scaRNA binds specifically to the folded FTN_1103 mRNA, Cas9 selectively degrades the mRNA and not the FTN_1103 DNA sequence. In some embodiments, the mechanism of selection for a functional CRISPR/Cas based gene targeting and degradation system comprises a lethal gene that has been integrated into the chromosome of the engineered probiotic and a CRISPR/Cas system designed to target the mRNA encoded by the lethal gene for degradation while leaving the lethal gene intact. Thus, in some embodiments, the lethal gene is the toxin-encoding gene *mazF* and the CRISPR/Cas system is designed to target the *mazF* mRNA toxin for degradation based on its predicted mRNA secondary structure. In an alternative embodiment, the Cas gene is co-located and/or co-transcribed with the *mazE* gene which encodes the antitoxin. In this embodiment, there is a selection for Cas gene maintenance and/or expression rather than function.

In accordance with the claims, an engineered probiotic comprises a genetic system encoding a nuclease, a MGE, and an antibiotic-free selection module. Genetic systems containing all three modules may serve to transfer from the host cell to cells of interest in a microbial community via the MGE. The nuclease may target a gene of interest for degradation, and the antibiotic-free selection module provides a means of encouraging the propagation of the genetic system in the intended microbial community.

These genetic systems may optionally include additional genetic elements, such as a nuclease, transcriptional activator, or transcriptional repressor.

### Sequences provided by the disclosure

Table 10 provides a summary of SEQ ID NOs:1-20 disclosed herein.

**Table 10: Sequences**

| **SEQ ID NO** | **Sequence** |
|---|---|
| 1 | Cas9 nuclease expression cassette |
| 2 | tracrRNA under control of an inducible promoter |
| 3 | CRISPR array targeting *cat* gene |
| 4 | Plasmid encoding *cat* gene and fluorescent reporter |
| 5 | Plasmid encoding *cat* gene and fluorescent reporter |
| 6 | Plasmid encoding *cat* gene and fluorescent reporter |
| 7 | Plasmid encoding *cat* gene and fluorescent reporter |
| 8 | Plasmid encoding *cat* gene and fluorescent reporter |
| 9 | Plasmid encoding tetracycline antibiotic resistance gene |
| 10 | Plasmid encoding guide RNA targeting *cat* gene, target site #8 |
| 11 | Plasmid encoding guide RNA targeting *cat* gene, target site #8 |
| 12 | Plasmid encoding guide RNA targeting *cat* gene, target site #7 |
| 13 | Plasmid encoding guide RNA targeting *cat* gene, target site #8 |
| 14 | Plasmid encoding guide RNA targeting off-target gene |
| 15 | Plasmid encoding guide RNA targeting *cat* gene, target site #8 |
| 16 | Plasmid encoding guide RNA targeting *cat* gene, target site #7 |
| 17 | Mobile conjugative plasmid encoding guide RNA targeting off-target gene |
| 18 | Mobile conjugative plasmid encoding guide RNA targeting *cat* gene, target site #7 |
| 19 | Alternative mobile conjugative plasmid with transposase, encoding guide RNA targeting off target gene |
| 20 | Alternative mobile conjugative plasmid with transposase, encoding guide RNA targeting *cat* gene, target site #7 |

### EXAMPLES

The examples below are provided herein for illustrative purposes and are not intended to be restrictive. For example, while the below examples focus on probiotic engineering, other cells can also be engineered using similar methods and designs.

### Example 1: A model of dispersal of the gene targeting and degradation system within a microbial population

A basic model can be formulated to describe the spread of the gene targeting and degradation system from the engineered probiotic to other members of a microbial community (FIG. 1). In the equations, P(t) and F(t) denotes the subpopulations with and without the gene targeting and degradation system, respectively. R(t) denotes the pool of growth resources available. The model is derived from previously validated models of mobile genetic element dispersion [Bergstrom, 2000; Stewart, 1977]. The other variables are defined as in Table 11.

**Table 11: Model parameters**

| ***Variable*** | ***Definition*** |
|---|---|
| µ | Growth rate |
| c | Cost of bearing the system |
| b | Benefit of bearing the system |
| κ | Rate of conjugation |
| σ | Rate of plasmid loss due to segregation |
| kᵣ | Critical level of the resource pool |
| ρ | Flow of resources into the environment |
| r | Amount of resources consumed to produce a daughter cell |

The model supports the determination of the initial inoculum density of the engineered probiotic required to achieve colonization, the residence time of the gene targeting and degradation system in the gastrointestinal system, and the ratio of engineered to virulent/antibiotic-resistant microbial cells required for effective clearance of the virulent/antibiotic-resistant genes.

### Example 2: Candidate target sites in a gene of interest

CRISPR/Cas gene targeting and degradation systems may be targeted to select sites within a gene of interest. In the case of systems derived from the *Streptomyces pyogenes* Cas9 nuclease, target sequences must be immediately 5' to the sequence NGG, where "N" can be any nucleotide. FIG. 2 depicts a schematic of the *Yersinia pestis* biovar Orientalis str IP275 chloramphenicol acetyltransferase coding sequence (CAT, Genbank accession NC_009141 40824...41483). Sites suitable for targeting with the *S. pyogenes* Cas9 nuclease are annotated in dark gray. Selected target sites and gene features of interest are annotated in light gray. Selected target sites are chosen to coincide with important functional or structural motifs within a gene of interest.

### Example 3: Design and construction of a CRISPR/Cas gene targeting and degradation system

Three components are needed for the proper functioning of a CRISPR/Cas gene targeting and degradation system derived from the *Streptomyces pyogenes* Cas9 nuclease: the Cas9 protein itself, a CRISPR array containing one or more target DNA "spacers" flanked by CRISPR direct repeats, and a tracrRNA that forms a complex with Cas9 and the crRNA transcribed from the CRISPR array. FIG. 3 depicts example designs of a CRISPR/Cas gene targeting system. The CRISPR array is based on the *Streptomyces pyogenes* CRISPR array and is designed to target a gene of interest: the chloramphenicol acetyltransferase coding sequence (CAT) described in Example 2. Both the CRISPR array (FIG. 3A) and the tracrRNA (FIG. 3B) are placed under the control of inducible promoters. The Cas9 protein may be expressed constitutively (FIG. 3C). In an alternative design of the CRISPR array, a five spacer array targeted at the CAT gene was constructed (FIG. 3D). However, multiple bands corresponding to different length arrays were observed during gel electrophoresis of arrays synthesized via commercial gene synthesis. The fact that we were unable to obtain a clonal population of this DNA design from commercial gene synthesis providers suggests that this five spacer array design is vulnerable to recombination between repeat units. In an alternative design, the tracrRNA and target spacer are combined into a single guide RNA (FIG. 3E) that is easier to construct and less vulnerable to recombination.

### Example 4: Engineered probiotic strains capable of repelling invading antibiotic resistance genes

An engineered probiotic strain was designed and constructed comprising a *Streptomyces pyogenes* Cas9 nuclease expression cassette (SEQ ID NO:1), a tracrRNA (SEQ ID NO:2) and a CRISPR array (SEQ ID NO:3). The CRISPR array was designed to target a single 30 base pair site in the *Yersinia pestis* biovar Orientalis str IP275 chloramphenicol acetyltransferase coding sequence (CAT). A second engineered strain was constructed that omitted the CRISPR array to serve as a control strain.

Both strains were challenged via transformation with a plasmid encoding CAT and a fluorescent protein (SEQ ID NO:4). The engineered probiotic strain was found to be effective in repelling plasmids encoding a gene expression cassette comprising CAT (FIG. 4). We observed a 10⁴-10⁵ fold decrease in colony forming units, when selecting on the antibiotic chloramphenicol (FIG. 4A and 4B show results from the engineered probiotic and control strain, respectively). In the absence of selection, the engineered probiotic is 100% effective in repelling the plasmid encoding the CAT gene (FIG. 4C and 4D show results from the engineered probiotic and control strain, respectively).

The engineered probiotic and control strain were challenged with five different plasmid designs, each of which encodes the *Y. pestis* CAT gene (SEQ ID NOs:4-8). In each case, the engineered probiotic successfully repelled the plasmid relative to the control strain even in the presence of chloramphenicol selection (FIG. 5).

To verify that the observed results were as a result of activity of the CRISPR/Cas gene targeting and degradation system rather than reduced cell competence from maintenance of plasmid encoding the CRISPR array, both the engineered probiotic and control strain were challenged with plasmids that did not encode the *Y. pestis* CAT gene but did encode a tetracycline antibiotic resistance gene (SEQ ID NO:9). No significant difference in the number of colonies obtained after transformation and growth on tetracycline plates (FIG. 6). These results indicate that the engineered probiotic is specific for the target gene of interest.

To determine whether this effect was specific to laboratory strains of *Escherichia coli,* we repeated the experiment using the commensal *Escherichia coli* strains ECOR-44 and ECOR-61 as hosts for the CRISPR/Cas gene targeting and degradation system. Upon challenging these strains with CAT expressing plasmids, we observed that the commensal strains similarly showed a 10⁴-10⁵ decrease in colony forming units when selecting on the antibiotic chloramphenicol (FIG. 9).

### Example 5: Removal of previously acquired antibiotic resistance genes using a CRISPR/Cas gene targeting and degradation system

A target strain was designed and constructed comprising a low copy plasmid encoding a *Streptomyces pyogenes* Cas9 nuclease expression cassette (SEQ ID NO:1) and a high copy plasmid encoding a *Yersinia pestis* biovar Orientalis str IP275 chloramphenicol acetyltransferase coding sequence (CAT) and fluorescent protein (SEQ ID NO:4). The target strain was subsequently challenged via transformation with guide RNA (gRNA) constructs targeted at different sequences (SEQ ID NOs:10-16). Target strains challenged with gRNAs targeted at the CAT gene (SEQ ID NO:10-13 and SEQ ID NO:15-16) showed a loss of fluorescence and chloramphenicol resistance, whereas a gRNA targeted at a different gene (SEQ ID NO:14) did not impact fluorescence or chloramphenicol resistance phenotypes of the target strain (FIG. 7). Two different on-target gRNAs were tested, each of which targeted a different 20 base pair sequence within the CAT gene; both were effective and did not show evidence of off-target activity irrespective of the identity of the promoter driving transcription of the gRNA or the plasmid propagating the gRNA. The off-target gRNA did not show any evidence of deleterious activity. These results suggest that designed CRISPR/Cas systems can specifically target and remove undesirable DNA elements from target strains.

### Example 6: Design of an engineered probiotic

An engineered probiotic was designed comprising a CRISPR/Cas gene targeting and degradation system and selection and containment mechanism derived from the *raf* operon (FIG. 8). The gene targeting and degradation system and selection and containment mechanism are flanked by inverted repeats and are adjacent to a mobilizable origin and transposon (FIG. 8A) to facilitate dispersal and integration into the genomes of other cells within a microbial community. FIG. 8B shows a selection module design based on the *raf* operon, in which raffinose inhibits transcription of the *rafA, rafB, rafD* and *rafY* genes.

### Example 7: Antibiotic-free promotion of engineered probiotic growth

Carbon utilization operons are being used as a means to promote the growth of engineered probiotic strains over competing bacterial strains, without the use of antibiotics. For example, we have demonstrated that the *raf* operon confers a growth advantage to host *Escherichia coli* strains when grown in the presence of raffinose. FIG. 10 illustrates how the *raf* operon can be engineered to function constitutively ("Constitutive Selection Module") or in an inducible fashion based on the presence of raffinose ("Inducible Selection Module"). The "Gemini Control" design in FIG. 10 is a control plasmid that is identical to the Constitutive Selection Module and Inducible Selection Module plasmids, with the exception that the hybrid fluorescent reporter Gemini is expressed in the place of a *raf* operon [Martin 2009].

Laboratory strains of *Escherichia coli* containing the Constitutive Selection Module were placed in competition with strains containing the Gemini Control plasmid. When raffinose is present at a concentration of 1.0% (weight per volume) in the growth media, strains containing the Constitutive Selection Module grow to a higher final titer than identical strains containing the Gemini Control plasmid instead (FIG. 11).

Some commensal strains of *Escherichia coli* also outgrow Gemini Control strains when transformed with the Constitutive Selection Module. For example, commensal strains *E. coli* ECOR-08 and E. *coli* ECOR-51 (each transformed with the Constitutive Selection Module) outgrow a laboratory strain of *Escherichia coli* transformed with the Gemini Control plasmid (FIG. 12). Laboratory strains of *Escherichia coli* are better adapted to the growth conditions used in FIG. 12, thus the other ECOR strains tested in this experiment may also exhibit a growth advantage with raffinose when grown in the mammalian gut or similar environments.

### Other embodiments

The examples have focused on *Escherichia coli.* Nevertheless, the key concept of using CRISPR/Cas systems to confer the ability to target and degrade undesirable genes of interest is, as one of ordinary skill in the art would understand, extensible to other commensal strains and/or probiotic strains such as other prokaryotes including *Lactobacillus* or eukaryotic single cell organisms.

The examples have focused on, by way of example only, targeting the chloramphenicol resistance gene for degradation. Nevertheless, the key concept of using an engineered probiotic to target and degrade a gene or genes of interest is, as one of ordinary skill in the art would understand, extensibleto other nucleic acids such as genetic elements that encode pathogenic, virulent, virulence factors, or alternative antibiotic resistance traits. It is also extensible to other nucleic acids such as RNA that is transcribed from a gene of interest, pathogenic element or non-coding genetic elements, as one of ordinary skill in the art would understand.

Various aspects of the present disclosure may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

### EQUIVALENTS

### References Cited

Adamczyk M, Jagura-Burdzy G. Spread and survival of promiscuous IncP-1 plasmids. Acta Biochim Pol. 2003;50(2):425-53.

Aizenman E, Engelberg-Kulka H, Glaser G. An Escherichia coli chromosomal "addiction module" regulated by guanosine [corrected] 3',5'-bispyrophosphate: a model for programmed bacterial cell death. Proc Natl Acad Sci U S A. 1996 Jun 11;93(12):6059-63. Erratum in: Proc Natl Acad Sci U S A 1996 Sep 3;93(18):9991.

Allen HK. Antibiotic resistance gene discovery in food-producing animals. Curr Opin Microbiol. 2014 Jun;19:25-9.

Archer CT, Kim JF, Jeong H, Park JH, Vickers CE, Lee SY, Nielsen LK. The genome sequence of E. coli W (ATCC 9637): comparative genome analysis and an improved genome-scale reconstruction of E. coli. BMC Genomics. 2011 Jan 6;12:9.

Aslanidis C, Schmid K, Schmitt R. Nucleotide sequences and operon structure of plasmid-borne genes mediating uptake and utilization of raffinose in Escherichia coli. J Bacteriol. 1989 Dec;171(12):6753-63.

Barrangou R, Fremaux C, Deveau H, Richards M, Boyaval P, Moineau S, Romero DA, Horvath P. CRISPR provides acquired resistance against viruses in prokaryotes. Science. 2007 Mar 23;315(5819):1709-12.

Bergstrom CT, Lipsitch M, Levin BR. Natural selection, infectious transfer and the existence conditions for bacterial plasmids. Genetics. 2000 Aug;155(4):1505-19.

Bhaya D, Davison M, Barrangou R. CRISPR-Cas systems in bacteria and archaea: versatile small RNAs for adaptive defense and regulation. Annu Rev Genet. 2011;45:273-97.

Brumbaugh, A. R. & Mobley, H. L. T. Preventing urinary tract infection: progress toward an effective Escherichia coli vaccine. Expert Review of Vaccines. 2012; 11, 663-676.

Cermak T, Doyle EL, Christian M, Wang L, Zhang Y, Schmidt C, Baller JA, Somia NV, Bogdanove AJ, Voytas DF. Efficient design and assembly of custom TALEN and other TAL effector-based constructs for DNA targeting. 2011 Jul;39(12):e82.

Cong L, Ran FA, Cox D, Lin S, Barretto R, Habib N, Hsu PD, Wu X, Jiang W, Marraffini LA, Zhang F. Multiplex genome engineering using CRISPR/Cas systems. Science. 2013 Feb 15;339(6121):819-23.

Deltcheva E, Chylinski K, Sharma CM, Gonzales K, Chao Y, Pirzada ZA et al. (). CRISPR RNA maturation by trans-encoded small RNA and host factor RNase III. Nature. 2011;471 (7340): 602-607.

Deveau H, Barrangou R, Garneau JE, Labonté J, Fremaux C, Boyaval P, Romero DA, Horvath P, Moineau S. Phage response to CRISPR-encoded resistance in Streptococcus thermophilus. J Bacteriol. 2008 Feb;190(4):1390-400. DiCarlo JE, Norville JE, Mali P, Rios X, Aach J, Church GM. Genome engineering in Saccharomyces cerevisiae using CRISPR-Cas systems. Nucleic Acids Res. 2013 Apr;41(7):4336-43. doi: 10.1093/nar/gkt135.

Fekety R, Kim KH, Brown D, Batts DH, Cudmore M, Silva J Jr. Epidemiology of antibiotic-associated colitis; isolation of Clostridium difficile from the hospital environment. Am J Med. 1981 Apr;70(4):906-8.

Heidelberg JF, Nelson WC, Schoenfeld T, Bhaya D. Germ warfare in a microbial mat community: CRISPRs provide insights into the co-evolution of host and viral genomes. PLoS One. 2009;4(1):e4169.

Horvath P, Romero DA, Coûté-Monvoisin AC, Richards M, Deveau H, Moineau S, Boyaval P, Fremaux C, Barrangou R. Diversity, activity, and evolution of CRISPR loci in Streptococcus thermophilus. J Bacteriol. 2008 Feb;190(4):1401-12.

Horvath P, Coûté-Monvoisin AC, Romero DA, Boyaval P, Fremaux C, Barrangou R. Comparative analysis of CRISPR loci in lactic acid bacteria genomes. Int J Food Microbiol. 2009 Apr 30;131(1):62-70.

Ishino Y, Shinagawa H, Makino K, Amemura M, Nakata A. Nucleotide sequence of the iap gene, responsible for alkaline phosphatase isozyme conversion in Escherichia coli, and identification of the gene product. J Bacteriol. 1987 Dec;169(12):5429-33.

Jiang W, Bikard D, Cox D, Zhang F, Marraffini LA. RNA-guided editing of bacterial genomes using CRISPR-Cas systems. Nat Biotechnol. 2013 Mar;31(3):233-9.

Jinek M, Chylinski K, Fonfara I, Hauer M, Doudna JA, Charpentier E. A programmable dual-RNA-guided DNA endonuclease in adaptive bacterial immunity. Science. 2012 Aug 17;337(6096):816-21.

Jinek M, East A, Cheng A, Lin S, Ma E, Doudna J. RNA-programmed genome editing in human cells. Elife. 2013;2:e00471.

Joung et al., "TALENs: a widely applicable technology for targeted genome editing," Nat. Rev. Mol. Cell Biol. 14, 49-55 (2012).

Kaper JB, Nataro JP, Mobley HL. Pathogenic Escherichia coli. Nat Rev Microbiol. 2004 Feb;2(2):123-40.

Kunin V, Sorek R, Hugenholtz P. Evolutionary conservation of sequence and secondary structures in CRISPR repeats. Genome Biol. 2007;8(4):R61.

Ley RE, Peterson DA, Gordon JI. Ecological and evolutionary forces shaping microbial diversity in the human intestine. Cell. 2006 Feb 24;124(4):837-48.

Li T, Huang S, Zhao X, Wright DA, Carpenter S, Spalding MH, Weeks DP, Yang B. Modularly assembled designer TAL effector nucleases for targeted gene knockout and gene replacement in eukaryotes. 2011 Aug;39(14):6315-25.

Maeder, et al., Rapid "open-source" engineering of customized zinc-finger nucleases for highly efficient gene modification. Mol Cell. 2008 Jul 25;31(2):294-301.

Mali P, Yang L, Esvelt KM, Aach J, Guell M, DiCarlo JE, Norville JE, Church GM. RNA-guided human genome engineering via Cas9. Science. 2013 Feb 15;339(6121):823-6.

Martin L, Che A, Endy D. Gemini, a bifunctional enzymatic and fluorescent reporter of gene expression. PLoS ONE. 2009;4(11):e7569.

Miyada CG, Stoltzfus L, Wilcox G. Regulation of the araC gene of Escherichia coli: catabolite repression, autoregulation, and effect on araBAD expression. Proc Natl Acad Sci USA. 1984 Jul;81(13):4120-4.

Murooka Y, Harada T. Expansion of the host range of coliphage PI and gene transfer from enteric bacteria to other gram-negative bacteria. Appl Environ Microbiol. 1979 Oct;38(4):754-7.

Ochman H, Selander RK. Standard reference strains of Escherichia coli from natural populations. J Bacteriol. 1984 Feb;157(2):690-3.

O'Connell MR, Oakes BL, Sternberg SH, East-Seletsky A, Kaplan M, Doudna JA. Programmable RNA recognition and cleavage by CRISPR/Cas9. Nature. 2014 Dec 11;516(7530):263-6.

Périchon B, Bogaerts P, Lambert T, Frangeul L, Courvalin P, Galimand M. Sequence of conjugative plasmid pIP1206 mediating resistance to aminoglycosides by 16S rRNA methylation and to hydrophilic fluoroquinolones by efflux. Antimicrob Agents Chemother. 2008 Jul;52(7):2581-92.

Phadnis SH, Sasakawa C, Berg DE. Localization of action of the IS50-encoded transposase protein. Genetics. 1986 Mar 112(3):421-7.

Saavedra De Bast M, Mine N, Van Melderen L. Chromosomal toxin-antitoxin systems may act as antiaddiction modules. J Bacteriol. 2008 Jul;190(13):4603-9.

Salyers AA, Shoemaker NB, Stevens AM, Li LY. Conjugative transposons: an unusual and diverse set of integrated gene transfer elements. Microbiol Rev. 1995 Dec;59(4):579-90.

Silbergeld EK, Graham J, Price LB. Industrial Food Animal Production, Antimicrobial Resistance, and Human Health. Annu. Rev. Public. Health. 2008 Apr;29(1):151-69.

Silva et al., "Meganucleases and Other Tools for Targeted Genome Engineering: Perspectives and Challenges for Gene Therapy," Curr Gene Ther. Feb 2011; 11(1): 11-27.

Smillie CS, Smith MB, Friedman J, Cordero OX, David LA, Alm EJ. Ecology drives a global network of gene exchange connecting the human microbiome. Nature. 2011 Oct 30;480(7376):241-4.

Song S, Park C. Organization and regulation of the D-xylose operons in Escherichia coli K-12: XylR acts as a transcriptional activator. J Bacteriol. American Society for Microbiology; 1997 Nov;179(22):7025-32.

Stewart, F.M. & Levin, B.R., 1977. The Population Biology of Bacterial Plasmids: A PRIORI Conditions for the Existence of Conjugationally Transmitted Factors. Genetics, 87(2), pp.209-228.

Tsuda M, Tan HM, Nishi A, Furukawa K. Mobile catabolic genes in bacteria. J Biosci Bioeng. 1999;87(4):401-10.

Ulmke C, Lengeler JW, Schmid K. Identification of a new porin, RafY, encoded by raffinose plasmid pRSD2 of Escherichia coli. J Bacteriol. 1997 Sep;179(18):5783-8.

Urnov, et al., "Genome editing with engineered zinc finger nucleases," Nat. Rev. Genet. 11, 636-646 (2010).

Van Melderen L, De Bast MS. Bacterial Toxin-Antitoxin Systems: More Than Selfish Entities? Rosenberg SM, editor. PLoS Genet. 2009 Mar 27;5(3):e1000437.

## Claims

1. An engineered genetic system, comprising:
(a) a nuclease module that degrades a nucleic acid encoding a virulence factor, toxin, effector, pathogenic component and/or antibiotic resistance trait;
(b) a synthetic mobile genetic element (MGE) module capable of dispersing the system from one host cell to another;
wherein the nuclease module comprises a nuclease encoded by a gene located in the MGE module, wherein the nuclease module comprises:
i. a Cas protein and one or more synthetic crRNAs wherein each crRNA comprises a spacer having a target sequence derived from the nucleic acid of interest;
ii. a Transcription Activator-Like Effector Nuclease (TALEN) designed to target and degrade the nucleic acid of interest;
iii. a Zinc Finger Nuclease (ZFN) designed to target and degrade the nucleic acid of interest; or
iv. a meganuclease designed to target and degrade the nucleic acid of interest; and
(c) an antibiotic-free maintenance module, wherein the antibiotic-free maintenance module comprises one or more nucleic acid molecules comprising a carbon utilization operon.

2. The system of claim 1, wherein the crRNA(s) is transcribed and processed from a CRISPR array.

3. The system of claim 2, wherein the CRISPR array is placed under the control of an inducible promoter or a constitutive promoter.

4. The system of any one of claims 2-3, wherein the nuclease module further comprises a tracrRNA that forms a complex with the Cas protein and the crRNA.

5. The system of claim 4, wherein the tracrRNA is placed under the control of an inducible promoter or a constitutive promoter.

6. The system of claim 4 or 5, wherein the tracrRNA and the crRNA are provided in a single guide RNA.

7. The system of claim 1, wherein the Cas protein is expressed constitutively or inducibly.

8. The system of claim 1, wherein the target sequence is immediately adjacent to a Protospacer Associated Motif (PAM) in the nucleic acid of interest.

9. The system of claim 8, wherein the Cas protein is *Streptomyces pyogenes* Cas9 nuclease and the PAM has the NGG sequence 3' of the target sequence.

10. The system of claim 1, wherein the virulence factor, toxin, effector, pathogenic component and/or antibiotic resistance trait are selected from those listed in Tables 1 and 2.

11. The system of claim 1, wherein the MGE module comprises a gene encoding a transposase and a MGE selected from a bacteriophage, conjugative plasmid, or conjugative transposon.

12. The system of claim 11, wherein the transposase is derived from Tn3 or Tn5, and the MGE is derived from Tn916, RK2, P1, Tn5280, or Tn4651.

13. The system of claim 1, wherein the carbon utilization operon comprises a raf operon, a csc operon, an xyl operon, and/or an ara operon.

## Patentansprüche

1. Gentechnisch verändertes genetisches System, umfassend:
(a) ein Nuclease-Modul, das eine Nucleinsäure abbaut, die für einen Virulenz-Faktor, ein Toxin, einen Effektor, eine pathogene Komponente und/oder ein Antibiotikaresistenz-Merkmal kodiert;
(b) ein Modul aus einem synthetischen mobilen genetischen Element (MGE), das dazu in der Lage ist, das System von einer Wirtszelle zu einer anderen zu verteilen;
wobei das Nuclease-Modul eine Nuclease umfasst, für die ein Gen kodiert, das sich in dem MGE-Modul befindet, wobei das Nuclease-Modul Folgendes umfasst:
i. ein Cas-Protein und eine oder mehrere synthetische crRNAs, wobei jede crRNA einen Spacer umfasst, der eine Zielsequenz aufweist, die von der Nucleinsäure von Interesse abgeleitet ist;
ii. eine Transkriptionsaktivator-ähnliche Effektornuclease (TALEN), die konstruiert ist, um auf die Nucleinsäure von Interesse abzuzielen und diese abzubauen;
iii. eine Zinkfingernuclease (ZFN), die konstruiert ist, um auf die Nucleinsäure von Interesse abzuzielen und diese abzubauen; oder
iv. eine Meganuclease, die konstruiert ist, um auf die Nucleinsäure von Interesse abzuzielen und diese abzubauen; und
(c) ein antibiotikafreies Erhaltungsmodul, wobei das antibiotikafreie Erhaltungsmodul ein oder mehrere Nucleinsäuremoleküle umfasst, die ein Kohlenstoffnutzungsoperon umfassen.

2. System nach Anspruch 1, wobei die crRNA(s) von einer CRISPR-Anordnung transkribiert und verarbeitet wird.

3. System nach Anspruch 2, wobei die CRISPR-Anordnung unter die Kontrolle eines induzierbaren Promotors oder eines konstitutiven Promotors gebracht wird.

4. System nach einem der Ansprüche 2 bis 3, wobei das Nucleasemodul weiters eine tracrRNA umfasst, die mit dem Cas-Protein und der crRNA einen Komplex bildet.

5. System nach Anspruch 4, wobei die tracrRNA unter die Kontrolle eines induzierbaren Promotors oder eines konstitutiven Promotors gebracht wird.

6. System nach Anspruch 4 oder 5, wobei die tracrRNA und die crRNA in einer einzelnen Leit-RNA bereitgestellt sind.

7. System nach Anspruch 1, wobei das Cas-Protein konstitutiv oder induzierbar exprimiert wird.

8. System nach Anspruch 1, wobei die Zielsequenz unmittelbar benachbart zu einem Protospacer-assoziierten Motiv (PAM) in der Nucleinsäure von Interesse vorliegt.

9. System nach Anspruch 8, wobei das Cas-Protein Streptomyces-pyogenes-Cas9-Nuclease ist und das PAM die NGG-Sequenz 3' in Bezug auf die Zielsequenz aufweist.

10. System nach Anspruch 1, wobei der Virulenzfaktor, das Toxin, der Effektor, die pathogene Komponente und/oder das Antibiotikaresistenz-Merkmal aus jenen ausgewählt sind, die in Tabelle 1 und 2 aufgelistet sind.

11. System nach Anspruch 1, wobei das MGE-Modul ein Gen, das für eine Transposase kodiert, und ein MGE umfasst, das aus einem Bakteriophagen, einem konjugativen Plasmid oder einem konjugativen Transposon ausgewählt ist.

12. System nach Anspruch 11, wobei die Transposase von Tn3 oder Tn5 abgeleitet ist und wobei das MGE von Tn916, RK2, P1, Tn5280 oder Tn4651 abgeleitet ist.

13. System nach Anspruch 1, wobei das Kohlenstoffnutzungsoperon ein raf-Operon, ein csc-Operon, ein xyl-Operon und/oder ein ara-Operon umfasst.

## Revendications

1. Système génétique modifié, comprenant :
(a) un module de nucléase qui dégrade un acide nucléique codant pour un facteur de virulence, une toxine, un effecteur, un composant pathogène et/ou un caractère de résistance aux antibiotiques ;
(b) un module d'élément génétique mobile synthétique (MGE) capable de disperser le système d'une cellule hôte à une autre ;
dans lequel le module de nucléase comprend une nucléase codée par un gène situé dans le module MGE, dans lequel le module de nucléase comprend :
i. une protéine Cas et un ou plusieurs ARNcr synthétiques, chaque ARNcr comprenant un espaceur ayant une séquence cible dérivée de l'acide nucléique d'intérêt ;
ii. une nucléase effectrice de type activateur de transcription (TALEN) conçue pour cibler et dégrader l'acide nucléique d'intérêt ;
iii. une nucléase à doigt de zinc (ZFN) conçue pour cibler et dégrader l'acide nucléique d'intérêt ; ou
iv. une méganucléase conçue pour cibler et dégrader l'acide nucléique d'intérêt ; et
(c) un module de maintenance exempt d'antibiotique, le module de maintenance exempt d'antibiotique comprenant une ou plusieurs molécules d'acide nucléique comprenant un opéron d'utilisation de carbone.

2. Système selon la revendication 1, dans lequel les un ou plusieurs ARNcr sont transcrits et traités à partir d'un réseau CRISPR.

3. Système selon la revendication 2, dans lequel le réseau CRISPR est placé sous la commande d'un promoteur inductible ou d'un promoteur constitutif.

4. Système selon l'une quelconque des revendications 2 à 3, dans lequel le module de nucléase comprend en outre un ARNtracr qui forme un complexe avec la protéine Cas et l'ARNcr.

5. Système selon la revendication 4, dans lequel l'ARNtracr est placé sous la commande d'un promoteur inductible ou d'un promoteur constitutif.

6. Système selon la revendication 4 ou 5, dans lequel l'ARNtrac et l'ARNcr sont fournis dans un ARN guide unique.

7. Système selon la revendication 1, dans lequel la protéine Cas est exprimée de manière constitutive ou inductible.

8. Système selon la revendication 1, dans lequel la séquence cible est immédiatement associée à un motif adjacent au protospacer (PAM) dans l'acide nucléique d'intérêt.

9. Système selon la revendication 8, dans lequel la protéine Cas est la nucléase Cas9 de *Streptomyces pyogenes* et le PAM a la séquence NGG 3' de la séquence cible.

10. Système selon la revendication 1, dans lequel le facteur de virulence, la toxine, l'effecteur, le composant pathogène et/ou le caractère de résistance aux antibiotiques sont choisis parmi ceux énumérés dans les tableaux 1 et 2.

11. Système selon la revendication 1, dans lequel le module MGE comprend un gène codant pour une transposase et un MGE choisi parmi un bactériophage, un plasmide conjugué ou un transposon conjugué.

12. Système selon la revendication 11, dans lequel la transposase est dérivée de Tn3 ou Tn5, et le MGE est dérivé de Tn916, RK2, P1, Tn5280 ou Tn4651.

13. Système selon la revendication 1, dans lequel l'opéron d'utilisation de carbone comprend un opéron raf, un opéron csc, un opéron xyle et/ou un opéron ara.
